(19) Europäisches Patentamt
European Patent Office
Office européen des brevets

(11) EP 3 147 278 B1

(12) **EUROPEAN PATENT SPECIFICATION**

(45) Date of publication and mention of the grant of the patent:
**12.02.2020 Bulletin 2020/07**

(51) Int Cl.:
***C07C 255/51*** $^{(2006.01)}$ ***A61K 31/167*** $^{(2006.01)}$
***A61P 15/16*** $^{(2006.01)}$ ***C07C 255/60*** $^{(2006.01)}$

(21) Application number: **16194602.5**

(22) Date of filing: **12.07.2007**

(54) **COMPOUND FOR TREATING BREAST CANCER AND PROGESTIN-DEPENDENT CONDITIONS**

VERBINDUNG ZUR BEHANDLUNG VON BRUSTKREBS UND PROGESTINABHÄNGIGE BEDINGUNGEN

COMPOSÉ POUR LE TRAITEMENT DU CANCER DU SEIN ET CONDITIONS DÉPENDANT DES PROGESTATIFS DE SYNTHÈSE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.07.2006 US 830158 P**
**24.08.2006 US 839665 P**
**16.04.2007 US 907748 P**

(43) Date of publication of application:
**29.03.2017 Bulletin 2017/13**

(62) Document number(s) of the earlier application(s) in accordance with Art. 76 EPC:
**07796823.8 / 2 038 252**

(73) Proprietor: **University of Tennessee Research Foundation**
**Knoxville, TN 37996 (US)**

(72) Inventors:
- **DALTON, James T.**
**Ann Arbor, MI 48103 (US)**
- **MILLER, Duane D.**
**Collierville, TN 38017 (US)**

(74) Representative: **Pearl Cohen Zedek Latzer Baratz UK LLP**
**The Gridiron Building**
**One Pancras Square**
**London N1C 4AG (GB)**

(56) References cited:
**WO-A2-2004/064747 WO-A2-2005/037201**
**US-A1- 2006 111 441**

- **KIM, JUHYUN ET AL: "The para substituent of S-3-(phenoxy)-2-hydroxy-2-methyl-N-(4-nitr o-3-trifluoromethyl-phenyl)-propionamides is a major structural determinant of in vivo disposition and activity of selective androgen receptor modulators", JOURNAL OF PHARMACOLOGY AND EXPERIMENTAL THERAPEUTICS , 315(1), 230-239 CODEN: JPETAB; ISSN: 0022-3565, 2005, XP002609516,**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 147 278 B1

Printed by Jouve, 75001 PARIS (FR)

**Description**

**FIELD OF THE INVENTION**

This invention provides a substituted acylanilide compound for use in treating a variety of diseases or conditions in a subject.

**BACKGROUND OF THE INVENTION**

The nuclear hormone receptor superfamily is one of the largest classes of transcription factors and is involved in abundant physiological processes. The 48 members of this family are divided into three classes with class 1 comprised of receptors for androgens (AR), estrogens (ER-$\alpha$ and ER-$\beta$), glucocorticoids (GR), progesterone (PR) and mineralocorticoids (MR). Class 2 contains receptors for retinoids, thyroids and vitamin D, while class 3 includes receptors for which ligands are yet to be identified (orphans). Nuclear hormone receptors have an N-terminal domain (NTD) whose function is less characterized, a DNA binding domain (DBD) which is responsible for the binding of receptor to DNA response elements, a hinge region that contains the nuclear localization signal and a ligand binding domain (LBD) to which ligands bind and activate or inhibit receptor action. In addition, there are two activation function domains, one in the NTD (AF-1) and the other in the LBD (AF-2). Due to the high amino acid sequence homology of the DBD, moderate homology and similar secondary and tertiary structural features of the LBD, and common chemical features of steroidal ligands, class I receptors are often capable of binding (i.e., cross reacting) with the ligands of other class I receptors. For example, early studies with AR and ER suggest that the orientation of the steroid in the LBD, with the steroid A-ring in contact with helix-3 and the D-ring in contact with helix-11 residues, is likely to be general for all the steroid hormone receptors.

The androgen receptor ("AR") is a ligand-activated transcriptional regulatory protein that mediates induction of male sexual development and function through its activity with endogenous androgens. Androgens are generally known as the male sex hormones. The androgenic hormones are steroids which are produced in the body by the testes and the cortex of the adrenal gland or can be synthesized in the laboratory. Androgenic steroids play an important role in many physiologic processes, including the development and maintenance of male sexual characteristics such as muscle and bone mass, prostate growth, spermatogenesis, and the male hair pattern (Matsumoto, Endocrinol. Met. Clin. N. Am. 23:857-75 (1994)). The endogenous steroidal androgens include testosterone and dihydrotestosterone ("DHT"). Testosterone is the principal steroid secreted by the testes and is the primary circulating androgen found in the plasma of males. Testosterone is converted to DHT by the enzyme 5 alpha-reductase in many peripheral tissues. DHT is thus thought to serve as the intracellular mediator for most androgen actions (Zhou, et al., Molec. Endocrinol. 9:208-18 (1995)). Other steroidal androgens include esters of testosterone, such as the cypionate, propionate, phenylpropionate, cyclopentylpropionate, isocarporate, enanthate, and decanoate esters, and other synthetic androgens such as 7-Methyl-Nortestosterone ("MENT') and its acetate ester (Sundaram et al., "7 Alpha-Methyl-Nortestosterone(MENT): The Optimal Androgen For Male Contraception," Ann. Med., 25:199-205 (1993). Because the AR is involved in male sexual development and function, the AR is a likely target for effecting male contraception or other forms of hormone replacement therapy.

WO 2004/064747 A2 discloses a method of treating, preventing, suppressing, inhibiting or reducing the incidence of an Androgen Deficiency in Female (ADIF)-associated condition in a female subject, by administering to the subject a selective androgen receptor modulator (SARM) compound.

WO 2005/037201 A2 discloses a method of treating, preventing, suppressing, inhibiting, or reducing the risk of developing a bone-related disorder, for example osteoporosis, osteopenia, increased bone resorption, bone fracture, bone frailty and/or loss of bone mineral density (BMD), by administrating a therapeutically effective amount of a selective androgen receptor modulator (SARM).

US 2006/111441 A1 discloses the prevention and treatment of wasting disorders with a selective androgen receptor modulator (SARM).

Kim et al., Journal of Pharmacology and Experimental Therapeutics, 315(1), 2005, 230-239 discloses studies on the *para* substituent of *S*-3-(phenoxy)-2-hydroxy-2-methyl-*N*-(4-nitro-3-trifluoromethyl-phenyl)-propionamides as a major structural determinant of *in vivo* disposition and activity of selective androgen receptor modulators.

The human progesterone receptor (PR) occurs as three different isoforms: PR-A, PR-B, and PR-C (Kastner et al., EMBO J 9:1603-1614, 1990; Wei et al., Mol Endo 10:1379-1387, 1996), of which PR-A and PR-B are the most abundant. However, the ratio of PR-A vs. PR-B isoforms is not constant among target tissues, and this can alter the cellular response, because the activity of each isoform can vary.

There are very few compounds that exhibit partial progestin activity under a wide variety of conditions. RU-486, the most commonly used antiprogestin, displays partial agonist activity only under selected conditions. Antiprogestin compounds with partial agonist activity are useful for treating various progestin-regulated diseases and conditions,

however, the few known antiprogestins have only limited partial agonist activity, and there remains a need in the art for antiprogestins with broad-range partial agonist activity.

Worldwide population growth and social awareness of family planning have stimulated a great deal of research in contraception. Contraception is a difficult subject under any circumstance. It is fraught with cultural and social stigma, religious implications, and, most certainly, significant health concerns. This situation is only exacerbated when the subject focuses on male contraception. Despite the availability of suitable contraceptive devices, historically, society has looked to women to be responsible for contraceptive decisions and their consequences. Although concern over sexually transmitted diseases has made men more aware of the need to develop safe and responsible sexual habits, women still often bear the brunt of contraceptive choice. Women have a number of choices, from temporary mechanical devices such as sponges and diaphragms to temporary chemical devices such as spermicides. Women also have at their disposal more permanent options, such as physical devices including IUDs and cervical caps as well as more permanent chemical treatments such as birth control pills and subcutaneous implants. However, to date, the only options available for men include the use of condoms and vasectomy. Condom use, however is not favored by many men because of the reduced sexual sensitivity, the interruption in sexual spontaneity, and the significant possibility of pregnancy caused by breakage or misuse. Vasectomies are also not favored. If more convenient methods of birth control were available to men, particularly long-term methods which require no preparative activity immediately prior to a sexual act, such methods could significantly increase the likelihood that men would take more responsibility for contraception.

Administration of the male sex steroids (e.g., testosterone and its derivatives) has shown particular promise in this regard due to the combined gonadotropin-suppressing and androgen-substituting properties of these compounds (Steinberger et al., "Effect of Chronic Administration of Testosterone Enanthate on Sperm Production and Plasma Testosterone, Follicle Stimulating Hormone, and Luteinizing Hormone Levels: A Preliminary Evaluation of a Possible Male Contraceptive, Fertility and Sterility 28:1320- 28 (1977)). Chronic administration of high doses of testosterone completely abolishes sperm production (azoospermia) or reduces it to a very low level (oligospermia). The degree of spermatogenic suppression necessary to produce infertility is not precisely known. However, a recent report by the World Health Organization showed that weekly intramuscular injections of testosterone enanthate result in azoospermia or severe oligospermia (i.e., less than 3 million sperm per ml) and infertility in 98% of men receiving therapy (World Health Organization Task Force on Methods And Regulation of Male Fertility, "Contraceptive Efficacy of Testosterone-Induced Azoospermia and Oligospermia in Normal Men," Fertility and Sterility 65:821-29 (1996)).

A variety of testosterone esters have been developed which are more slowly absorbed after intramuscular injection and thus result in greater androgenic effect. Testosterone enanthate is the most widely used of these esters. While testosterone enanthate has been valuable in terms of establishing the feasibility of hormonal agents for male contraception, it has several drawbacks, including the need for weekly injections and the presence of supraphysiologic peak levels of testosterone immediately following intramuscular injection (Wu, "Effects of Testosterone Enanthate in Normal Men: Experience From a Multicenter Contraceptive Efficacy Study," Fertility and Sterility 65:626-36 (1996)).

Bone mineral density (BMD) decreases with age in both males and females. Decreased amounts of bone mineral content (BMC) and BMD correlate with decreased bone strength and predispose patients to fracture.

Osteoporosis is a systemic skeletal disease, characterized by low bone mass and deterioration of bone tissue, with a consequent increase in bone fragility and susceptibility to fracture. In the U.S., the condition affects more than 25 million people and causes more than 1.3 million fractures each year, including 500,000 spine, 250,000 hip and 240,000 wrist fractures annually. Hip fractures are the most serious consequence of osteoporosis, with 5-20% of patients dying within one year, and over 50% of survivors being incapacitated. The elderly are at greatest risk of osteoporosis, and the problem is therefore predicted to increase significantly with the aging of the population. Worldwide fracture incidence is forecasted to increase three-fold over the next 60 years, and one study estimated that there will be 4.5 million hip fractures worldwide in 2050.

Women are at greater risk of osteoporosis than men. Women experience a sharp acceleration of bone loss during the five years following menopause. Other factors that increase the risk include smoking, alcohol abuse, a sedentary lifestyle and low calcium intake. However, osteoporosis also occurs frequently in males. It is well established that the bone mineral density of males decrease with age. Decreased amounts of bone mineral content and density correlates with decreased bone strength, and predisposes to fracture. The molecular mechanisms underlying the pleiotropic effects of sex-hormones in non-reproductive tissues are only beginning to be understood, but it is clear that physiologic concentrations of androgens and estrogens play an important role in maintaining bone homeostasis throughout the life-cycle. Consequently, when androgen or estrogen deprivation occurs there is a resultant increase in the rate of bone remodeling that tilts the balance of resorption and formation to the favor of resorption that contributes to the overall loss of bone mass. In males, the natural decline in sex-hormones at maturity (direct decline in androgens as well as lower levels of estrogens derived from peripheral aromatization of androgens) is associated with the frailty of bones. This effect is also observed in males who have been castrated.

Muscle wasting refers to the progressive loss of muscle mass and/or to the progressive weakening and degeneration of muscles, including the skeletal or voluntary muscles, which control movement, cardiac muscles, which

control the heart (cardiomyopathics), and smooth muscles. Chronic muscle wasting is a chronic condition (i.e. persisting over a long period of time) characterized by progressive loss of muscle mass, weakening and degeneration of muscle.

The loss of muscle mass that occurs during muscle wasting can be characterized by muscle protein degradation by catabolism. Protein catabolism occurs because of an unusually high rate of protein degradation, an unusually low rate of protein synthesis, or a combination of both. Muscle protein catabolism, whether caused by a high degree of protein degradation or a low degree of protein synthesis, leads to a decrease in muscle mass and to muscle wasting.

Muscle wasting is associated with chronic, neurological, genetic or infectious pathologies, diseases, illnesses or conditions. These include muscular dystrophies such as Duchenne muscular dystrophy and myotonic dystrophy; muscle atrophies such as post-polio muscle atrophy (PPMA); cachexias such as cardiac cachexia, aids cachexia and cancer cachexia, malnutrition, leprosy, diabetes, renal disease, chronic obstructive pulmonary disease (COPD), cancer, end stage renal failure, sarcopenia, emphysema, osteomalacia, HIV infection, AIDS, and cardiomyopathy.

In addition, other circumstances and conditions are linked to and can cause muscle wasting. These include chronic lower back pain, advanced age, central nervous system (CNS) injury, peripheral nerve injury, spinal cord injury, chemical injury, central nervous system (CNS) damage, peripheral nerve damage, spinal cord damage, chemical damage, burns, disuse deconditioning that occurs when a limb is immobilized, long term hospitalization due to illness or injury, and alcoholism.

An intact androgen receptor (AR) signaling pathway is crucial for appropriate development of skeletal muscles. Furthermore, an intact AR-signaling pathway increases lean muscle mass, muscle strength and muscle protein synthesis.

Muscle wasting, if left unabated, can have dire health consequences. For example, the changes that occur during muscle wasting can lead to a weakened physical state that is detrimental to an individual's health, resulting in increased susceptibility to bone fracture and poor physical performance status. In addition, muscle wasting is a strong predictor of morbidity and mortality in patients suffering from cachexia and AIDS.

New innovative approaches are urgently needed at both the basic science and clinical levels to develop compounds which are useful for a) male contraception; b) treatment of a variety of hormone-related conditions, for example conditions associated with Androgen Decline in Aging Male (ADAM), such as fatigue, depression, decreased libido, sexual dysfunction, erectile dysfunction, hypogonadism, osteoporosis, hair loss, anemia, obesity, sarcopenia, osteopenia, osteoporosis, benign prostate hyperplasia, alterations in mood and cognition and prostate cancer; c) treatment of conditions associated with androgen deficiency in a female (ADIF), such as sexual dysfunction, decreased sexual libido, hypogonadism, sarcopenia, osteopenia, osteoporosis, alterations in cognition and mood, depression, anemia, hair loss, obesity, endometriosis, breast cancer, uterine cancer and ovarian cancer; d) treatment and/or prevention of chronic muscular wasting or sarcopenia; e) decreasing the incidence of, halting or causing a regression of prostate cancer; f) oral androgen replacement and/or other clinical therapeutic and/or diagnostic areas.

A wide variety of diseases and/or conditions are affected by hypogonadism, and catabolic effects, including kidney disease, central nervous system injuries, burns and chronic wounds.

In the United States (US), there is a rising incidence and prevalence of kidney failure. The number of patients enrolled in end-stage renal disease (ESRD) Medicare-funded programs has increased from approximately 10,000 beneficiaries in 1973 to 86,354 in 1983, and to 431,284 as of December 31, 2002. In 2002 alone, 100,359 patients entered the US ESRD program. Chronic kidney disease (CKD) is a precursor to ESRD and occurs when the kidneys are not able to adequately remove wastes from the body. CKD is a slowly progressing disease, in which diabetes, hypertension and anemia may be co-morbid conditions.

CKD is diagnosed using a staging system that demonstrates the amount of kidney function available (stage 1 = normal kidney function) and patients often do not present symptoms in the early stages. Stage 5 of CKD is ESRD, which is a complete or near complete failure of the kidneys and usually occurs when kidney function is less than 10% of baseline.

Accompanying symptoms associated with ESRD include hypogonadism, involuntary weight loss, fatigue and others.

Burns result in a testosterone reduction, nitrogen level reduction and a reduction in bone mineral density (BMD), which may persist even as long as one year following the injury and is associated with impaired wound healing, increased infection risks, erosion of lean body mass, hampered rehabilitation, and delayed reintegration of burn survivors into society. Catabolic effects initiated as a result of the burn lead to significant involuntary weight loss, further compounding the problem.

Spinal cord injuries (SCI) may result in the alteration central neurotransmitter secretion or production, which in turn may cause a hypothalamus-pituitary-adrenal axis dysfunction, leading to decreases in testosterone and other hormone levels. SCI or other acute illness or trauma characteristically includes heightened catabolism in conjunction with the lowered anabolic activity resulting in a condition that is prone to loss of lean body tissue. As long as the catabolic process goes uninterrupted, disturbed nutrient utilization will continue. The effects of the loss of lean body mass include the development of wounds and impaired healing mechanisms. Because of poor nutrition and protein combined with immobilization, patients with spinal cord injury are at high risk for bed sores.

Chronic wounds may be caused by any number of conditions, including diabetes, circulatory problems, immobilization and others. Compounding the problem, for example in diabetes, is the presence of neuropathy, which increases the risk of foot ulceration.

While there are many treatments and therapies for these conditions, none are ideal. Since the androgen receptor (AR) signaling pathway has been shown to increase lean muscle mass, muscle strength and muscle protein synthesis, and since hypogonadism accompanies these conditions, molecules targeting the AR signaling pathway may be useful in treating these diseases and/or conditions.

## SUMMARY OF THE INVENTION

The present invention provides a pharmaceutical composition comprising a compound represented by the structure of formula *S*-(I):

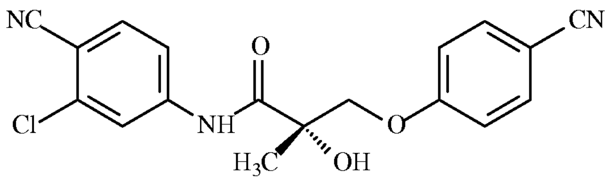

*S*-(I)

or its optical isomer, for use in treating a progestin-responsive tumor in a subject.

In an embodiment, the progestin-responsive tumor is a breast carcinoma, an ovarian carcinoma, a prostate carcinoma, an endometrial carcinoma, a cervical carcinoma, a leiomyosarcoma, or a meningioma. In an embodiment, the progestin-responsive tumor is breast cancer.

The present invention also provides a pharmaceutical composition comprising a compound of formula *S*-(I):

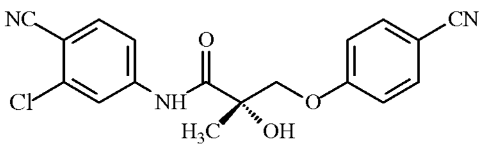

*S*-(I)

or its optical isomer, for use in treating a condition associated with androgen deficiency in aging male (ADAM). In various embodiments, the condition associated with androgen deficiency in aging male (ADAM) is fatigue, depression, decreased libido, sexual dysfunction, erectile dysfunction, hypogonadism, osteoporosis, hair loss, obesity, sarcopenia, osteopenia, benign prostate hyperplasia, or alterations in mood and cognition.

The present invention also provides a pharmaceutical composition comprising a compound of formula *S*-(I):

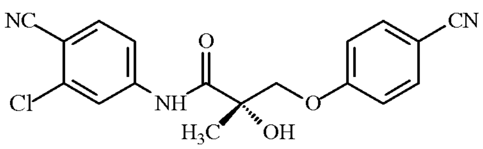

*S*-(I)

or its optical isomer, for use in treating a condition associated with androgen deficiency in a female (ADIF). In various embodiments, the condition associated with androgen deficiency in a female (ADIF) is sexual dysfunction, decreased sexual libido, hypogonadism, sarcopenia, osteopenia, osteoporosis, alterations in cognition and mood, depression, anemia, hair loss, obesity, endometriosis, breast cancer, uterine cancer and ovarian cancer.

As described herein, the compound is a selective androgen receptor modulator (SARM). In one embodiment, the SARM is a partial agonist. In one embodiment, the SARM is a tissue-selective agonist, or in some embodiments, a tissue-selective antagonist.

## BRIEF DESCRIPTION OF THE DRAWINGS

The present invention will be understood and appreciated more fully from the following detailed description taken in conjunction with the appended drawings in which:

**Figure 1:** Synthetic schemes for the preparation of compound of formula **(I). Fig 1A** is a synthetic scheme for the preparation of an (*S*) enantiomer of a compound of formula **(I)** (*S*-(I)). **Fig IB** is a synthetic scheme for the preparation of an (*R*) enantiomer of a compound of formula (**I**) (*R*-(I)). **Fig 1C** is a synthetic scheme for the preparation of an (*S*) enantiomer of a compound of formula **(I)** (*S*-(I)) including an oxirane intermediate. **Fig ID** is a synthetic scheme for the preparation of an (*R*) enantiomer of a compound of formula **(I)** (*R*-(I)) including an oxirane intermediate. **Fig IE** is a synthetic scheme for the preparation of an (*S*) enantiomer of a compound of formula **(I)** (*S*-(I)) involving B-ring addition prior to A-ring addition. **Fig IF** is a synthetic scheme for the preparation of an (*R*) enantiomer of a compound of formula **(I)** (*R*-(I)) involving B-ring addition prior to A-ring addition. **Fig 1G** is a synthetic scheme for the preparation of an (*S*) enantiomer of a compound of formula **(I)** (*S*-(I)) using 2-tribromomethyl-[1,3]dioxolan-4-one intermediate and involving B-ring addition prior to A-ring addition. **Fig 1H** is a synthetic scheme for the preparation of an (*R*) enantiomer of a compound of formula (**I**) (*R*-(I)) using 2-tribromomethyl-[1,3]dioxolan-4-one intermediate and involving B-ring addition prior to A-ring addition. **Fig 1I** is a synthetic scheme for preparation of a racemic mixture of a compound of formula **(I),** involving oxazolidinedione intermediate and B ring addition prior to A ring. **Fig 1J** is a synthetic scheme for preparation of a racemic mixture of a compound of formula **(I),** involving an oxirane intermediate and A ring addition prior to B ring. **Fig 1K** is a synthetic scheme for preparation of a large scale of an (*S*) enantiomer of a compound of formula **(I)** (*S*-(I)). **Fig 1L** is a synthetic scheme for preparation of a large scale of an (S) enantiomer of a compound of formula (I) (S-(I)), including an oxirane intermediate.

**Figure 2:** Effect of *S*-(III) on steroid receptor transactivation (agonist mode).

**Figure 3:** Effect of *S*-(III) on steroid receptor transactivation (antagonist mode).

**Figure 4:** Effect of *S*-(II) on steroid receptor transactivation (agonist mode).

**Figure 5:** Effect of *S*-(II) on steroid receptor transactivation (antagonist mode).

**Figure 6:** Effect of *S*-(I) on steroid receptor transactivation (agonist mode).

**Figure 7:** Effect of *S*-(I) on steroid receptor transactivation (antagonist mode).

**Figure 8:** Anabolic and androgenic activity of compound *S*-(I).

## DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

This invention provides medical uses of a substituted acylanilide characterized by the structure of Formula *S*-(I). In one embodiment, the compound is a SARM. It is disclosed herein that the compound is useful in treating a variety of conditions or diseases, including, *inter alia,* oral testosterone replacement therapy, male contraception, maintaining sexual desire in women, osteoporosis, treating prostate cancer and/or imaging prostate cancer. In some embodiments, the compounds of this invention are nonsteroidal ligands for the AR and exhibit androgenic and/or anabolic activity. In some embodiments, the compounds are partial agonists or partial antagonists in a tissue selective manner. In some embodiments, the compounds are full agonists or full antagonists in a tissue selective manner, which in some embodiments, allows for tissue-selective androgenic and/or anabolic effects. These agents may be active alone or in combination with progestins or estrogens, or other agents, as herein described. In other embodiments, the agents are agonists, antagonists, partial agonists or partial antagonists.

It is also disclosed that the compound, of Formula *S*-(I) is useful in androgen replacement therapy (ART), useful in a) improving body composition; b) increasing bone mineral density (BMD); c) increasing bone mass; d) increasing bone strength; e) improving bone function; f) decreasing fracture risk; g) increasing muscle strength; h) increasing muscle function; i) improving exercise tolerance; j) enhancing libido; k) improving sexual performance; l) improving mood and/or m) improving cognition.

In some embodiments, described herein are synthetic processes of preparation of the SARM compounds of this invention. In some embodiments, the invention provides compositions comprising the selective androgen modulator compounds or use of the same for binding an AR, modulating spermatogenesis, bone formation and/or resorption, treating muscle wasting or diseases associated with muscle wasting, treating prostate cancer, and/or providing hormonal therapy for androgen-dependent conditions.

Described herein is a compound represented by the structure of formula (I):

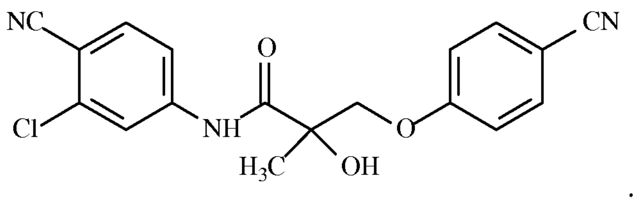

(I)

The present invention relates to medical uses of a compound represented by the structure of formula *S*-(I):

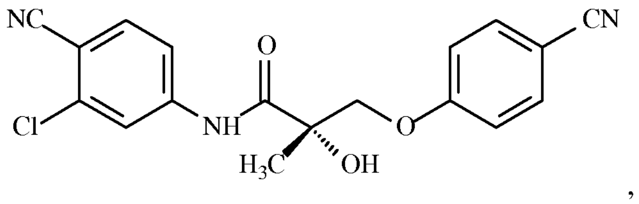

,

*S*-(I)

or its optical isomer.

Also described herein is a compound represented by the structure of formula *R*-(I):

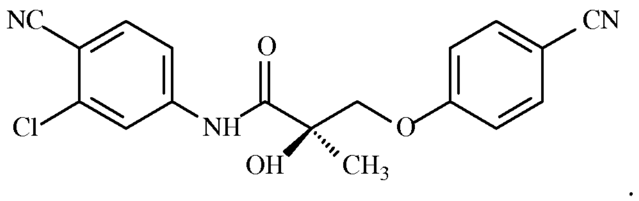

*R*-(I)

The present invention provides a pharmaceutical composition comprising a compound represented by the structure of formula *S*-(I) or its optical isomer, for use in treating a progestin-responsive tumor in a subject. In an embodiment, the progestin-responsive tumor is a breast carcinoma, an ovarian carcinoma, a prostate carcinoma, an endometrial carcinoma, a cervical carcinoma, a leiomyosarcoma, or a meningioma. In an embodiment, the progestin-responsive tumor is breast cancer.

The present invention also provides a pharmaceutical composition comprising a compound of formula *S*-(I) or its optical isomer, for use in treating a condition associated with androgen deficiency in aging male (ADAM). The condition associated with androgen deficiency in aging male (ADAM) may be fatigue, depression, decreased libido, sexual dysfunction, erectile dysfunction, hypogonadism, osteoporosis, hair loss, obesity, sarcopenia, osteopenia, benign prostate hyperplasia, or alterations in mood and cognition.

The present invention also provides a pharmaceutical composition comprising a compound of formula *S*-(I) or its optical isomer, for use in treating a condition associated with androgen deficiency in a female (ADIF). The condition associated with androgen deficiency in a female (ADIF) may be sexual dysfunction, decreased sexual libido, hypogonadism, sarcopenia, osteopenia, osteoporosis, alterations in cognition and mood, depression, anemia, hair loss, obesity, endometriosis, breast cancer, uterine cancer and ovarian cancer.

The invention may utilise "pharmaceutically acceptable salts" of the compounds of formula *S*-(I) of this invention, which may be produced, by reaction of a compound of this invention with an acid or base.

Suitable pharmaceutically acceptable salts of amines of Formula *S*-(I) may be prepared from an inorganic acid or from an organic acid. In one embodiment, examples of inorganic salts of amines are bisulfates, borates, bromides, chlorides, hemisulfates, hydrobromates, hydrochlorates, 2-hydroxyethylsulfonates (hydroxyethanesulfonates), iodates, iodides, isothionates, nitrate, persulfates, phosphates, sulfates, sulfamates, sulfanilates, sulfonic acids (alkylsulfonates, arylsulfonates, halogen substituted alkylsulfonates, halogen substituted arylsulfonates), sulfonates and thiocyanates.

In one embodiment, examples of organic salts of amines comprise aliphatic, cycloaliphatic, aromatic, araliphatic,

heterocyclic, carboxylic and sulfonic classes of organic acids, examples of which are acetates, arginines, aspartates, ascorbates, adipates, anthranilate, alkane carboxylates, substituted alkane carboxylates, alginates, benzenesulfonates, benzoates, bisulfates, butyrates, bicarbonates, bitartrates, carboxylate, citrates, camphorates, camphorsulfonates, cyclohexylsulfamates, cyclopentanepropionates, calcium edetates, camsylates, carbonates, clavulanates, cinnamates, dicarboxylates, digluconates, dodecylsulfonates, dihydrochlorides, decanoates, enanthuates, ethanesulfonates, edetates, edisylates, estolates, esylates, fumarates, formates, fluorides, galacturonate, gluconates, glutamates, glycolates, glucorate, glucoheptanoates, glycerophosphates, gluceptates, glycollylarsanilates, glutarates, glutamate, heptanoates, hexanoates, hydroxymaleates, hydroxycarboxlic acids, hexylresorcinates, hydroxybenzoates, hydroxynaphthoate, hydrofluorate, lactates, lactobionates, laurates, malates, maleates, methylenebis(beta-oxynaphthoate), malonates, mandelates, mesylates, methane sulfonates, methylbromides, methylnitrates, methylsulfonates, monopotassium maleates, mucates, monocarboxylates, naphthalenesulfonates, 2-naphthalenesulfonates, nicotinates, napsylates, N-methylglucamines, oxalates, octanoates, oleates, pamoates, phenylacetates, picrates, phenylbenzoates, pivalates, propionates, phthalates, phenylacetate, pectinates, phenylpropionates, palmitates, pantothenates, polygalacturates, pyruvates, quinates, salicylates, succinates, stearates, sulfanilate, subacetates, tartarates, theophyllineacetates, *p*-toluenesulfonates (tosylates), trifluoroacetates, terephthalates, tannates, teoclates, trihaloacetates, triethiodide, tricarboxylates, undecanoates or valerates.

In one embodiment, examples of inorganic salts of carboxylic acids or phenols comprise ammonium, alkali metals to include lithium, sodium, potassium, cesium; alkaline earth metals to include calcium, magnesium, aluminum; zinc, barium, chlorines or quaternary ammoniums.

In another embodiment, examples of organic salts of carboxylic acids or phenols comprise arginine, organic amines to include aliphatic organic amines, alicyclic organic amines, aromatic organic amines, benzathines, *t*-butylamines, benethamines (N-benzylphenethylamine), dicyclohexylamines, dimethylamines, diethanolamines, ethanolamines, ethylenediamines, hydrabamines, imidazoles, lysines, methylamines, meglamines, *N*-methyl-D-glucamines, *N,N'*-dibenzylethylenediamines, nicotinamides, organic amines, ornithines, pyridines, picolies, piperazines, procain, tris(hydroxymethyl)methylamines, triethylamines, triethanolamines, trimethylamines, tromethamines or ureas.

The salts may be formed by conventional means, such as by reacting the free base or free acid form of the product with one or more equivalents of the appropriate acid or base in a solvent or medium in which the salt is insoluble or in a solvent such as water, which is removed *in vacuo* or by freeze drying or by exchanging the ions of a existing salt for another ion or suitable ion-exchange resin.

This invention may utilise, in other embodiments, pharmaceutical products of the compounds. The term "pharmaceutical product" refers, in other embodiments, to a composition suitable for pharmaceutical use (pharmaceutical composition), for example, as described herein.

In another embodiment, described herein is a process for the preparation of a compound of formula *S*-(**I**). In one embodiment, the first step in such a process comprises that of the scheme below:

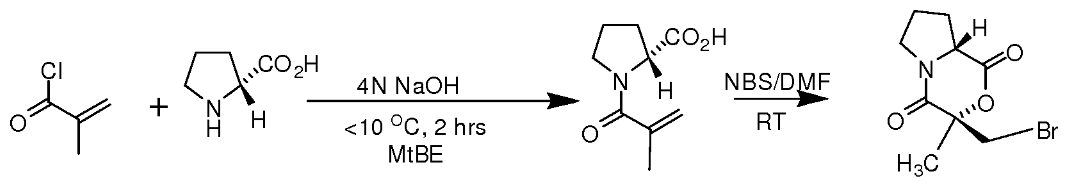


**12**

Figures 1K and 1L provide a process for the preparation of a large scale synthesis of compounds of formulas ***S*-(I)**.

The present application provides a process for preparing a compound represented by the structure of formula *S*-(**I**), as depicted in Figure 1 and Example 1:

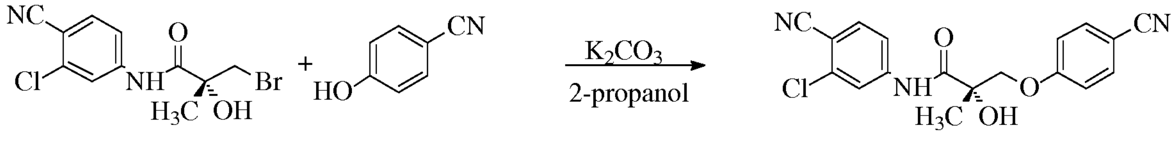


***S*-(I)**

In another embodiment, described herein is a process for preparing an (*S*) enantiomer of a compound represented by the structure of formula S-(I):

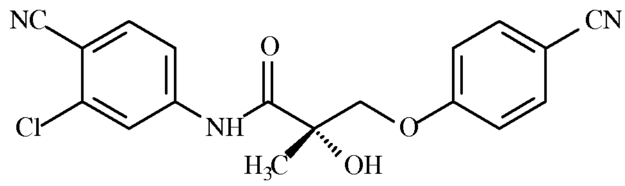

***S*-(I)**

said process comprising the steps of:

a) coupling an amine of formula **17:**

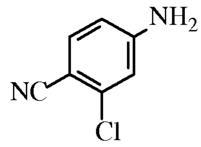

**17**

with the carboxylic acid of formula ***R*-18**

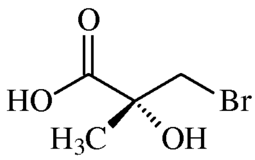

***R*-18**

in the presence of a coupling reagent, to produce an amide of formula ***R*-19**

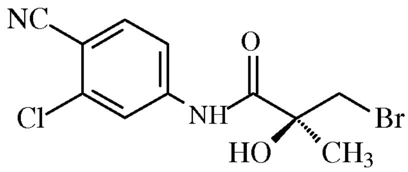

;

***R*-19**

and

b) reacting the amide of formula *R*-**19** with a compound of formula **20:**

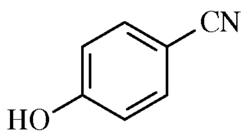

**20**

to produce a compound of formula **S-(I).**

In one embodiment, compound *R*-**18** of step (a) is reacted with a coupling agent prior to addition of compound of formula **17.**

Figure 1A and Example 1 provide one embodiment of a process for the preparation of a compound of formula ***S*-(I).**

In another embodiment, the conditions of step (b) of the process outlined hereinabove may comprise potassium carbonate, sodium carbonate, or cesium carbonate, or another base appropriate for this reaction, using 2-propanol, THF or methylethylketone as a solvent, optionally with a transition catalyst, BTBAC (benzyltributylammonium chloride) or other suitable agent.

In another embodiment, described herein is a process for preparing an (*R*) enantiomer of a compound represented by the structure of formula ***R*-(I):**

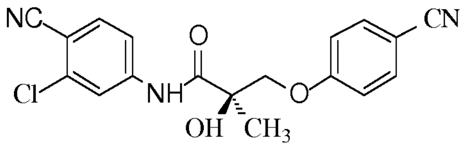

***R*-(I) ;**

said process comprising the steps of:

a) coupling an amine of formula **17:**

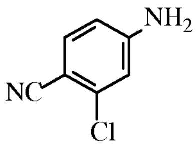

**17**

with the carboxylic acid of formula ***S*-18**

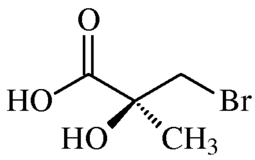

***S*-18**

in the presence of a coupling reagent, to produce an amide of formula ***S*-19**

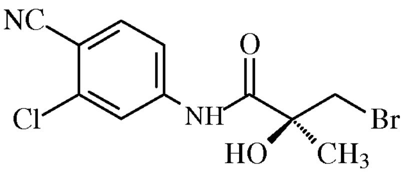

;

***S*-19**

and

b) reacting the amide of formula ***S*-19** with a compound of formula **20**

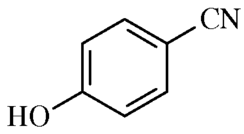

**20**

to produce a compound of ***R*-(I).**

In one embodiment, compound ***S*-18** of step (a) is reacted with a coupling agent prior to addition of compound of formula **17.**

Figure 1B depicts one embodiment of such a process for the preparation of compound of formula ***R*-(I).**

In another embodiment, the conditions of step (b) of the process outlined hereinabove may comprise potassium carbonate, sodium carbonate, or cesium carbonate, or another base appropriate for this reaction, using 2-propanol, THF or methylethylketone as a solvent, optionally with a transition catalyst, BTBAC (benzyltributylammonium chloride) or other suitable agent.

Described herein is a process for preparing an (*S*) enantiomer of a compound represented by the structure of formula ***S*-(I)**

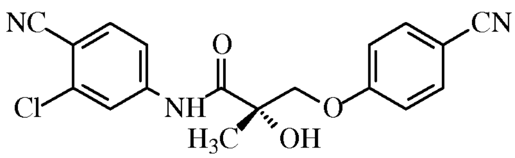

***S*-(I)**

said process comprising the steps of:

a) coupling an amine of formula **17:**

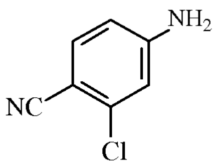

**17**

with the carboxylic acid of formula ***R*-18**

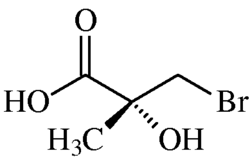

***R*-18**

in the presence of a coupling reagent, to produce an amide of formula ***R*-19**

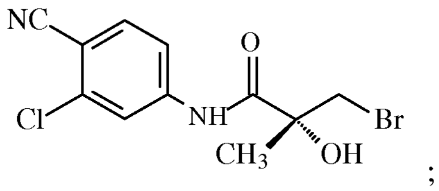

***R*-19**

b) reacting the amide of formula *R*-**19,** with a base to form an oxirane ***S*-21**

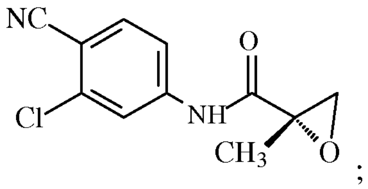

***S*-21**

and

c) reacting the oxirane of formula *S*-**21** with a compound of formula **20:**

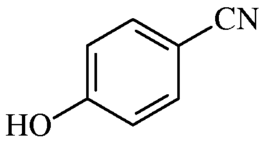

**20**

to produce a compound of ***S*-(I).**

In one embodiment, compound ***R*-18** of step (a) is reacted with a coupling agent prior addition of compound of formula **17.**

Figure 1C depicts an embodiment of such a process for the preparation of compound of formula ***S*-(I).**

Also described herein is a process for preparing an (*R*) enantiomer of compound represented by the structure of formula ***R*-(I):**

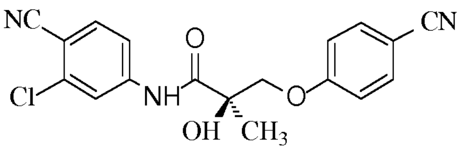

***R*-(I)**

said process comprising the steps of:

a) coupling an amine of formula **17:**

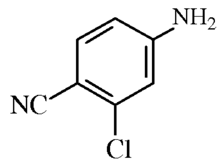

**17**

with the carboxylic acid of formula ***S*-18**

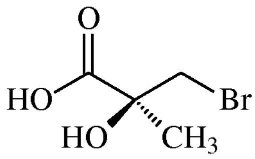

***S*-18**

in the presence of a coupling reagent, to produce an amide of formula ***S*-19**

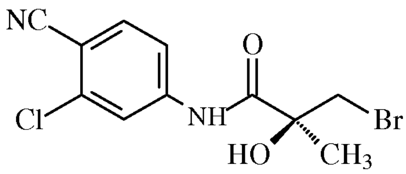

;

***S*-19**

b) reacting the amide of formula ***S*-19,** with a base to form an oxirane ***R*-21**

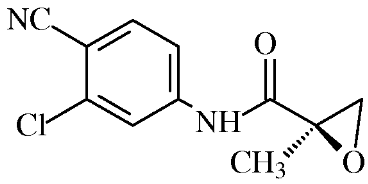

;

and

***R*-21**

c) reacting the oxirane of formula ***R*-21** with a compound of formula **20;**

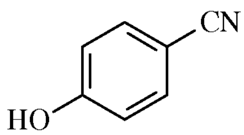

**20**

to produce a compound of ***R*-(I).**

In one embodiment, compound ***S*-18** of step (a) is reacted with a coupling agent prior to addition of compound of formula **17.**

Figure 1D depicts an embodiment of such a process for the preparation of compound of formula ***R*-(I).**

Also described herein is a process for preparing an (S) enantiomer of a compound represented by the structure of formula ***S*-(I)**

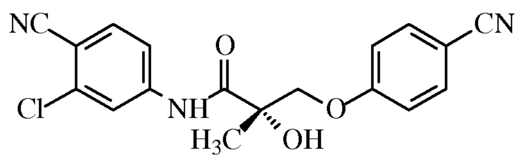

***S*-(I)**

said process comprising the steps of:

a) reacting a ring of formula ***S*-22**

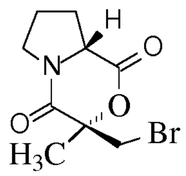

***S*-22**

with a compound of **20**

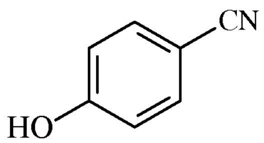

**20**

to produce a compound of formula ***R*-23;**

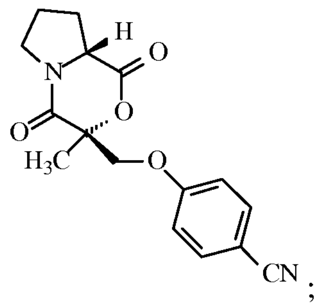

***R*-23**

b) ring opening of compound of formula ***R*-23** to produce a compound of formula ***S*-24**

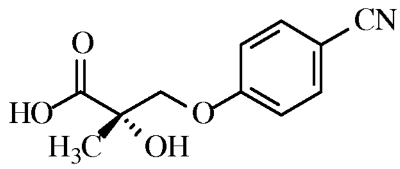

;

***S*-24**

and coupling the carboxylic acid of compound of formula ***S*-24** with the amine of formula **17**

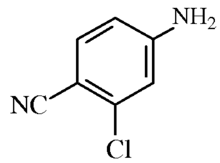

**17**

to produce the compound of formula ***S*-(I)**

Figure IE depicts an embodiment of such a process for the preparation of compound of formula ***S*-(I).**

Further described herein is a process for preparing an (*R*) enantiomer of a compound represented by the structure of formula ***R*-(I):**

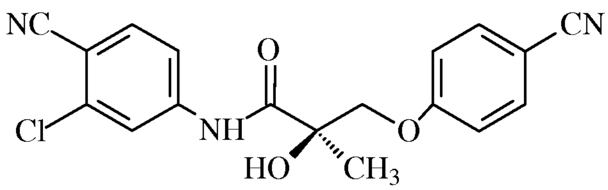

***R*-(I)**

said process comprising the steps of:

a) reacting a ring of formula ***R*-22**

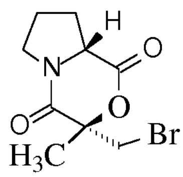

***R*-22**

with a compound of **20**

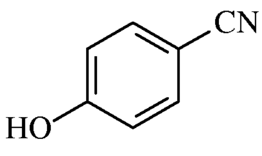

**20**

to produce a compound of formula *S*-23;

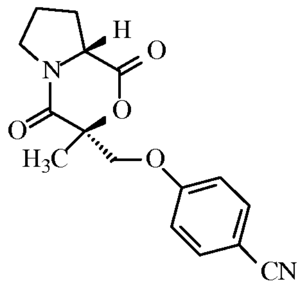

***S*-23**

b) ring opening of compound of formula ***S*-23** to produce a compound of formula ***R*-24:**

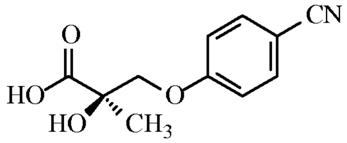

;

***R*-24**

and

c) coupling the carboxylic acid of compound of formula ***R*-24** with the amine of formula 17

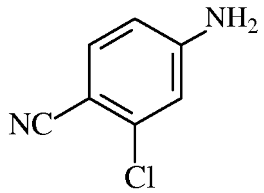

**17**

to produce the compound of formula ***R*-(I).**

Figure 1F depicts an embodiment of such a process for the preparation of compound of formula ***R*-(I).**

Described herein is a process for preparing an (*S*) enantiomer of a compound represented by the structure of formula ***S*-(I)**

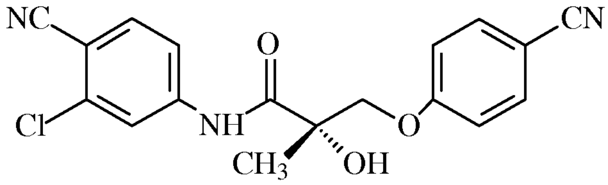

***S*-(I)**

said process comprising the steps of:

a) reacting the carboxylic acid of formula ***R*-18**

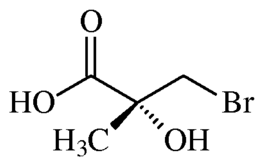

***R*-18**

with tribromoacetaldehyde to produce a compound of formula ***R*-25:**

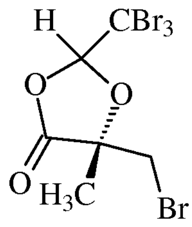

***R*-25**

b) reacting the dioxalane derivative ***R*-25** with a compound of formula **20**

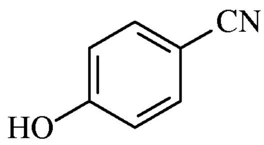

**20**

to produce a compound of formula ***R*-26;**

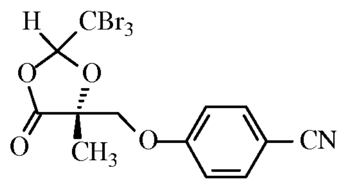

;

***R*-26**

c) ring opening of compound of formula ***R*-26** to produce a compound of formula ***S*-24**

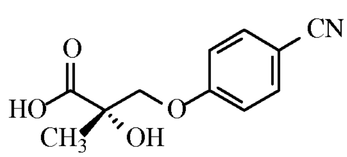

;

***S*-24**

and

d) coupling the carboxylic acid of compound of formula ***S*-24** with the amine of formula **17:**

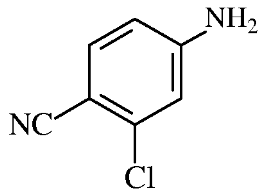

**17**

to produce the compound of formula ***S*-(I).**

Figure 1G depicts an embodiment of such a process for the preparation of compound of formula ***S*-(I).**
Also described herein is a process for preparing an (*R*) enantiomer of a compound represented by the structure of formula ***R*-(I)**

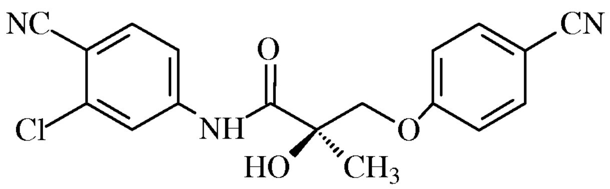

***R*-I**

said process comprising the steps of:

a) reacting the carboxylic acid of formula ***S*-18**

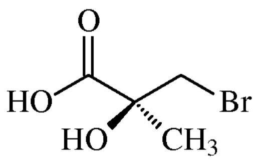

***S*-18**

with tribromoacetaldehyde to produce a compound of formula ***S*-25**:

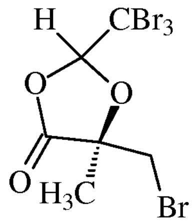

***S*-25**

b) reacting the dioxalane derivative ***S*-25** with a compound of formula **30:**

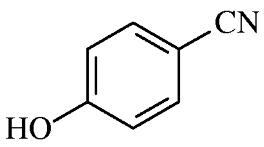

**20**

to produce a compound of formula S-26;

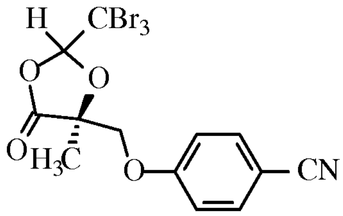

;

***S*-26**

c) ring opening of compound of formula ***S*-26** to produce a compound of formula ***R*-24**

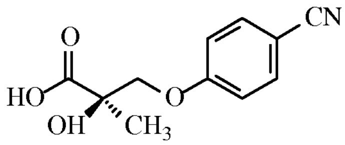

;

***R*-24**

and

d) coupling the carboxylic acid of compound of formula ***R*-24** with the amine of formula **17**:

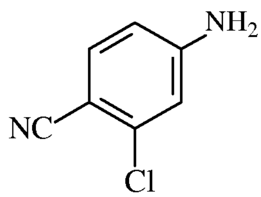

**17**

to produce the compound of formula ***R*-(I).**

Figure 1H depicts an embodiment of such a process for the preparation of compound of formula ***R*-(I).**

Still further described herein is a process for preparing a racemic mixture of a compound represented by the structure of formula **(I)**

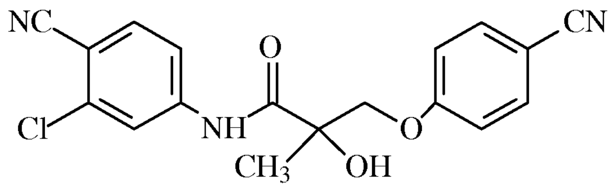

**(I)**

said process comprising the steps of:

a) reacting a compound of formula **24**

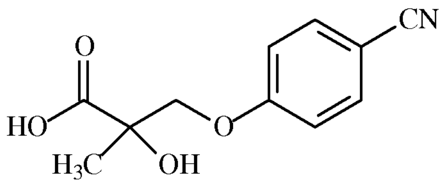

**24**

with a compound of formula **27**

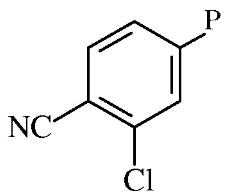

**27**

wherein P is selected from isocyanate (NCO) or isothiocyanate (NCS) to produce a compound of formula **28a** or **28b,** respectively

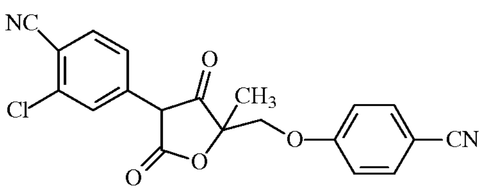

; or

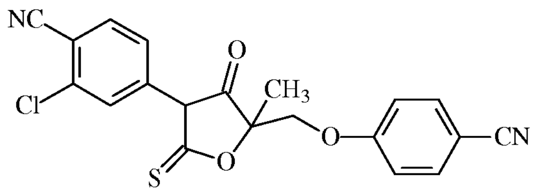

**28a** **28b**

b) ring opening of the oxazolidinedione or 2-thioxooxazolid-4-one ring of formula **28a** or **28b** in a presence of a base to produce a compound of formula **(I).**

Figure 1I depicts an embodiment of such a process for the preparation of racemic compound of formula **(I).**

Also described herein is a process for preparing a racemic mixture of a compound represented by the structure of formula **(I):**

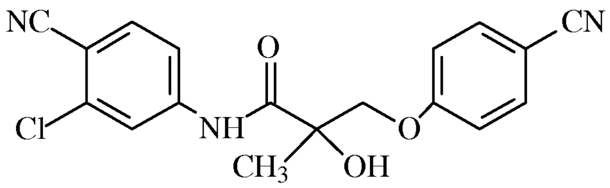

**(I)**

said process comprising the steps of:

a) chlorinating methacrylic acid

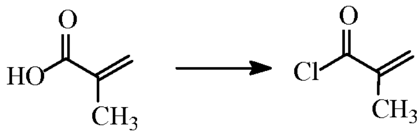

b) coupling an 3-cyano 4-trifluoromethyl aniline of formula 17 with methacryloyl chloride:

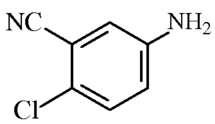

**17**

to produce the amide of formula **29:**

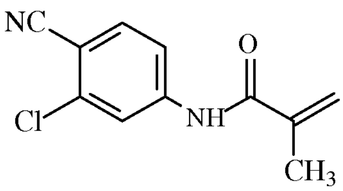

**29**

c) oxidizing an amide of formula **29,** to produce the oxirane of formula **21**

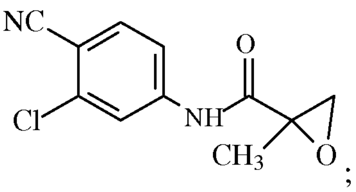

**21**

and

d) reacting the oxirane of formula **21** with a compound of formula **20**

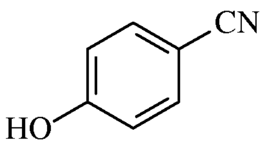

**20**

to produce the compound of formula (I).

In another embodiment, the oxidizing an amide of formula **29** of step (c) comprises ozone. In another embodiment, the oxidizing agent is a peroxyacid, for example, peracetic acid, ($CH_3COOOH$). In another embodiment, the oxidizing agent is *meta*-chloroperbenzoic acid (*m*-CPBA). In another embodiment, the oxidizing agent is Magnesium MonoPeroxyPthalic Acid (MMPP). In another embodiment, the oxidizing agent is hydrogen peroxide together with catalytic amounts (1.0-0.1 mol %) of manganese ($2^+$) salts.

Figure 1J depicts an embodiment of a process for the preparation of racemic compound of formula **(I).**

Further described herein is a process for preparing pure enantiomers of the compounds of this invention, comprising the steps of a) preparing a racemic mixture of a compound described herein; and b) separating pure compounds of this invention from their racemic mixture.

In one embodiment, separation of the optically-active (*R*) isomer or (*S*) enantiomer, from the racemic compounds comprises crystallization techniques. In another embodiment, the crystallization techniques include differential crystallization of enantiomers. In another embodiment, the crystallization techniques include differential crystallization of diastereomeric salts (tartaric salts or quinine salts). In another embodiment, the crystallization techniques include differential crystallization of chiral auxiliary derivatives (menthol esters, etc). In another embodiment, separation of the optically-active (*R*) isomer or (*S*) enantiomer, from the racemic compounds comprises reacting the racemate mixture with another chiral group, forming of a diastereomeric mixture followed by separation of the diastereomers and removing the additional chiral group to obtain pure enantiomers. In another embodiment, separation of the optically-active (*R*) isomer or (*S*) enantiomer, from the racemic mixtures of compounds comprises chiral synthesis. In another embodiment, separation of the optically-active (*R*) isomer or (*S*) enantiomer, from the racemic mixture of the compounds comprises biological resolution. In another embodiment, separation of the optically-active (*R*) isomer or (*S*) enantiomer, from the racemic mixture of the compounds comprises enzymatic resolution. In another embodiment, separation of the optically-active (*R*) isomer or (*S*) enantiomer, from the racemic mixture of the compounds comprises chromatographic separation using a chiral stationary phase. In another embodiment, separation of the optically-active (*R*) isomer or (*S*) enantiomer, from the racemic mixture of the compounds comprises affinity chromatography. In another embodiment, separation of the optically-active (*R*) isomer or (*S*) enantiomer, from the racemic mixture of the compounds comprises capillary electrophoresis. In another embodiment, separation of the optically-active (*R*) isomer or (*S*) enantiomer, from the racemic mixture of the compounds comprises forming an ester group of the hydroxyl group of the chiral carbon with an optically-active acid, for example (-)-camphanic acid, separating the diastereomers esters, thus obtained, by fractional crystallization or preferably, by flash-chromatography, and then hydrolyzing each separate ester to the alcohol.

In another embodiment, the purity, and selectivity of an enantiomer obtained by the process described herein, or by chiral separation of a racemic mixture of this invention can be determined by HPLC analysis.

In one embodiment, the reagent used for reacting the amide derivative, for example compound of formula **19** and the phenol derivative such as for example **20,** are carried out in the presence of a base. Any suitable base that will deprotonate the hydrogen of the -XH moiety (for example, a phenol moiety when X is O) and allow the coupling may be used. Nonlimiting examples of bases are carbonates such as alkali carbonates, for example sodium carbonate ($Na_2CO_3$), potassium carbonate ($K_2CO_3$) and cesium carbonate ($Cs_2CO_3$); bicarbonates such as alkali metal bicarbonates, for example sodium bicarbonate ($NaHCO_3$), potassium bicarbonate ($KHCO_3$), alkali metal hydrides such as sodium hydride (NaH), potassium hydride (KH) and lithium hydride (LiH), and the like.

The leaving group L, according to this aspect, and in one embodiment, may comprise any removable group customarily considered for chemical reactions, as will be known to the person skilled in the art. Suitable leaving groups are halogens, for example F, Cl, Br and I; alkyl sulfonate esters ($-OSO_2R$) wherein R is an alkyl group, for example methanesulfonate (mesylate), trifluoromethanesulfonate, ethanesulfonate, 2,2,2-trifluoroethanesulfonate, perfluoro butanesulfonate; aryl sulfonate esters ($-OSO_2Ar$) wherein Ar is an aryl group, for example p-toluoylsulfonate (tosylate), benzenesulphonate which may be unsubstituted or substituted by methyl, chlorine, bromine, nitro and the like; $NO_3$, $NO_2$, or sulfate, sulfite, phosphate, phosphite, carboxylate, imino ester, $N_2$ or carbamate.

The reaction may be carried out in a suitable inert solvent or diluent such as, for example, tetrahydrofuran, diethyl ether, acetone, methyl ethyl ketone, 2-propanol, aromatic amines such as pyridine; aliphatic and aromatic hydrocarbons such as benzene, toluene, and xylene; dimethylsulfoxide (DMSO), dimethylformamide (DMF), and dimethylacetamide (DMAC). The reaction may be carried out in a suitable inert solvent or diluent as described hereinabove, suitably in the presence of a base such as triethylamine, and at a temperature in the range, as described above. The reaction may be carried out at an appropriate temperature, as will be known to one skilled in the art, for example, in the range, of -20 to 120 °C, or for example at or near ambient temperature.

The coupling reagent defined hereinabove is a reagent capable of turning the carboxylic acid of formula **24** or **18** into a reactive derivative thereof, thus enabling coupling with the respective amine to form an amide bond. A suitable reactive derivative of a carboxylic acid is, for example, an acyl halide / thioacyl halide, for example an acyl chloride formed by the reaction of the acid and an inorganic acid chloride, for example thionyl chloride; a mixed anhydride, for example an anhydride formed by the reaction of the acid and a chloroformate such as isobutyl chloroformate; an active ester, for example an ester formed by the reaction of the acid and a phenol such as pentafluorophenol, an ester such as pentafluorophenyl trifluoroacetate or an alcohol such as methanol, ethanol, isopropanol, butanol or N-hydroxybenzotriazole; an acyl azide, for example an azide formed by the reaction of the acid and azide such as diphenylphosphoryl azide; an acyl cyanide, for example a cyanide formed by the reaction of an acid and a cyanide such as diethylphosphoryl cyanide; or the product of the reaction of the acid and a carbodiimide such as dicyclohexylcarbodiimide.

In one embodiment, the process for preparing a compound of this invention may involve ring opening in the presence of less acidic conditions, which may diminish the likelihood of obtaining the compound mixtures, and provide higher yield and purity of a compound of interest. In one embodiment, the ring opening of a process as described herein, to produce a carboxylic acid of formula **18,** is carried out in the presence of HBr, which, in one embodiment, is at a concentration of up to 30 %, or in another embodiment, of up to 40 %, or in another embodiment, is of up to 25 %, or in another embodiment, of up to 23 %, or in another embodiment, of up to between 20 - 25 %. In one embodiment, the compounds of this invention may be produced via large-scale synthesis, providing highly pure products in high yields.

In one embodiment, the reaction may be carried out in a suitable inert solvent or diluent as described hereinabove, suitably in the presence of a base such as triethylamine, and at a temperature in the range, as described above.

In some embodiments, the compounds as described herein are useful, either alone or as a composition, in males and females for the treatment of a variety of hormone-related conditions, such as hypogonadism, sarcopenia, erectile dysfunction, lack of libido, osteoporosis and fertility.

In one embodiment, the use of the described compound includes contacting or binding a receptor, and thereby mediating the described effects. In some embodiments, the receptor is a nuclear receptor, which in one embodiment, is an androgen receptor, or in another embodiment, is an estrogen receptor, or in another embodiment, is a progesterone receptor, or in another embodiment, is a glucocorticoid receptor. In some embodiments, the multitude of effects may occur simultaneously, as a function of binding to multiple receptors in the subject. In some embodiments, the tissue selective effects of the compounds as described herein provide for simultaneous action on different target organs.

## Pharmaceutical Compositions

In some embodiments, the uses of the present invention comprise administering a composition comprising the described compounds. As used herein, "pharmaceutical composition" means a "therapeutically effective amount" of the active ingredient, i.e. the compound of Formula S-(I), together with a pharmaceutically acceptable carrier or diluent. A "therapeutically effective amount" as used herein refers to that amount which provides a therapeutic effect for a given condition and administration regimen.

As used herein, the term "administering" refers to bringing a subject in contact with a compound of the present invention. As used herein, administration can be accomplished *in vitro,* i.e. in a test tube, or *in vivo,* i.e. in cells or tissues of living organisms, for example humans. In one embodiment, the present invention encompasses administering the compounds of the present invention to a subject.

The pharmaceutical compositions containing the compounds of this invention can be formulated to be administered to a subject by any route known to a person skilled in the art, such as orally, parenterally, intravascularly, paracancerally, transmucosally, transdermally, intramuscularly, intranasally, intravenously, intradermally, subcutaneously, sublingually, intraperitoneally, intraventricularly, intracranially, intravaginally, by inhalation, rectally, intratumorally, or by any means in which the recombinant virus/composition can be delivered to tissue (e.g., needle or catheter). Alternatively, topical administration may be desired for application to mucosal cells, for skin or ocular application. Another route of administration is via aspiration or aerosol formulation.

In one embodiment, the pharmaceutical compositions are to be administered orally, and are thus formulated in a form suitable for oral administration, i.e. as a solid or a liquid preparation. Suitable solid oral formulations include tablets, capsules, pills, granules, pellets, powders, and the like. Suitable liquid oral formulations include solutions, suspensions, dispersions, emulsions, oils and the like. In one embodiment of the present invention, the SARM compounds are formulated in a capsule. In accordance with this embodiment, the compositions of the present invention comprise in addition to a compound of this invention and the inert carrier or diluent, a hard gelatin capsule.

In one embodiment, the micronized capsules comprise particles containing a compound of this invention,

wherein the term "micronized" used herein refers to particles having a particle size is of less than 100 microns, or in another embodiment, less than 60 microns, or in another embodiment, less than 36 microns, or in another embodiment, less than 16 microns, or in another embodiment, less than 10 microns, or in another embodiment, less than 6 microns.

Further, in another embodiment, the pharmaceutical compositions are to be administered by intravenous, intraarterial, or intramuscular injection of a liquid preparation. Suitable liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are to be administered intraarterially, and are thus formulated in a form suitable for intraarterial administration. In another embodiment, the pharmaceutical compositions are to be administered intramuscularly, and are thus formulated in a form suitable for intramuscular administration.

Further, in another embodiment, the pharmaceutical compositions are to be administered topically to body surfaces, and are thus formulated in a form suitable for topical administration. Suitable topical formulations include gels, ointments, creams, lotions, drops and the like. For topical administration, the compounds of this invention or their physiologically tolerated derivatives such as salts, esters, *N*-oxides, and the like are prepared as solutions, suspensions, or emulsions in a physiologically acceptable diluent with or without a pharmaceutical carrier.

Further, in another embodiment, the pharmaceutical compositions are to be administered as a suppository, for example a rectal suppository or a urethral suppository. Further, in another embodiment, the pharmaceutical compositions are to be administered by subcutaneous implantation of a pellet. In a further embodiment, the pellet provides for controlled release of a compound as herein described over a period of time. In a further embodiment, the pharmaceutical compositions are administered intravaginally.

In another embodiment, the active compound can be delivered in a vesicle, in particular a liposome (see Langer, Science 249:1627-1633 (1990); Treat et al., in Liposomes in the Therapy of Infectious Disease and Cancer, Lopez-Berestein and Fidler (eds.), Liss, New York, pp. 363-366 (1989); Lopez-Berestein, ibid., pp. 317-327; see generally ibid).

As used herein "pharmaceutically acceptable carriers or diluents" are well known to those skilled in the art. The carrier or diluent may be a solid carrier or diluent for solid formulations, a liquid carrier or diluent for liquid formulations, or mixtures thereof.

Solid carriers/diluents include a gum, a starch (e.g. corn starch, pregeletanized starch), a sugar (e.g., lactose, mannitol, sucrose, dextrose), a cellulosic material (e.g. microcrystalline cellulose), an acrylate (e.g. polymethylacrylate), calcium carbonate, magnesium oxide, talc, or mixtures thereof.

In one embodiment, the compositions of this invention may include, a compound of this invention, together with one or more pharmaceutically acceptable excipients.

It is to be understood that this invention encompasses any embodiment of a compound as described herein, which in some embodiments is referred to as "a compound of this invention".

Suitable excipients and carriers may be, according to embodiments of the invention, solid or liquid and the type is generally chosen based on the type of administration being used. Liposomes may also be used to deliver the composition. Examples of suitable solid carriers include lactose, sucrose, gelatin and agar. Oral dosage forms may contain suitable binders, lubricants, diluents, disintegrating agents, coloring agents, flavoring agents, flow-inducing agents, and melting agents. Liquid dosage forms may contain, for example, suitable solvents, preservatives, emulsifying agents, suspending agents, diluents, sweeteners, thickeners, and melting agents. Parenteral and intravenous forms should also include minerals and other materials to make them compatible with the type of injection or delivery system chosen. Of course, other excipients may also be used.

For liquid formulations, pharmaceutically acceptable carriers may be aqueous or non-aqueous solutions, suspensions, emulsions or oils. Examples of non-aqueous solvents are propylene glycol, polyethylene glycol, and injectable organic esters such as ethyl oleate. Aqueous carriers include water, alcoholic/aqueous solutions, cyclodextrins, emulsions or suspensions, including saline and buffered media. Examples of oils are those of petroleum, animal, vegetable, or synthetic origin, for example, peanut oil, soybean oil, mineral oil, olive oil, sunflower oil, and fish-liver oil.

Parenteral vehicles (for subcutaneous, intravenous, intraarterial, or intramuscular injection) include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Examples are sterile liquids such as water and oils, with or without the addition of a surfactant and other pharmaceutically acceptable adjuvants. In general, water, saline, aqueous dextrose and related sugar solutions, and glycols such as propylene glycols or polyethylene glycol are preferred liquid carriers, particularly for injectable solutions. Examples of oils are those of petroleum, animal, vegetable, or synthetic origin, for example, peanut oil, soybean oil, mineral oil, olive oil, sunflower oil, and fish-liver oil.

In addition, the compositions may further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCl, acetate, phosphate) of various pH and ionic strength, additives such as albumin or

gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., Cremophor, glycerol, polyethylene glycerol, benzlkonium chloride, benzyl benzoate, cyclodextrins, sobitan esters, stearic acids), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents (e.g. carbomer, colloidal silicon dioxide, ethyl cellulose, guar gum), sweeteners (e.g. aspartame, citric acid), preservatives (e.g., Thimerosal, benzyl alcohol, parabens), coloring agents, lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates), and/or adjuvants.

In one embodiment, the pharmaceutical compositions provided herein are controlled release compositions, i.e. compositions in which the compound of this invention is released over a period of time after administration. Controlled or sustained release compositions include formulation in lipophilic depots (e.g. fatty acids, waxes, oils). In another embodiment, the composition is an immediate release composition, i.e. a composition in which all of the compound is released immediately after administration.

In yet another embodiment, the pharmaceutical composition can be delivered in a controlled release system. For example, the agent may be administered using intravenous infusion, an implantable osmotic pump, a transdermal patch, liposomes, or other modes of administration. In one embodiment, a pump may be used (see Langer, supra; Sefton, CRC Crit. Ref. Biomed. Eng. 14:201 (1987); Buchwald et al., Surgery 88:607 (1980); Saudek et al., N. Engl. J. Med. 321:674 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 116-138 (1984). Other controlled release systems are discussed in the review by Langer (Science 249:1627-1633 (1990).

The compositions may also include incorporation of the active material into or onto particulate preparations of polymeric compounds such as polylactic acid, polglycolic acid, hydrogels, etc., or onto liposomes, microemulsions, micelles, unilamellar or multilamellar vesicles, erythrocyte ghosts, or spheroplasts. Such compositions will influence the physical state, solubility, stability, rate of *in vivo* release, and rate of *in vivo* clearance.

Also comprehended by the invention are particulate compositions coated with polymers (e.g. poloxamers or poloxamines) and the compound coupled to antibodies directed against tissue-specific receptors, ligands or antigens or coupled to ligands of tissue-specific receptors.

Also comprehended by the invention are compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. The modified compounds are known to exhibit substantially longer half-lives in blood following intravenous injection than do the corresponding unmodified compounds (Abuchowski et al., 1981; Newmark et al., 1982; and Katre et al., 1987). Such modifications may also increase the compound's solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-compound abducts less frequently or in lower doses than with the unmodified compound.

The preparation of pharmaceutical compositions which contain an active component is well understood in the art, for example by mixing, granulating, or tablet-forming processes. The active therapeutic ingredient is often mixed with excipients which are pharmaceutically acceptable and compatible with the active ingredient. For oral administration, the compounds of this invention or their physiologically tolerated derivatives such as salts, esters, N-oxides, and the like are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily solutions. For parenteral administration, the compounds of this invention or their physiologically tolerated derivatives such as salts, are converted into a solution, suspension, or emulsion, if desired with the substances customary and suitable for this purpose, for example, solubilizers or other.

An active component can be formulated into the composition as neutralized pharmaceutically acceptable salt forms. Pharmaceutically acceptable salts include the acid addition salts (formed with the free amino groups of the polypeptide or antibody molecule), which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

For use in medicine, the salts of the compound will be pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which

may, for example, be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid.

In one embodiment, this invention provides pharmaceutical compositions a compound *S*-(I) for use in oral testosterone replacement therapy.

In one embodiment, this invention also provides a composition comprising two or more compounds of this invention. The present invention also relates to compositions and pharmaceutical compositions which comprise a compound of this invention alone or in combination with a progestin or estrogen, or in another embodiment, a chemotherapeutic compound, osteogenic or myogenic compound, or other agents suitable for the applications as herein described. In one embodiment, the compositions of this invention will comprise a suitable carrier, diluent or salt.

In one embodiment, the medical uses of this invention may comprise administration of a compound of formula *S*-(I) of this invention at various dosages. In one embodiment, the compound of this invention is to be administered at a dosage of 0.1 - 200 mg per day. In one embodiment, the compound of this invention is to be administered at a dose of 0.1 - 10 mg, or in another embodiment, 0.1 - 26 mg, or in another embodiment, 0.1- 60 mg, or in another embodiment, 0.3 - 16 mg, or in another embodiment, 0.3 - 30 mg, or in another embodiment, 0.6 - 26 mg, or in another embodiment, 0.6 - 60 mg, or in another embodiment, 0.76 - 16 mg, or in another embodiment, 0.76 - 60 mg, or in another embodiment, 1 - 6 mg, or in another embodiment, 1 - 20 mg, or in another embodiment, 3 - 16 mg, or in another embodiment, 30 - 60 mg, or in another embodiment, 30 - 76 mg, or in another embodiment, 100 - 2000 mg.

In one embodiment, the medical uses of this invention may comprise administration of a compound of formula *S*-(I) of this invention at various dosages. In one embodiment, the compound of this invention is to be administered at a dosage of 1 mg. In another embodiment the compound of this invention is to be administered at a dosage of 3 mg, 6 mg, 10 mg, 16 mg, 20 mg, 26 mg, 30 mg, 36 mg, 40 mg, 46 mg, 50 mg, 56 mg, 60 mg, 66 mg, 70 mg, 76 mg, 80 mg, 86 mg, 90 mg, 96 mg or 100 mg.

In one embodiment, the present invention utilizes a pharmaceutical composition comprising a) a compound as described herein; and b) a pharmaceutically acceptable carrier or diluent.

In some embodiments, the present invention utilizes a pharmaceutical composition comprising a) any embodiment of the compound *S*-(I) as described herein; b) a pharmaceutically acceptable carrier or diluent; c) a flow-aid; and d) a lubricant.

In another embodiment, the present invention provides the use of a pharmaceutical composition comprising a) any embodiment of compound *S*-(I) as described herein; b) lactose monohydrate; c) microcrystalline cellulose; d) magnesium stearate; and e) colloidal silicon dioxide.

In some embodiments, compositions comprising compound *S*-(I) offer the advantage that the compounds are nonsteroidal ligands for the androgen receptor, and exhibit anabolic activity *in vivo.* According to this aspect, such compounds are unaccompanied by serious side effects, provide convenient modes of administration, and lower production costs and are orally bioavailable, lack significant cross-reactivity with other undesired steroid receptors, and may possess long biological half-lives.

For administration to mammals, and particularly humans, it is expected that the physician will determine the actual dosage and duration of treatment, which will be most suitable for an individual and can vary with the age, weight and response of the particular individual.

In one embodiment, the compositions for administration may be sterile solutions, or in other embodiments, aqueous or non-aqueous, suspensions or emulsions. In one embodiment, the compositions may comprise propylene glycol, polyethylene glycol, injectable organic esters, for example ethyl oleate, or cyclodextrins. In another embodiment, compositions may also comprise wetting, emulsifying and/or dispersing agents. In another embodiment, the compositions may also comprise sterile water or any other sterile injectable medium.

In one embodiment, the invention utilizes compounds and compositions, for the medical uses of this invention, as described herein. In one embodiment, a composition comprising compound *S*-(I) of this invention will have utility in inhibiting, suppressing, enhancing or stimulating a desired response in a subject, as will be understood by one skilled in the art. In another embodiment, the compositions may further comprise additional active ingredients, whose activity is useful for the particular application for which the compound of this invention is being administered.

In some embodiments, the compositions will further comprise a 5alpha-reductase inhibitors (5ARI), a SARM or SARMs, a selective estrogen receptor modulator (SERM), an aromatase inhibitor, such as but not limited to anastrazole, exemestane, or letrozole; a GnRH agonist or antagonist, a steroidal or nonsteroidal GR ligand, a steroidal or nonsteroidal PR ligand, a steroidal or nonsteroidal AR antagonist, a 17-aldoketoreductase inhibitor or 17β-hydroxysteroid dehydrogenase inhibitor. Such compositions may be used for treating a hormone dependent condition, such as, for example, infertility, neoplasia of a hormone-responsive cancer, for example, a gonadal cancer, or a urogenital cancer.

In some embodiments, the composition will comprise the compounds as described herein, as well as another therapeutic compound, including *inter alia,* a 5ARI such as finasteride, dutasteride, izonsteride; other SARMs, such as, RU-58642, RU-56279, WS9761 A and B, RU-59063, RU-58841, bexlosteride, LG-2293, L-245976, LG-121071, LG-121091, LG-121104, LGD-2226, LGD-2941, YM-92088, YM-175735, LGD-1331, BMS-357597, BMS-391197, S-40503, BMS-482404, EM-4283, EM-4977, BMS-564929, BMS-391197, BMS-434588, BMS-487745, BMS-501949, SA-766, YM-92088, YM-580, LG-123303, LG-123129, PMCol, YM-175735, BMS-591305, BMS-591309, BMS-665139, BMS-665539, CE-590, 116BG33, 154BG31, arcarine, ACP-105; SERMs, such as tamoxifene, 4-hydroxytamoxifene, idoxifene, toremifene, ospemifene, droloxifene, raloxifene, arzoxifene, bazedoxifene, PPT (1,3,5-tris(4-hydroxyphenyl)-4-propyl-1*H*-pyrazole), DPN (diarylpropionitrile), lasofoxifene, pipendoxifene, EM-800, EM-652, nafoxidine, zindoxifene, tesmilifene, miproxifene phosphate, RU 58,688, EM 139, ICI 164,384, ICI 182,780, clomiphene, MER-25, diethylstibestrol, coumestrol, genistein, GW5638, LY353581, zuclomiphene, enclomiphene, delmadinone acetate, DPPE, (*N,N*-diethyl-2-{4-(phenylmethyl)-phenoxy}ethanamine), TSE-424, WAY-070, WAY-292, WAY-818, cyclocommunol, prinaberel, ERB-041, WAY-397, WAY-244, ERB-196, WAY-169122, MF-101, ERb-002, ERB-037, ERB-017, BE-1060, BE-380, BE-381, WAY-358, [18F]FEDNP, LSN-500307, AA-102, Ban zhi lian, CT-101, CT-102, VG-101; GnRH agonists or antagonists, such as, leuprolide, goserelin, triptorelin, alfaprostol, histrelin, detirelix, ganirelix, antide iturelix, cetrorelix, ramorelix, ganirelix, antarelix, teverelix, abarelix, ozarelix, sufugolix, prazarelix, degarelix, NBI-56418, TAK-810, acyline; FSH agonist/antagonist, LH agonist/antagonists, aromatase inhibitors, such as, letrozole, anastrazole, atamestane, fadrozole, minamestane, exemestane, plomestane, liarozole, NKS-01, vorozole, YM-511, fmrozole, 4-hydroxyandrostenedione, aminogluethimide, rogletimide; steroidal or nonsteroidal glucocorticoid receptor ligands, such as, ZK-216348, ZK-243149, ZK-243185, LGD-5552, mifepristone, RPR-106541, ORG-34517, GW-215864X, Sesquicillin, CP-472555, CP-394531, A-222977, AL-438, A-216054, A-276575, CP-394531, CP-409069, UGR-07; steroidal or nonsteroidal progesterone receptor ligands; Steroidal or nonsteroidal AR antagonists such as flutamide, hydroxyflutamide, bicalutamide, nilutamide, hydroxysteroid dehydrogenase inhibitors, PPAR$\alpha$ ligand such as bezafibrate, fenofibrate, gemfibrozil; PPAR$\gamma$ ligands such as darglitazone, pioglitazone, rosiglitazone, isaglitazone, rivoglitazone, netoglitazone; dual acting PPAR ligands , such as naveglitazar, farglitazar, tesaglitazar, ragaglitazar, oxeglitazar, PN-2034, PPAR $\delta$; 17-ketoreductase inhibitors, 3$\beta$-DH$\Delta$4,6-isomerase inhibitors, 3$\beta$-DH$\Delta$4,5-isomerase inhibitors, 17,20 desmolase inhibitors, p450c17 inhibitors, p450ssc inhibitors, 17,20-lyase inhibitors, or combinations thereof.

In some embodiments, the compositions will further comprise ghrelin receptor ligand or growth hormone analogues and secretagogues, IGF-1, IGF-1 analogues and secretagogues, myostatin analogues, proteasome inhibitors, androgenic/anabolic steroid, Enbrel, melanocortin 4 receptor agonist, insulins, or combinations thereof. Such compositions may be used for treating sarcopenia or a musculoskeletal condition.

In some embodiments, the composition will comprise the compounds as described herein, as well as another therapeutic compound, including *inter alia,* ghrelin receptor ligand or growth hormone analogues and secretagogues, such as, pralmorelin, examorelin, tabimorelin, capimorelin, capromorelin, ipamorelin, ep-01572, ep-1572, jmv-1843, an androgenic/anabolic steroid such as testosterone/oxandrolone; a melanocortin 4 receptor agonist , such as bremelanotide; a ghrelin or analogue thereof, such as human ghrelin, CYT-009-GhrQb, L-692429, GHRP-6, SK&F-110679, U-75799E); leptin (metreleptin, pegylated leptin; a leptin receptor agonist, such as LEP(116-130), OB3, [D-Leu4]-OB3, rAAV-leptin, AAV-hOB, rAAVhOB; an insulin (short-, intermediate-, and long acting formulations); a cortisol or corticosteroid, or a combination thereof.

The invention contemplates, in some embodiments, compositions comprising the individual agents, administered separately and by similar or alternative routes, formulated as appropriately for the route of administration. The invention contemplates, in some embodiments, administration of compositions comprising the individual agents, administered in the same formulation. The invention contemplates, in some embodiments, staggered administration, concurrent administration, of administration of the various agents over a course of time, however, their effects are synergistic in the subject.

It is to be understood that any of the above means, timings, routes, or combinations thereof, of administration of two or more agents is to be considered as being encompassed by the phrase "administered in combination", as described herein.

In one embodiment, compound *S*-(I) of this invention is to be administered in combination with an anti-cancer agent. In one embodiment, the anti-cancer agent is a monoclonal antibody. In some embodiments, the monoclonal antibodies are used for diagnosis, monitoring, or treatment of cancer. In one embodiment, monoclonal antibodies react against specific antigens on cancer cells. In one embodiment, the monoclonal antibody acts as a cancer cell receptor antagonist. In one embodiment, monoclonal antibodies enhance the patient's immune response. In one embodiment, monoclonal antibodies act against cell growth factors, thus blocking cancer cell growth. In one embodiment, anti-cancer monoclonal antibodies are conjugated or linked to anti-cancer drugs, radioisotopes, other biologic response modifiers, other toxins, or a combination thereof. In one embodiment, anti-cancer monoclonal antibodies are conjugated or linked to a SARM compound as described hereinabove.

In another embodiment, the present invention includes compounds and compositions in which compound *S*-(I) of the invention is either combined with, or covalently bound to, an agent bound to a targeting agent, such as a monoclonal antibody (e.g., a murine or humanized monoclonal antibody). In one embodiment, the agent bound to a targeting agent is a cytotoxic agent. It will be appreciated that the latter combination may allow the introduction of cytotoxic agents into

for example cancer cells with greater specificity. Thus, the active form of the cytotoxic agent (i.e., the free form) will be present only in cells targeted by the antibody. Of course, the compounds of the invention may also be combined with monoclonal antibodies that have therapeutic activity against cancer.

In one embodiment, the compound *S*-(I) is to be administered in combination with a selective tyrosine kinase inhibitor. In some embodiments, the selective tyrosine kinase inhibitor inhibits catalytic sites of cancer promoting receptors thereby inhibiting tumor growth. In one embodiment, a selective tyrosine kinase inhibitor modulates growth factor signaling. In some embodiments, the selective tyrosine kinase inhibitor targets EGFR (ERB B/HER) family members. In one embodiment, the selective tyrosine kinase inhibitor is a BCR-ABL tyrosine kinase inhibitor. In one embodiment, the selective tyrosine kinase inhibitor is an epidermal growth factor receptor tyrosine kinase inhibitor. In one embodiment, the selective tyrosine kinase inhibitor is a vascular endothelial growth factor (VEGF) tyrosine kinase inhibitor. In one embodiment, the selective tyrosine kinase inhibitor is a Platelet Derived Growth Factor (PDGF) inhibitor.

In one embodiment, the compound *S*-(I) is to be administered in combination with a cancer vaccine. In one embodiment, the cancer vaccine is a therapeutic vaccine thus, treating an existing cancer. In some embodiments, the cancer vaccine is a prophylactic vaccine thus, preventing the development of cancer. In one embodiment, both types of vaccines have the potential to reduce the burden of cancer. In one embodiment, treatment or therapeutic vaccines are administered to cancer patients and are designed to strengthen the body's natural defenses against cancers that have already developed. In one embodiment, therapeutic vaccines may prevent additional growth of existing cancers, prevent the recurrence of treated cancers, or eliminate cancer cells not killed by prior treatments. In some embodiments, prevention or prophylactic vaccines are administered to healthy individuals and are designed to target cancer in individuals who present high risk for the disease. In one embodiment, the cancer vaccine is an antigen/adjuvant vaccine. In one embodiment, the cancer vaccine is a whole cell tumor vaccine. In one embodiment, the cancer vaccine is a dendritic cell vaccine. In one embodiment, the cancer vaccine comprises viral vectors and/or DNA vaccines. In one embodiment, the cancer vaccine is an idiotype vaccine.

In one embodiment, the compound *S*-(I) is to be administered in combination with an anti-cancer chemotherapeutic agent. In one embodiment, the anti-cancer chemotherapeutic agent is an alkylating agent, such as but not limited to cyclophosphamide. In one embodiment, the anti-cancer chemotherapeutic agent is a cytotoxic antibiotic such as but not limited to doxorubicin. In one embodiment, the anti-cancer chemotherapeutic agent is an antimetabolite, such as but not limited to methotrexate. In one embodiment, the anti-cancer chemotherapeutic agent is a vinca alkaloid, such as but not limited to vindesine. In some embodiments, the anti-cancer chemotherapeutic agents include platinum compounds such as but not limited to carboplatin, and taxanes such as docetaxel. In one embodiment, the anti-cancer chemotherapeutic agent is an aromatase inhibitor such as but not limited to anastrazole, exemestane, or letrozole.

In one embodiment, the compound *S*-(I) is to be administered in combination with a Bax activity modulator such as alisol B acetate. In one embodiment, the compound is administered in combination with an angiotensin II receptor blocker such as losartan. In one embodiment, the compound is administered in combination with selenium, green tea cachecins, saw palmetto, lycopene, vitamin D, dietary soy, genistein or isoflavone.

In one embodiment, the compound *S*-(I) is to be administered in combination with antineoplastic agents, such as alkylating agents, antibiotics, hormonal antineoplastics and antimetabolites. Examples of useful alkylating agents include alkyl sulfonates such as busulfan, improsulfan and piposulfan; aziridines, such as a benzodizepa, carboquone, meturedepa and uredepa; ethylenimines and methylmelamines such as altretamine, triethylenemelamine, triethylenephosphoramide, triethylenethiophos-phoramide and trimethylolmelamine; nitrogen mustards such as chlorambucil, chlornaphazine, cyclophosphamide, estramustine, ifosfamide, mechlorethamine, mechlorethamine oxide hydrochloride, melphalan, novembichine, phenesterine, prednimustine, trofosfamide, and uracil mustard; nitroso ureas, such as carmustine, chlorozotocin, fotemustine, lomustine, nimustine, ranimustine, dacarbazine, mannomustine, mitobronitol, mitolactol and pipobroman. More such agents will be known to those having skill in the medicinal chemistry and oncology arts.

In some embodiments, other agents suitable for combination with compound *S*-(I) of this invention include protein synthesis inhibitors such as abrin, aurintricarboxylic acid, chloramphenicol, colicin E3, cycloheximide, diphtheria toxin, edeine A, emetine, erythromycin, ethionine, fluoride, 5-fluorotryptophan, fusidic acid, guanylyl methylene diphosphonate and guanylyl imidodiphosphate, kanamycin, kasugamycin, kirromycin, and O-methyl threonine, modeccin, neomycin, norvaline, pactamycin, paromomycine, puromycin, ricin, α-sarcin, shiga toxin, showdomycin, sparsomycin, spectinomycin, streptomycin, tetracycline, thiostrepton and trimethoprim. Inhibitors of DNA synthesis, including alkylating agents such as dimethyl sulfate, mitomycin C, nitrogen and sulfur mustards, MNNG and NMS; intercalating agents such as acridine dyes, actinomycins, adriamycin, anthracenes, benzopyrene, ethidium bromide, propidium diiodide-intertwining, and agents such as distamycin and netropsin, can also be combined with compounds of the present invention in pharmaceutical compositions. DNA base analogs such as acyclovir, adenine, β-1-D-arabinoside, amethopterin, aminopterin, 2-aminopurine, aphidicolin, 8-azaguanine, azaserine, 6-azauracil, 2'-azido-2'-deoxynucliosides, 5-bromodeoxycytidine, cytosine, β-1-D-arabinoside, diazooxynorleucine, dideoxynucleosides, 5-fluorodeoxycytidine, 5-fluorodeoxyuridine, 5-fluorouracil, hydroxyurea and 6-mercaptopurine also can be used in combination therapies with the compounds of the invention. Topoisomerase inhibitors, such as coumermycin, nalidixic acid, novobiocin and oxolinic acid,

inhibitors of cell division, including colcemide, colchicine, vinblastine and vincristine; and RNA synthesis inhibitors including actinomycin D, α-amanitine and other fungal amatoxins, cordycepin (3'-deoxyadenosine), dichlororibofuranosyl benzimidazole, rifampicine, streptovaricin and streptolydigin also can be combined with the compounds of the invention to provide pharmaceutical compositions.

In one embodiment, the compound *S*-(I) is to be administered in combination with a vaccine for prostate cancer, alisol B acetate, angiotensin II receptor blocker, or others known in the art. In one embodiment, the compound is administered in combination with an agent to decrease prostate (benign or malignant) hypertrophy, such as, for example, selenium, green tea cachecins, saw palmetto, lycopene, vitamin D, dietary soy, genistein and isoflavone food product and others.

In one embodiment, the compound *S*-(I) is to be administered in combination with an immunomodulating agent. In one embodiment, the immunomodulating agent is an immunosuppressive agent. In one embodiment, immunosuppressive agents comprise corticosteroids, cyclosporine, azathioprine, methotrexate, cyclophosphamide, tacrolimus - FK-506, anti-thymocyte globulin, mycophenylate moeftil, or a combination thereof. In one embodiment, the corticosteroid is a glucocorticoid.

In one embodiment, the immunomodulating agent is an immunostimulatory agent. In one embodiment, the immunostimulatory agent is a specific immunostimulator thus, provides antigenic specificity during an immune response, such as a vaccine or any antigen. In one embodiment, the immunostimulatory agent is a non-specific immunostimulator thus, acting irrespective of antigenic specificity to augment immune response of other antigen or stimulate components of the immune system without antigenic specificity. In one embodiment, the non-specific immunostimulator is Freund's complete adjuvant. In one embodiment, the non-specific immunostimulator is Freund's incomplete adjuvant. In one embodiment, the non-specific immunostimulator is a montanide ISA adjuvant. In one embodiment, the non-specific immunostimulator is a Ribi's adjuvant. In one embodiment, the non-specific immunostimulator is a Hunter's TiterMax. In one embodiment, the non-specific immunostimulator is an aluminum salt adjuvant. In one embodiment, the non-specific immunostimulator is a nitrocellulose-adsorbed protein. In one embodiment, the non-specific immunostimulator is a Gerbu Adjuvant.

In one embodiment, the compound *S*-(I) is to be administered in combination with an agent, which treats bone diseases, disorders or conditions, such as osteoporosis, and this invention comprises treating the same, with the compounds as herein described, alone or in combination with other agents.

In one embodiment, bone turnover markers have been demonstrated as an effective, validated tool for the clinical scientist to monitor bone activity. In another embodiment, urinary hydroxyproline, serum alkaline phosphatase, tartrate-resistant acid phosphatase, and osteocalcin levels, along with the urinary calcium-creatinine ratio are used as bone turnover markers. In another embodiment osteocalcin levels is used as a bone formation marker. In another embodiment c-telopeptide is used as a bone resorption marker.

In one embodiment, this invention provides a compound or composition for use in the treatment, prevention, suppression or inhibition of, or the reduction of the risk of developing a skeletal-related event (SRE), such as bone fractures, surgery of the bone, radiation of the bone, spinal cord compression, new bone metastasis, bone loss, or a combination thereof in a subject with cancer. The invention relates, *inter alia* to treatment of an SRE with the compound of formula *S*-(I) in a subject with prostate cancer undergoing or having undergone androgen deprivation therapy (ADT).

In one embodiment, the skeletal-related events treated using the compositions provided herein, are fractures, which in one embodiment, are pathological fractures, non-traumatic fractures, vertebral fracture, non-vertebral fractures, morphometric fractures, or a combination thereof. In some embodiments, fractures may be simple, compound, transverse, greenstick, or comminuted fractures. In one embodiment, fractures may be to any bone in the body, which in one embodiment, is a fracture in any one or more bones of the arm, wrist, hand, finger, leg, ankle, foot, toe, hip, collar bone, or a combination thereof.

In another embodiment, the compositions provided herein are effective in treatment, prevention, suppression, inhibition or reduction of the risk of skeletal-related events such as pathologic fractures, spinal cord compression, hypercalcemia, bone-related pain, or their combination.

In another embodiment, the skeletal-related events sought to be treated using the compositions provided herein, comprise the necessity for bone surgery and/or bone radiation, which in some embodiments, is for the treatment of pain resulting in one embodiment from bone damage, or nerve compression. In another embodiment, the skeletal-related events sought to be treated using the the compositions provided herein, comprise spinal cord compression, or the necessity for changes in antineoplastic therapy, including changes in hormonal therapy, in a subject. In some embodiments, skeletal-related events sought to be treated using the compositions provided herein, comprise treating, suppressing, preventing, reducing the incidence of, or delaying progression or severity of bone metastases, or bone loss. In one embodiment, bone loss may comprise osteoporosis, osteopenia, or a combination thereof. In one embodiment, skeletal-related events may comprise any combination of the embodiments listed herein.

In one embodiment, the compositions provided herein are effective in reducing metastases to the bone, such as in terms of number of foci, the size of foci, or a combination thereof. According to this aspect of the invention and in

one embodiment, provided herein is a composition comprising toremifene, raloxifene, tamoxifen or an analogue, functional derivative, metabolite or a combination thereof, or a pharmaceutically acceptable salt thereof for use in preventing or inhibiting cancer metastasis to bone in a subject. In one embodiment, such metabolites may comprise ospemifene, fispemifene or their combination. In one embodiment, the cancer is prostate cancer.

A person skilled in the art would readily recognize that changes in the antineoplastic therapy utilizing the compositions provided herein may be conducted as a function of, or adjusted or varied as a function of, *inter alia,* the severity of the underlying disease, the source of the underlying disease, the extent of the patients' pain and source of the patients' pain, as well as the stage of the disease. The therapeutic changes may include in certain embodiments, changes in the route of administration (e.g. intracavitarially, intraarterially, intratumorally etc.), forms of the compositions administered (e.g. tablets, elixirs, suspensions etc.), changes in dosage and the like. Each of these changes is well recognized in the art and is encompassed by the embodiments provided herein.

In one embodiment, the skeletal-related events are a result of cancer therapy. In one embodiment, the skeletal-related events are a result of hormone deprivation therapy, while in another embodiment, they are a product of androgen deprivation therapy (ADT).

In one embodiment, the compounds of this invention are useful in prevention or reversal of androgen-deprivation therapy (ADT) induced side effects such as reduced muscle mass, reduced muscle strength, frailty, hypogonadism, osteoporosis, osteopenia, decreased BMD and/or decreased bone mass.

In males, while the natural decline in sex-hormones at maturity (direct decline in androgens as well as lower levels of estrogens derived from peripheral aromatization of androgens) is associated with the frailty of bones, this effect is more pronounced in males who have undergone androgen deprivation therapy.

Such agents for combined use may comprise a SERM, as herein described, a bisphosphonate, for example, alendronate, tiludroate, clodroniate, pamidronate, etidronate, alendronate, zolendronate, cimadronate, neridronate, minodronic acid, ibandronate, risedronate, homoresidronate, a calcitonin, for example, salmon, Elcatonin, SUN-8577, TJN-135; a vitamin D or derivative (ZK-156979); a vitamin D receptor ligand or analogues thereof, such as calcitriol, topitriol, ZK-150123, TEI-9647, BXL-628, Ro-26-9228, BAL-2299, Ro-65-2299, DP-035, an estrogen, estrogen derivative, or conjugated estrogen; an antiestrogen, progestin, synthetic estrogen/progestin; a RANK ligand mAb, for example, denosumab or AMG162 (Amgen); an $\alpha v\beta 3$ integrin receptor antagonist; an osteoclast vacuolar ATPase inhibitor; an antagonist of VEGF binding to osteoclast receptors; a calcium receptor antagonist; PTh (parathyroid hormone) or analogues thereof such as Forteo®, PTHrP analogues (parathyroid hormone-related peptide), cathepsin K inhibitors (AAE581); strontium ranelate; tibolone; HCT-1026, PSK3471, gallium maltolate; Nutropin AQ®; prostaglandins, p38 protein kinase inhibitor; a bone morphogenetic protein (BMP); an inhibitor of BMP antagonism, an. HMG-CoA reductase inhibitor, a vitamin K or derivative, an antiresorptive, an ipriflavone, a fluoride salt, dietary calcium supplement, osteoprotegerin, or any combination thereof. In one embodiment, the combined administration of a SARM as herein described, osteoprotegerin and parathyroid hormone is contemplated for treating any disease, disorder or condition of the bone.

In one embodiment, the immunomodulating agent is an anti-inflammatory agent. In one embodiment, the anti-inflammatory agent is a non-steroidal anti-inflammatory agent. In one embodiment, the non-steroidal anti-inflammatory agent is a cox-1 inhibitor. In one embodiment, the non-steroidal anti-inflammatory agent is a Cox-2 inhibitor. In one embodiment, the non-steroidal anti-inflammatory agent is a Cox-1 and Cox-2 inhibitor. In some embodiments, non-steroidal anti-inflammatory agents include aspirin, salsalate, diflunisal, ibuprofen, fenoprofen, flubiprofen, fenamate, ketoprofen, nabumetone, piroxicam, naproxen, diclofenac, indomethacin, sulindac, tolmetin, etodolac, ketorolac, oxaprozin, or celecoxib. In one embodiment, the anti-inflammatory agent is a steroidal anti-inflammatory agent. In one embodiment, the steroidal anti-inflammatory agent is a corticosteroid.

In one embodiment, the compound is to be administered in with an agent treating the endocrine system. In some embodiments, agents treating the endocrine system include radioactive iodine, antithyroid agent, thyroid hormone supplement, growth hormone, cabergoline, bromocriptine, thyroxine, gonadotropin, glucocorticoid, glucocorticoid analogue, corticotrophin, metyrapone, aminoglutethimide, mitotane, ketoconazole, mifepristone, dexamethasone somatostatin analogue, gonadotropin-releasing hormone analogue, leuprolide, goserelin, antidiuretic hormone, antidiuretic hormone analogue, oxytocin, calcium supplement, vitamin D, or a combination thereof.

In one embodiment, the agent treating the endocrine system is a 5-alpha-reductase inhibitor. In some embodiments, 5-alpha-reductase inhibitors include finasteride, dutasteride, or izonsteride.

In one embodiment, the agent treating the endocrine system is a SARM compound. In some embodiments, SARMs include RU-58642, RU-56279, WS9761 A and B, RU-59063, RU-58841, bexlosteride, LG-2293, L-245976, LG-121071, LG-121091, LG-121104, LGD-2226, LGD-2941, YM-92088, YM-175735, LGD-1331, BMS-357597, BMS-391197, S-40503, BMS-482404, EM-4283, EM-4977, BMS-564929, BMS-391197, BMS-434588, BMS-487745, BMS-501949, SA-766, YM-92088, YM-580, LG-123303, LG-123129, PMCol, YM-175735, BMS-591305, BMS-591309, BMS-665139, BMS-665539, CE-590, 116BG33, 154BG31, arcarine, or ACP-105.

In one embodiment, the additional agent treating the endocrine system is a SERM compound. In some embodiments, SERMs include tamoxifene, 4-hydroxytamoxifene, idoxifene, toremifene, ospemifene, droloxifene, raloxifene,

arzoxifene, bazedoxifene, PPT (1,3,5-tris(4-hydroxyphenyl)-4-propyl-1H-pyrazole), DPN, lasofoxifene, pipendoxifene, EM-800, EM-652, nafoxidine, zindoxifene, tesmilifene, miproxifene phosphate, RU 58,688, EM 139, ICI 164,384, ICI 182,780, clomiphene, MER-25, diethylstibestrol, coumestrol, genistein, GW5638, LY353581, zuclomiphene, enclomiphene, delmadinone acetate, DPPE, (*N,N*-diethyl-2-{4-(phenylmethyl)-phenoxy}ethanamine), TSE-424, WAY-070, WAY-292, WAY-818, cyclocommunol, prinaberel, ERB-041, WAY-397, WAY-244, ERB-196, WAY-169122, MF-101, ERb-002, ERB-037, ERB-017, BE-1060, BE-380, BE-381, WAY-358, [18F]FEDNP, LSN-500307, AA-102, Ban zhi lian, CT-101, CT-102, or VG-101.

In one embodiment, the agent treating the endocrine system is a gonadotropin-releasing hormone agonist or antagonist. In some embodiments, gonadotropin-releasing hormone agonists or antagonists include leuprolide, goserelin, triptorelin, alfaprostol, histrelin, detirelix, ganirelix, antide iturelix, cetrorelix, ramorelix, ganirelix, antarelix, teverelix, abarelix, ozarelix, sufugolix, prazarelix, degarelix, NBI-56418, TAK-810, or acyline.

In one embodiment, the agent treating the endocrine system is a luteinizing hormone agonist or antagonist. In some embodiments, luteinizing hormone agonists or antagonists include letrozole, anastrazole, atamestane, fadrozole, minamestane, exemestane, plomestane, liarozole, NKS-01, vorozole, YM-511, finrozole, 4-hydroxyandrostenedione, aminogluethimide, or rogletimide. In one embodiment, the agent treating the endocrine system is a follicle stimulating hormone agonist or antagonist. In one embodiment, the agent treating the endocrine system is a luteinizing hormone releasing hormone (LHRH) or a LHRH analog.

In one embodiment, the agent treating the endocrine system is a steroidal or nonsteroidal glucocorticoid receptor ligand. In some embodiments, nonsteroidal glucocorticoid receptor ligands include ZK-216348, ZK-243149, ZK-243185, LGD-5552, mifepristone, RPR-106541, ORG-34517, GW-215864X, sesquicillin, CP-472555, CP-394531, A-222977, AL-438, A-216054, A-276575, CP-394531 , CP-409069, or UGR-07.

In one embodiment, the agent treating the endocrine system is a steroidal or non-steroidal progesterone receptor ligand. In one embodiment, the agent treating the endocrine system is a steroidal or nonsteroidal androgen receptor antagonist. In some embodiments, steroidal or nonsteroidal androgen receptor antagonists include flutamide, hydroxyflutamide, bicalutamide, nilutamide, or hydroxysteroid dehydrogenase inhibitor.

In one embodiment, the agent treating the endocrine system is a peroxisome proliferator-activated receptor ligand. In some embodiments, peroxisome proliferator-activated receptor ligands include bezafibrate, fenofibrate, gemfibrozil, darglitazone, pioglitazone, rosiglitazone, isaglitazone, rivoglitazone, netoglitazone, naveglitazar, farglitazar, tesaglitazar, ragaglitazar, oxeglitazar, or PN-2034.

In one embodiment, an agent treating the endocrine system is a human growth hormone. In some embodiments, human growth hormones include somatotropin or analogues.

In one embodiment, the agent treating the endocrine system is a ghrelin. In some embodiments, ghrelins include human ghrelin, CYT-009-GhrQb, L-692429, GHRP-6, SK&F-110679, or U-75799E.

In one embodiment, the agent treating the endocrine system is a leptin. In some embodiments, leptins include metreleptin or pegylated leptin. In one embodiment, an agent treating the endocrine system is a leptin receptor agonist. In some embodiments, leptin receptor agonists include LEP(116-130), OB3, [Δ-Leu4]-OB3, rAAV-leptin, AAV-hOB, or rAAVhOB.

In one embodiment, the SARM compound is to be administered with an inhibitor of an enzyme involved in the androgen biosynthetic pathway. In some embodiments, inhibitors of enzymes involved in the androgen biosynthetic pathway include 17-ketoreductase inhibitor, 3-ΔH4,6-isomerase inhibitor, 3-ΔH4,5-isomerase inhibitor, 17,20 desmolase inhibitor, p450c17 inhibitor, p450ssc inhibitor, or 17,20-lyase inhibitor.

In one embodiment, the SARM compound is to be administered with an agent treating osteoporosis. In some embodiments, osteoporosis is induced by alcohol and/or smoking. In some embodiments, agents treating osteoporosis include SERMs, calcitonin, vitamin D, vitamin D derivatives, vitamin D receptor ligand, vitamin D receptor ligand analogue, estrogen, estrogen derivative, conjugated estrogen, antiestrogen, progestin, synthetic estrogen, synthetic progestin, RANK ligand monoclonal antibody, integrin receptor antagonist, osteoclast vacuolar ATPase inhibitor, antagonist of VEGF binding to osteoclast receptors, calcium receptor antagonist, parathyroid hormone, parathyroid hormone analogue, parathyroid hormone-related peptide, cathepsin K inhibitor, strontium ranelate, tibolone, HCT-1026, PSK3471, gallium maltolate, Nutropin AQ®, prostaglandin, p38 protein kinase inhibitor, bone morphogenetic protein (BMP), inhibitor of BMP antagonism, HMG-CoA reductase inhibitor, vitamin K, vitamin K derivative, ipriflavone, fluoride salts, dietary calcium supplement, or osteoprotegerin.

In one embodiment, the agent treating osteoporosis is a calcitonin. In some embodiments, calcitonins include salmon, elcatonin, SUN-8577, or TJN-135.

In one embodiment, the agent treating osteoporosis is a vitamin D receptor ligand or analogue. In some embodiments, vitamin D receptor ligands or analogues include calcitriol, topitriol, ZK-150123, TEI-9647, BXL-628, Ro-26-9228, BAL-2299, Ro-65-2299, or DP-035.

In one embodiment, the SARM compound is to be administered with an agent treating pharmacotherapy induced hypogonadal and/or osteopenic and/or sarcopenic state. In some embodiments, agents treating pharmacotherapy induced hypogonadal and/or osteopenic and/or sarcopenic states include opioids, narcotics, opiates, opioids, methadone, Kadian, D2 dopamine receptor antagonist, zotepine, haloperidol, amisulpride, risperidone, anti-epileptic agent, valproic acid, carbamazepine, oxcarbamazepine, chemotherapeutic agent, methotrexate, cyclophosphamide, ifosfamide, adriamycin, doxorubicin, glucocorticoids, cyclosporine, L-thyroxine, SERMs, AI, fulvestrant, gonadotropin-releasing hormone agent, androgen deprivation agent, prolactinemia-inducing agent, serotonergic antidepressant, selective serotonin reuptake inhibitor, monoamine oxidase inhibitor, tricyclic antidepressant, antihypertensive agents, methyldopa, reserpine, clonidine, verapamil, antidopaminergic agent, anti-emetic agent, metoclopramide, H2 receptor antagonist, cimetidine, ranitidine, estrogen, or amphetamine.

In one embodiment, the compound of this invention is to be administered with a behavior-modulating agent. In some embodiments, behavior-modulating agents include an anti-anxiety agent, anti-psychotic agent, anti-depressant, beta-blocker, beta-2 agonist, anticholinergic bronchodilator, theophylline, aminophylline, nedocromil sodium, sodium cromoglycate, leukotriene receptor antagonist, corticosteroid, expectorant, mucolytic agent, antihistamine, pseudoephedrine, methylphenidate, amphetamine, buspirone, benzodiazepine, dextroamphetamine, tricyclic antidepressant, serotonin reuptake inhibitor, phenothiazines, benztropine, bupropion, propranolol, lithium, venlafaxine, haloperidol, buspirone, or a neuraminidase inhibitor.

In one embodiment, the behavior-modulating agent is a benzodiazepine. In one embodiment, benzodiazepines comprise alprazolam, chlordiazepoxide, diazepam, flurazepam, lorazepam, oxazepam, temazepam, or triazolam.

In one embodiment, the behavior-modulating agent is a phenothiazine. In one embodiment, phenothiazines comprise fluphenazine, perphenazine, thioridazine, or trifluoperazine.

In one embodiment, the behavior-modulating agent is a tricyclic antidepressant or a serotonin reuptake inhibitor. In one embodiment, tricyclic antidepressants or serotonin reuptake inhibitors comprise phenothiazine, protriptyline, fluoxetine, paroxetine, or sertraline.

In some embodiments, any of the compositions of this invention will comprise a compound of formula S-(I), in any form or embodiment as described herein. In some embodiments, any of the compositions of this invention will consist of a compound of formula S-(I), in any form or embodiment as described herein. In some embodiments, of the compositions of this invention will consist essentially of a compound of S-(I), in any form or embodiment as described herein. In some embodiments, the term "comprise" refers to the inclusion of the indicated active agent, such as the compound of formula S-(I), as well as inclusion of other active agents, and pharmaceutically acceptable carriers, excipients, emollients, stabilizers, etc., as are known in the pharmaceutical industry. In some embodiments, the term "consisting essentially of' refers to a composition, whose only active ingredient is the indicated active ingredient, however, other compounds may be included which are for stabilizing, preserving, etc. the formulation, but are not involved directly in the therapeutic effect of the indicated active ingredient. In some embodiments, the term "consisting essentially of' may refer to components which facilitate the release of the active ingredient. In some embodiments, the term "consisting" refers to a composition, which contains the active ingredient and a pharmaceutically acceptable carrier or excipient.

### *Biological Activity of Selective Androgen Modulator Compounds*

The compounds of this invention may be useful, in some embodiments, for oral testosterone replacement therapy. In other embodiments, appropriately substituted compounds are useful for a) male contraception; b) treatment of a variety of hormone-related conditions, for example conditions associated with ADAM, such as fatigue, depression, decreased libido, sexual dysfunction, erectile dysfunction, hypogonadism, osteoporosis, hair loss, obesity, sarcopenia, osteopenia, benign prostate hyperplasia, and alterations in mood and cognition; c) treatment of conditions associated with ADIF, such as sexual dysfunction, decreased sexual libido, hypogonadism, sarcopenia, osteopenia, osteoporosis, alterations in cognition and mood, depression, anemia, hair loss, obesity, endometriosis, breast cancer, uterine cancer and ovarian cancer; d) treatment and/or prevention of chronic muscular wasting; e) treatment of prostate cancer, imaging of prostate cancer; decreasing the incidence of, halting or causing a regression of prostate cancer; f) treatment of diabetes type I; g) treatment of diabetes type II; h) suppressing or inhibiting or reducing the incidence of diabetes i) treatment of glucose intolerance; j) treatment of hyperinsulinemia; k) treatment of insulin resistance l) treatment of diabetic nephropathy; m) treatment of diabetic neuropathy; n) treatment of diabetic retinopathy; o) treatment of fatty liver condition; p) treatment of cachexia; q) oral androgen replacement and/or other clinical therapeutic and/or diagnostic areas, including any embodiment of what is encompassed by the term "treating" as described herein.

In some embodiments, the compounds of this invention possess *in vivo* tissue selective androgenic and anabolic activity, which is accordingly utilized for particular applications, as will be appreciated by one skilled in the art.

In some embodiments, the compound *S*-(I) as described herein and/or compositions comprising the same are for use in treating diseases in which the improvement of cognition, or reduction or treatment of depression, are desired.

In one embodiment, the compositions comprising compound *S*-(I) of this invention are for use in a subject, which is a human. In another embodiment, the subject is a mammal.

In one embodiment, the subject is male. In another embodiment, the subject is female. In some embodiments,

while the compositions comprising compound *S*-(I) as described herein may be useful for treating either males or females, females may respond more advantageously to administration of certain compounds, for certain uses, as described and exemplified herein.

In some embodiments, while the compositions comprising compound *S*-(I) as described herein may be useful for treating either males or females, males may respond more advantageously to administration of certain compounds, for certain uses, as described herein.

In some embodiments, the compositions comprising compound *S*-(I) are for use in treating diseases and/or conditions associated with problems with a subject's libido, or erectile dysfunction in a subject. In one embodiment, "libido", may refer to sexual desire.

In one embodiment, the term "erectile" refers to the ability to be erect or upright. An erectile tissue is a tissue, which is capable of being greatly dilated and made rigid by the distension of the numerous blood vessels, which it contains.

In another embodiment of the present invention, the compositions comprising compound S-(I) are for use in hormonal therapy in a patient (i.e., one suffering from an androgen-dependent condition) which includes contacting an androgen receptor of a patient with a compound and/or a non steroidal agonist of the present invention in an amount effective to bind the compound to the androgen receptor and effect a change in an androgen-dependent condition.

In one embodiment of this invention, the compositions comprising compound *S*-(I) are for use in hormone replacement therapy in a patient (i.e., one suffering from an androgen-dependent condition) which includes administering a compound as herein described to a subject, in an amount sufficient to effect a change in a hormone-dependent condition in the subject.

Androgen-dependent conditions which may be treated with the compounds and/or compositions as herein described, include those conditions which are associated with aging, hypogonadism, sarcopenia, diminished erythropoiesis, osteoporosis, and any other conditions dependent upon low androgen (e.g., testosterone) or estrogen levels.

Androgen-dependent conditions which may be treated with the compounds and/or compositions as herein described, may comprise conditions characterized by elevated androgen or estrogen levels, including hirsutism, infertility, polycystic ovarian syndrome, endometrial carcinoma, breast cancer, male pattern baldness, prostate cancer, testicular cancer, and others, as will be known to one skilled in the art. For such conditions, the subject may be administered a compound as herein described, alone or in combination with another therapeutic agent, as will be appreciated by one skilled in the art.

Compositions are described comprising compound *S*-(I) for use in the treatment of a progeston-responsive cancer in a subject, reduction of incidence or severity or pathogenesis of a progeston-responsive cancer in a subject, or delaying progression, prolonging remission or delaying onset of progeston-responsive cancer in a subject. In some embodiments, such cancers are hormone-dependent or androgen receptor dependent tumors (malignant or benign) associated with reproductive tissue in males or females, such as cancer of the prostate, ovary, breast, uterus, testicle, or others.

Compositions comprising compound *S*-(I) are useful for the treatment of a precancerous precursor or lesion in a subject, or reduction of incidence of precancerous precursors or lesions in a subject. Such precancerous precursors may be androgen receptor dependent tumors found in hormone-responsive tissue or are associated with reproductive tissue in males or females, such as in the prostate, ovary, breast, uterus, testicle, or others. In some embodiments, such precancerous precursors comprise any local intraepithelial neoplasia, for example, of the prostate, the cervix, etc. In some embodiments, such compounds and compositions are useful in treating neoplasia or pre-neoplasia, dysplasia or hyperplasia in a tissue, such as in reproductive tissue in males or females.

Compositions comprising compound *S*-(I) are for use in in treating benign prostate hyperplasia (BPH). "BPH (benign prostate hyperplasia)" is a nonmalignant enlargement of the prostate gland, and is the most common non-malignant proliferative abnormality found in any internal organ and the major cause of morbidity in the adult male. BPH occurs in over 75% of men over 50 years of age, reaching 88% prevalence by the ninth decade. BPH frequently results in a gradual squeezing of the portion of the urethra which traverses the prostate (prostatic urethra). This causes patients to experience a frequent urge to urinate because of incomplete emptying of the bladder and urgency of urination. The obstruction of urinary flow can also lead to a general lack of control over urination, including difficulty initiating urination when desired, as well as difficulty in preventing urinary flow because of the inability to empty urine from the bladder, a condition known as overflow urinary incontinence, which can lead to urinary obstruction and to urinary failure.

Compositions are described for use in reducing the severity of, reducing the incidence of, or reducing pathogenesis of progestin-responsive cancer. In another embodiment, the cancer comprises androgen AR dependent tumors (malignant or benign) such as prostate cancer, breast cancer (male or female, operable or inoperable). In another embodiment the SARM compounds adjunct to ADT for treating prostate cancer; bladder cancers; brain cancers; bone tumors, colon cancer, endometrial cancer, liver cancer, lung cancer, lymphatic cancer , kidney cancer, osteosarcoma cancer, ovarian cancer, pancreas cancer, penis cancer, skin cancer, thyroid cancer; and/or hormone-dependent cancers.

Compositions comprising compound *S*-(I) are for use in treating osteoporosis or osteopenia.

"Osteoporosis" refers, in one embodiment, to a thinning of the bones with reduction in bone mass due to

depletion of calcium and bone protein. In another embodiment, osteoporosis is a systemic skeletal disease, characterized by low bone mass and deterioration of bone tissue, with a consequent increase in bone fragility and susceptibility to fracture. In osteoporotic patients, bone strength is abnormal, in one embodiment, with a resulting increase in the risk of fracture. In another embodiment, osteoporosis depletes both the calcium and the protein collagen normally found in the bone, in one embodiment, resulting in either abnormal bone quality or decreased bone density. In another embodiment, bones that are affected by osteoporosis can fracture with only a minor fall or injury that normally would not cause a bone fracture. The fracture can be, in one embodiment, either in the form of cracking (as in a hip fracture) or collapsing (as in a compression fracture of the spine). The spine, hips, and wrists are common areas of osteoporosis-induced bone fractures, although fractures can also occur in other skeletal areas. Unchecked osteoporosis can lead, in another embodiment, to changes in posture, physical abnormality, and decreased mobility.

In one embodiment, the osteoporosis results from androgen deprivation. In another embodiment, the osteoporosis follows androgen deprivation. In another embodiment, the osteoporosis is primary osteoporosis. In another embodiment, the osteoporosis is secondary osteoporosis. In another embodiment, the osteoporosis is postmenopausal osteoporosis. In another embodiment, the osteoporosis is juvenile osteoporosis. In another embodiment, the osteoporosis is idiopathic osteoporosis. In another embodiment, the osteoporosis is senile osteoporosis.

In another embodiment, the primary osteoporosis is type I primary osteoporosis. In another embodiment, the primary osteoporosis is type II primary osteoporosis.

The bone-related disorder is treated with a compound as herein described, or a combination thereof. In another embodiment, other bone-stimulating compounds can be provided to the subject, prior to, concurrent with or following administration of a compound or compounds as herein described. In one embodiment, such a bone stimulating compound may comprise natural or synthetic materials.

In one embodiment, the bone stimulating compound may comprise a bone morphogenetic protein (BMP), a growth factor, such as epidermal growth factor (EGF), a fibroblast growth factor (FGF), a transforming growth factor (TGF, an insulin growth factor (IGF), a platelet-derived growth factor (PDGF) hedgehog proteins such as *sonic, indian* and *desert* hedgehog, a hormone such as follicle stimulating hormone, parathyroid hormone, parathyroid hormone related peptide, activins, inhibins, follistatin, *frizzled, frzb* or *frazzled* proteins, BMP binding proteins such as chordin and fetuin, a cytokine such as IL-3, IL-7, GM-CSF, a chemokine, such as eotaxin, a collagen, osteocalcin, osteonectin and others, as will be appreciated by one skilled in the art.

In another embodiment, the compositions for use in treating a bone disorder may comprise a compound or compounds as herein described, an additional bone stimulating compound, or compounds, and osteogenic cells. In one embodiment, an osteogenic cell may be a stem cell or progenitor cell, which may be induced to differentiate into an osteoblast. In another embodiment, the cell may be an osteoblast. In another embodiment, nucleic acids which encode bone-stimulating compounds may be administered to the subject.

The compositions are for use in treating osteoporosis. In another embodiment, the compounds and compositions of this invention may be administered in combination with SERMs for treating osteoporosis. In another embodiment, the SERMs are tamoxifene, 4-hydroxytamoxifene, idoxifene, toremifene, ospemifene, droloxifene, raloxifene, arzoxifene, bazedoxifene, PPT (1,3,5-tris(4-hydroxyphenyl)-4-propyl-1H-pyrazole), DPN, lasofoxifene, pipendoxifene, EM-800, EM-652, nafoxidine, zindoxifene, tesmilifene, miproxifene phosphate, RU 58,688, EM 139, ICI 164,384, ICI 182,780, clomiphene, MER-25, diethylstibestrol, coumestrol, genistein, GW5638, LY353581, zuclomiphene, enclomiphene, delmadinone acetate, DPPE, (*N,N*-diethyl-2-{4-(phenylmethyl)-phenoxy}ethanamine), TSE-424, WAY-070, WAY-292, WAY-818, cyclocommunol, prinaberel, ERB-041, WAY-397, WAY-244, ERB-196, WAY-169122, MF-101, ERb-002, ERB-037, ERB-017, BE-1060, BE-380, BE-381, WAY-358, [18F]FEDNP, LSN-500307, AA-102, Ban zhi lian, CT-101, CT-102, or VG-101.

In another embodiment, the compositions may be administered in combination with bisphosphonates such as alendronate, tiludroate, clodroniate, pamidronate, etidronate, alendronate, zolendronate, cimadronate, neridronate, minodronic acid, ibandronate, risedronate, or homoresidronate for treating osteoporosis.

In another embodiment, the compositions of the present invention may be administered in combination with calcitonin such as salmon, Elcatonin, SUN-8577 or TJN-135 for treating osteoporosis.

The compositions for use in treating osteoporosis may be administered in combination with a) vitamin D or derivative such as ZK-156979; b) vitamin D receptor ligand and analogues such as calcitriol, topitriol, ZK-150123, TEI-9647, BXL-628, Ro-26-9228, BAL-2299, Ro-65-2299 or DP-035; c) estrogen, estrogen derivative, or conjugated estrogens; d) antiestrogen, progestins, or synthetic estrogen/progestins; e) RANK ligand mAb such as denosumab formerly AMG162 (Amgen); f) $\alpha v\beta 3$ integrin receptor antagonist; g) osteoclast vacuolar ATPase inhibitor; h) antagonist of VEGF binding to osteoclast receptors; i) calcium receptor antagonist; j) PTh (parathyroid hormone) and analogues, PTHrP analogues (parathyroid hormone-related peptide); k) cathepsin K inhibitors (AAE581, etc.); 1) strontium ranelate; m) tibolone; n) HCT-1026, PSK3471; o) gallium maltolate; p) Nutropin AQ; q) prostaglandins (for osteo); r) p38 protein kinase inhibitor; s) bone morphogenetic protein; t) inhibitor of BMP antagonism; u) HMG-CoA reductase inhibitor; v) vitamin K or derivative; w) ipriflavone; x) fluoride salts; y) dietary calcium supplement, and z) osteoprotegerin.

Compositions comprising compound *S*-(I) are for use in treating diseases or disorders caused by, or associated with a hormonal disorder, disruption or imbalance. In one embodiment, the hormonal disorder, disruption or imbalance comprises an excess of a hormone. In another embodiment, the hormonal disorder, disruption or imbalance comprises a deficiency of a hormone. In one embodiment, the hormone is a steroid hormone. In another embodiment, the hormone is an estrogen. In another embodiment, the hormone is an androgen. In another embodiment, the hormone is a glucocorticoid. In another embodiment, the hormone is a cortico-steroid. In another embodiment, the hormone is luteinizing hormone (LH). In another embodiment, the hormone is follicle stimulating hormone (FSH). In another embodiment, the hormone is any other hormone known in the art. In another embodiment, the hormonal disorder, disruption or imbalance is associated with menopause. In another embodiment, the hormonal disorder, disruption or imbalance is associated with andropause, andropausal vasomotor symptoms, andropausal gynecomastia, decreased muscle strength and/or function, decreased bone strength and/or function and anger. In another embodiment, hormone deficiency is a result of specific manipulation, as a byproduct of treating a disease or disorder in the subject. For example, the hormone deficiency may be a result of androgen depletion in a subject, as a therapy for prostate cancer in the subject.

In another embodiment the invention is directed to compositions for use in treating sarcopenia or cachexia, and associated conditions related thereto, for example diseases or disorders of the bone.

In another embodiment, it is described a composition comprising compound *S*-(I) for use in treating a subject having sarcopenia. In another embodiment, the composition may be administered intravenously, intraarterially, or intramuscularly injecting to said subject said pharmaceutical composition in liquid form; subcutaneously implanting in said subject a pellet containing said pharmaceutical composition; orally administering to said subject said pharmaceutical composition in a liquid or solid form; or topically applying to the skin surface of said subject said pharmaceutical composition.

Sarcopenia is a debilitating disease that afflicts the elderly and chronically ill patients and is characterized by loss of muscle mass and function. Further, increased lean body mass is associated with decreased morbidity and mortality for certain muscle-wasting disorders. In addition, other circumstances and conditions are linked to, and can cause muscle wasting disorders. For example, studies have shown that in severe cases of chronic lower back pain, there is paraspinal muscle wasting.

Muscle wasting and other tissue wasting is also associated with advanced age. It is believed that general weakness in old age is due to muscle wasting. As the body ages, an increasing proportion of skeletal muscle is replaced by fibrous tissue. The result is a significant reduction in muscle power, performance and endurance.

Long term hospitalization due to illness or injury, or disuse deconditioning that occurs, for example, when a limb is immobilized, can also lead to muscle wasting, or wasting of other tissue. Studies have shown that in patients suffering injuries, chronic illnesses, burns, trauma or cancer, who are hospitalized for long periods of time, there is a long-lasting unilateral muscle wasting, and a decrease in body mass.

The present disclosure provides a composition comprising compound *S*-(I) for use in a method of treating, reducing the incidence, delaying the onset or progression, or reducing and/or abrogating the symptoms associated with an endocrine disorder in a subject. In one embodiment, the method comprises administering to a subject a composition comprising a compound and anti-cancer agent, an immunomodulating agent, an antidiabetic agent, an agent treating the cardiovascular system, an agent treating the gastrointestinal system, an agent treating a dermatological disorder, an agent treating the central nervous system, an anti-infective agent, an agent treating the liver, an agent treating the kidney, an agent treating a metabolic disease, an agent treating a wasting disease, a gene therapy agent, an agent treating the endocrine system, vitamins, or a combination thereof. In some embodiments, endocrine disorders comprise acromegaly, Addison disease, adrenal gland diseases, adrenal hyperplasia, congenital, androgen-insensitivity syndrome, congenital hypothyroidism, Cushing syndrome, diabetes insipidus, diabetes mellitus, diabetes mellitus-type 1, diabetes mellitus-type 2, diabetic, ketoacidosis, empty Sella syndrome, endocrine gland neoplasms, endocrine system diseases, gigantism, gonadal disorders, graves disease, hermaphroditism, hyperaldosteronism, hyperglycemic hyperosmolar nonketotic coma, hyperpituitarism, hyperprolactinemia, hyperthyroidism, hypogonadism, hypopituitarism, hypothyroidism, Kallmann syndrome, Nelson syndrome, parathyroid diseases, pituitary diseases, polyendocrinopathies, autoimmune, puberty, delayed, puberty, precocious, renal osteodystrophy, thyroid diseases, thyroid hormone resistance syndrome, thyroid neoplasms, thyroid nodule, thyroiditis, thyroiditis, autoimmune, thyroiditis, subacute, or Wolfram syndrome.

In one embodiment, "Hypogonadism" is a condition resulting from or characterized by abnormally decreased functional activity of the gonads, with retardation of growth and sexual development.

In some embodiments, the present invention provides a composition for use in treating, reducing the incidence, delaying the onset or progression, or reducing and/or abrogating the symptoms associated with osteopenic state in a subject. In some embodiments, osteopenia is a mild thinning of the bone mass. In some embodiments, osteopenia is a precursor to osteoporosis. In some embodiments osteopenia is defined as a bone density between one standard deviation (SD) and 2.5 SD below the bone density of a normal young adult.

In some embodiments, it is described a composition for use in treating, reducing the incidence, delaying the

onset or progression, or reducing and/or abrogating the symptoms associated with a sarcopenic state in a subject. In some embodiments, sarcopenia is a significant loss of muscle mass. In one embodiment, sarcopenia definition is having a lean body mass less than two standard deviation below the mean for normal young adults. In some embodiments, sarcopenia is caused by genetic factors, altered circulation, decrease in the capillary:muscle fiber ratio, altered motor neurons, denervation, deterioration of motor end plates, selective reinnervation of Type I fibers, inflammatory responses causing muscle damage, reduced exercise, malnutrition, low dietary protein intake, vitamin D deficiency, age-related decline in vitamin D, oxidative stress, muscle mitochondrial mutations, changes in specific types of muscle fibers, decline in muscle protein, disabling disease, strokes, Alzheimer's disease, Parkinson's disease, osteoporsis, atherosclerosis, diabetes mellitus, hyperinsulimemia, renal failure, or hypogonadism.

It is to be understood that any composition for use according to this invention, as herein described, encompasses the administration of a composition comprising a compound *S*-(I) as herein described, to the subject, in order to treat the indicated disease, disorder or condition. The treatments as herein described each and/or all may further comprise administration of an additional therapeutic agent as herein described, and as will be appreciated by one skilled in the art.

In another embodiment, it is provided a composition for use in treating a hormone dependent disease, disorder or condition, the treatment comprising administering to the subject a compound as herein described, and optionally chemotherapeutics agents and therapies (methotrexate, cyclophosphamide, ifosfamide, adriamycin, doxorubicin, glucocorticoids, cyclosporine, L-thyroxine, SERMs, AI, fulvestrant, GnRH agents, ADT, discontinuation of hormone replacement therapy, cranial irradiation, peripheral irradiation, etc.; prolactinemia-inducing pharmacotherapeutics (serotonergic antidepressants acting through 5HT2 receptors, selective serotonin reuptake inhibitors, monoamine oxidase inhibitors, tricyclic antidepressants, antihypertensives such as methyldopa, reserpine, clonidine, and verapamil; antidopaminergic anti-emetics such as metoclopramide, H2 receptor antagonists such as cimetidine and ranitidine, estrogens, amphetamines, AR partial antagonists (ketoconazole, spironolactone, eplerenone)

In another embodiment, the subject has a hormonal imbalance, disorder, or disease. In another embodiment the subject has menopause.

Example 5 demonstrates that a compound of formula *S*-(I) is anabolic yet minimally androgenic, thus such compounds may be useful in treating patient groups in which androgens were contraindicated in the past. Compound of formula *S*-(I) was shown to stimulate muscle growth, whether in the presence or absence of testosterone while exerting anti-proliferative effects on the prostate, thus, in one embodiment, this invention provides for restoring lost muscle mass in patients with sarcopenia or cachexia.

It is disclosed that the compounds *S*-(I) as herein described are useful in a) treating, preventing, suppressing, inhibiting, or reducing obesity; b) promoting, increasing or facilitating weight loss; c) decreasing, suppressing, inhibiting or reducing appetite; d) altering the body composition; e) altering lean body mass or fat free body mass; f) converting fat to lean muscle; g) treating, preventing, suppressing, inhibiting, or reducing an obesity-associated metabolic disorder, for example hypertension, osteoarthritis, diabetes mellitus, maturity onset diabetes of the young (MODY), increased blood pressure, stroke, or heart disease; h) decreasing, suppressing, inhibiting or reducing adipogenesis; i) altering stem cell differentiation; and/or j) altering the level of leptin.

Androgen-dependent conditions which may be treated with compositions comprising compound *S*-(I) as herein described include those conditions which are associated with aging. In one embodiment, the compound as described herein is useful in a) Age-related functional decline (ARFD); b) reversal or prevention of ARFD; c) reversal or prevention of ARFD in elderly d) reversal or prevention of ARFD-induced sarcopenia or osteopenia; e) andropause, andropausal vasomotor symptoms, f) andropausal gynecomastia, muscle strength/function; g) bone strength/function; h) anger; i) asthenia; j) chronic fatigue syndrome; k) cognitive impairment; and/or 1) improving cognitive function.

In one embodiment, the compositions are for use in the treatment of human subjects, wherein, in one embodiment, the subject is male, or in another embodiment, the subject is female.

In one embodiment, the compositions for use of the present invention comprise a compound *S*-(I) as the sole active ingredient. However, also encompassed within the scope of the present invention are treatments for treating prostate cancer, delaying the progression of prostate cancer, and for preventing and/or treating the recurrence of prostate cancer, conditions associated with ADIF which comprise a combination with one or more therapeutic agents. These agents includeLHRH analogs, reversible antiandrogens, antiestrogens, anticancer drugs, 5-alpha reductase inhibitors, aromatase inhibitors, progestins, agents acting through other nuclear hormone receptors, selective estrogen receptor modulators (SERM), progesterone, estrogen, PDE5 inhibitors, apomorphine, bisphosphonate, and one or more additional SARMs.

Thus, in one embodiment, the compositions for use in accordance with the present invention may be administered in combination with an LHRH analog, a reversible antiandrogen, an antiestrogen, an anticancer drug, a 5-alpha reductase inhibitor, an aromatase inhibitor, a progestin, an agent acting through other nuclear hormone receptors, selective estrogen receptor modulators (SERM), a progesterone, an estrogen, a PDE5 inhibitor, apomorphine, a bisphosphonate, or one or more additional SARMs. In some embodiments, the compositions may comprise combined preparations comprising the compound and an agent as described hereinabove. In some embodiments, the combined

preparations can be varied, e.g., in order to cope with the needs of a patient subpopulation to be treated or the needs of the single patient which different needs can be due to the particular disease, severity of the disease, age, sex, or body weight as can be readily determined by a person skilled in the art. In some embodiments, the compositions for use in the present invention may be comprise personalized medicine which treat the needs of a single patient. In one embodiment, different needs can be due to the particular disease, severity of the disease, the overall medical state of a patient, or the age of the patient. In some embodiments, personalized medicine is the application of genomic data to better target the delivery of medical interventions. Personalized medicine, in some embodiments, serves as a tool in the discovery and clinical testing of new products of the present invention. In one embodiment, personalized medicine involves the application of clinically useful diagnostic tools that may help determine a patient's predisposition to a particular disease or condition. In some embodiments, personalized medicine is a comprehensive approach utilizing molecular analysis of both patients and healthy individuals to guide decisions throughout all stages of the discovery and development of pharmaceuticals and diagnostics; and applying this knowledge in clinical practice for a more efficient delivery of accurate and quality healthcare through improved prevention, diagnosis, treatment, and monitoring methods.

The compounds characterized by the structure of the formula *S*-(I) exhibited partial progesterone receptor (PR) agonist activity. In some embodiments, the compositions are provided for treatment of a disease or condition, which may be alleviated or improved by the administration of a PR agonist, by administration of a compound represented by the structure S-(I). In one embodiment the compound of formula *S*-(I) may be administered in an amount effective for treating a progestin-responsive tumor, such as a breast carcinoma, an ovarian carcinoma, a prostate carcinoma, an endometrial carcinoma, a cervical carcinoma, a leiomyosarcoma, or a meningioma.

In some embodiments, the compositions of this invention are provided for use in the treatment or amelioration of a progestin-dependent disease or condition. In some embodiments, the term "progestin-dependent disease" or "progestin-dependent condition" refers to an affliction, disorder, or physiological state that depends upon progestin activity for its existence or that is exacerbated by progestin activity.

In some embodiments, the compositions are provided for use in the treatment or amelioration of a progestin-responsive tumor. In some embodiments, the term "progestin-responsive tumor" refers to a tumor that contains progesterone receptors and whose growth and/or metastatic potential is stimulated by the binding of progestin to progesterone receptors within the tumor.

In some embodiments, for applications directed to its PR agonist activity, the compositions comprising the compound of formula *S*-(I) may be administered alone, or as any embodiment as herein described. The skilled artisan will readily appreciate routes of administration most suitable for treating the conditions related thereto, and as such represent embodiments herein. Similarly, dosaging, timing of administration, and other aspects of treatment with reference to treating progestin-dependent diseases or conditions may comprise any embodiment of treatment as described herein, including what is encompassed by the term "treatment" as herein described.

The following examples are presented in order to more fully illustrate the preferred embodiments of the invention.

## EXAMPLES

### EXAMPLE 1

### Synthesis of (S) Enantiomer of Compound of Formula (I) (Figures 1A-1L)

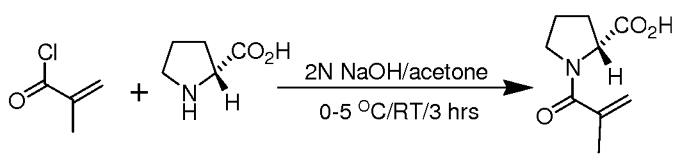


**(2*R*)-1-Methacryloylpyrrolidin-2-carboxylic Acid.** *D*-Proline, 14.93 g, 0.13 mol) was dissolved in 71 mL of 2 N NaOH and cooled in an ice bath; the resulting alkaline solution was diluted with acetone (71 mL). An acetone solution (71 mL) of metacryloyl chloride (13.56 g, 0.13 mol) and 2N NaOH solution (71 mL) were simultaneously added over 40 min to the aqueous solution of D-proline in an ice bath. The pH of the mixture was kept at 10-11°C during the addition of the metacryloyl chloride. After stirring (3 h, room temperature), the mixture was evaporated *in vacuo* at a temperature at 35-45 °C to remove acetone. The resulting solution was washed with ethyl ether and was acidified to pH 2 with concentrated HCl. The acidic mixture was saturated with NaCl and was extracted with EtOAc (100 mL x 3). The combined extracts were dried over $Na_2SO_4$, filtered through Celite®, and evaporated *in vacuo* to give the crude product as a colorless oil. Recrystallization of the oil from ethyl ether and hexanes afforded 16.2 (68%) of the desired compound as

colorless crystals: mp 102-103 °C (lit. [214] mp 102.5-103.5 °C); the NMR spectrum of this compound demonstrated the existence of two rotamers of the title compound. $^1$H NMR (300 MHz, DMSO-$d_6$) $\delta$ 5.28 (s) and 5.15 (s) for the first rotamer, 5.15 (s) and 5.03 (s) for the second rotamer (totally 2H for both rotamers, vinyl $CH_2$), 4.48-4.44 for the first rotamer, 4.24-4.20 (m) for the second rotamer (totally 1H for both rotamers, CH at the chiral canter), 3.57-3.38 (m, 2H, $CH_2$), 2.27-2.12 (1H, CH), 1.97-1.72 (m, 6H, $CH_2$, CH, Me); $^{13}$C NMR (75 MHz, DMSO-$d_6$) $\delta$ for major rotamer 173.3, 169.1, 140.9, 116.4, 58.3, 48.7, 28.9, 24.7, 19.5: for minor rotamer 174.0, 170.0, 141.6, 115.2, 60.3, 45.9, 31.0, 22.3, 19.7; IR (KBr) 3437 (OH), 1737 (C=O), 1647 (CO, COOH), 1584, 1508, 1459, 1369, 1348, 1178 $cm^{-1}$; $[\alpha]_D^{26}$ +80.8° (c = 1, MeOH); Anal. Calcd. for $C_9H_{13}NO_3$: C 59.00, H 7.15, N 7.65. Found: C 59.13, H 7.19, N 7.61.

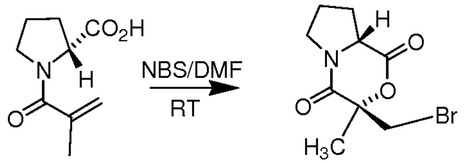


**(3*R*,8a*R*)-3-Bromomethyl-3-methyl-tetrahydro-pyrrolo[2,1-c][1,4]oxazine-1,4-dione.** A solution of NBS (23.5 g, 0.132 mol) in 100 mL of DMF was added dropwise to a stirred solution of the (methyl-acryloyl)-pyrrolidine (16.1g, 88 mmol) in 70 mL of DMF under argon at room temperature, and the resulting mixture was stirred 3 days. The solvent was removed *in vacuo,* and a yellow solid was precipitated. The solid was suspended in water, stirred overnight at room temperature, filtered, and dried to give 18.6 g (81%) (smaller weight when dried ~ 34%) of the title compound as a yellow solid: mp 152-154 °C; $^1$H NMR (300 MHz, DMSO-$d_6$) $\delta$ 4.69 (dd, *J* = 9.6 Hz, *J* = 6.7 Hz, 1H, CH at the chiral center), 4.02 (d, *J* = 11.4 Hz, 1H, $CHH_a$), 3.86 (d, *J* = 11.4 Hz, 1H, $CHH_b$), 3.53-3.24 (m, 4H, $CH_2$), 2.30-2.20 (m, 1H, CH), 2.04-1.72 (m, 3H, $CH_2$ and CH), 1.56 (s, 2H, Me); $^{13}$C NMR (75 MHz, DMSO-$d_6$) $\delta$ 167.3, 163.1, 83.9, 57.2, 45.4, 37.8, 29.0, 22.9, 21.6; IR (KBr) 3474, 1745 (C=O), 1687 (C=O), 1448, 1377, 1360, 1308, 1227, 1159, 1062$cm^{-1}$; $[\alpha]_D^{26}$ +124.5 ° (c = 1.3, chloroform); Anal. Calcd. for $C_9H_{12}BrNO_3$: C 41.24, H 4.61, N 5.34. Found: C 41.46, H 4.64, N 5.32.

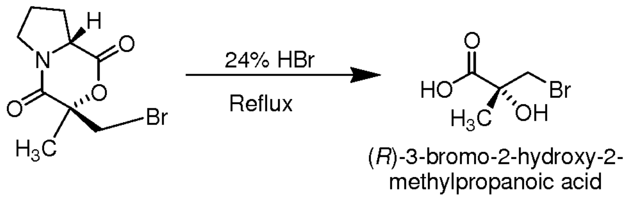


(*R*)-3-bromo-2-hydroxy-2-methylpropanoic acid

**(2*R*)-3-Bromo-2-hydroxy-2-methylpropanoic Acid.** A mixture of bromolactone (18.5 g, 71 mmol) in 300 mL of 24% HBr was heated at reflux for 1 h. The resulting solution was diluted with brine (200 mL), and was extracted with ethyl acetate (100 mL x 4). The combined extracts were washed with saturated $NaHCO_3$ (100 mL x 4). The aqueous solution was acidified with concentrated HCl to pH = 1, which, in turn, was extracted with ethyl acetate (100 mL x 4). The combined organic solution was dried over $Na_2SO_4$, filtered through Celite®, and evaporated *in vacuo* to dryness. Recrystallization from toluene afforded 10.2 g (86%) of the desired compound as colorless crystals: mp 107-109 °C (lit. [214] mp 109-113 °C for the *S*-isomer); $^1$H NMR (300 MHz, DMSO-$d_6$) $\delta$ 3.63 (d, *J* = 10.1 Hz, 1H, $CHH_a$), 3.52 (d, *J* = 10.1 Hz, 1H, $CHH_b$), 1.35 (s, 3H, Me); IR (KBr) 3434 (OH), 3300-2500 (COOH), 1730 (C=O), 1449, 1421, 1380, 1292, 1193, 1085 $cm^{-1}$; $[\alpha]_D^{26}$ +10.5° (c = 2.6, MeOH); Anal. Calcd. for $C_4H_7BrO_3$: C 26.25, H 3.86. Found: C 26.28, H 3.75.

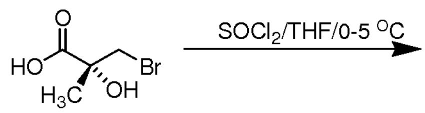


(*R*)-3-bromo-2-hydroxy-2-methylpropanoic acid

***R-18***

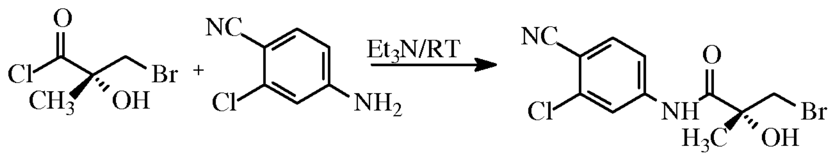


**Synthesis of (2*R*)-3-bromo-*N*-(3-chloro-4-cyanophenyl)-2-hydroxy-2-methylpropanamide.** Thionyl chloride (7.8 g, 65.5 mmol) was added dropwise to a cooled solution (less than 4 °C) of (*R*)-3-bromo-2-hydroxy-2-methylpropanoic acid (9.0 g, 49.2 mol) in 50 mL of THF under an argon atmosphere. The resulting mixture was stirred for 3 h under the same condition. To this was added $Et_3N$ (6.6 g, 65.5 mol) and stirred for 20 min under the same condition. After 20 min, 4-amino-2-chlorobenzonitrile (5.0 g, 32.8 mmol) and 100 mL of THF were added and then the mixture was allowed to stir overnight at room temperature. The solvent was removed under reduced pressure to give a solid which was treated with 100 mL of $H_2O$, extracted with EtOAc (2 × 150 mL). The combined organic extracts were washed with saturated $NaHCO_3$ solution (2 × 100 mL) and brine (300 mL), successively. The organic layer was dried over $MgSO_4$ and concentrated under reduced pressure to give a solid which was purified from column chromatography using EtOAc/hexane (50:50) to give 7.7 g (49.4%) of target compound as a brown solid.
$^1$H NMR ($CDCl_3$/TMS) δ 1.7 (s, *3H,* $CH_3$), 3.0 (s, *1H,* OH), 3.7 (d, *1H,* CH), 4.0 (d, *1H,* CH), 7.5 (d, *1H,* ArH), 7.7 (d, *1H,* ArH), 8.0 (s, *1H,* ArH), 8.8 (s, *1H,* NH). MS:342.1 (M+23). Mp 129°C.

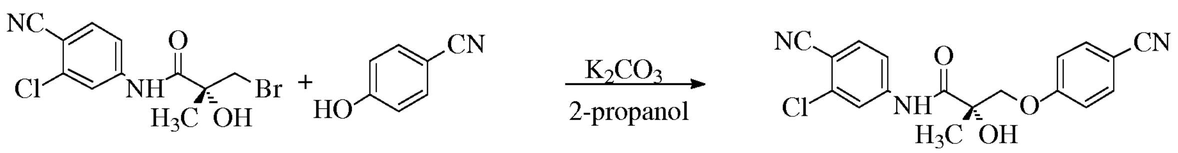


**Synthesis of (*S*)-*N*-(3-chloro-4-cyanophenyl)-3-(4-cyanophenoxy)-2-hydroxy-2-methylpropanamide.** A mixture of bromoamide (2.0 g, 6.3 mmol), anhydrous $K_2CO_3$ (2.6 g, 18.9 mmol) in 50 mL of acetone was heated to reflux for 2h and then concentrated under reduced pressure to give a solid. The resulting solid was treated with 4-cyanophenol (1.1 g, 9.5 mmol) and anhydrous $K_2CO_3$ (1.7 g, 12.6 mmol) in 50 mL of 2-propanol was heated to reflux for 3 h and then concentrated under reduced pressure to give a solid. The residue was treated with 100 mL of $H_2O$ and then extracted with EtOAc (2 X 100 mL). The combined EtOAc extracts were washed with 10% NaOH (4 X 100 mL) and brine, successively. The organic layer was dried over $MgSO_4$ and then concentrated under reduced pressure to give an oil which was purified by column chromatography using EtOAc/hexane (50:50) to give a solid. The solid was recrystallized from $CH_2Cl_2$/hexane to give 1.4 g (61.6 %) of (*S*)-*N*-(3-chloro-4-cyanophenyl)-3-(4-cyanophenoxy)-2-hydroxy-2-methylpropanamide as a colorless solid.
$^1$H NMR ($CDCl_3$/TMS) δ 1.61 (s, 3H, $CH_3$), 3.25 (s, 1H,O*H*), 4.06 (d, *J* = 9.15 Hz, 1H, CH), 4.50 (d, *J* = 9.15 Hz, 1H, CH), 6.97 - 6.99 (m, 2H, ArH), 7.53-7.59 (m, 4H, ArH), 7.97 (d, *J* = 2.01 Hz, 1H, ArH), 8.96 (s, 1H, N*H*). Calculated Mass: 355.1, $[M+Na]^+$ 378.0. Mp: 103-105 °C.

## EXAMPLE 2

### Inhibition of Cytochrome P450 Enzymes

CYP enzymes are a super-family of heme proteins located in the smooth endoplasmic reticulum of cells. These enzymes have wide substrate specificity, and are the major group of enzymes responsible for drug metabolism

CYP inhibition screening assays were performed for *S*-(I), *S*-(II) and *S*-(III). In order to assess whether *S*-(I) exhibited any inhibitory effects on CYP activity, the five major isoenzymes of the CYP family were evaluated *in vitro.* Human recombinant CYP enzymes and fluorescence-based substrates were used to determine the $IC_{50}$ values of *S*-(I)

against CYP3A4, 2D6, 2C19, 2C9, and 1A2.

**Materials and Methods:**

***Recombinant Enzyme (Fluorescent-based) Assay***

CYP inhibition screening procedures were performed essentially according to the manufacturer's instructions (Gentest, BD Biosciences, Waltham, MA). Briefly, CYP enzyme inhibition was measured using human cDNA-expressed CYP3A4, 2D6, 2C19, 2C9, and 1A2 enzymes. Analogs of the model substrate coumarin were utilized for each isoenzyme. 7-Benzyloxy-trifluoromethylcoumarin (BFC) for 3A4; 3-[2-(*N,N*-diethyl-*N*-methylamino)ethyl]-7-methoxy-4-methylcoumarin (AMMC) for 2D6; 3-cyano-7-ethoxycoumarin (CEC) for 2C19 and 1A2; and 7-methoxy-4-trifluoro-methylcoumarin (MFC) for 2C9. These substrates were utilized at a single concentration (either 50 μM or 75 μM) at or near the apparent $K_m$ for each substrate. The positive control inhibitors used for inhibition assays are shown in Table 1.

Compound stocks (10 mM in a 4:1 ratio of acetonitrile:DMSO, or neat acetonitrile) were prepared and diluted to provide an 8-point dose response curve in duplicate (final concentration ranging from 0.15 μM to 20.0 μM). The concentration of acetonitrile was kept constant at 0.4%, and the reaction was carried out at 37°C for 30 minutes. An $IC_{50}$ value was calculated as the concentration where 50% inhibition of the catalytic activity of the enzyme occurs.

**Table 1. Profile of Positive Control Inhibitors.**

| **Compound** | CYP Inhibition, $IC_{50}$ (μM) | | | | |
|---|---|---|---|---|---|
| | **3A4** | **2D6** | **2C19** | **2C9** | **1A2** |
| Ketoconazole | 0.006 | | | | |
| Quinidine | | 0.012 | | | |
| Tranylcypromine | | | 3.4 | | |
| Sulfaphenazole | | | | 0.33 | |
| Furafylline | | | | | 2.5 |

*Analytical Methods*

Fluorescent intensity was measured using a Wallac 1420 Victor$^3$ Multi-label Counter Model (Perkin-Elmer, Wellesley, MA), with an excitation wavelength filter of 405 nm, and an emission filter of 460 nm (535 nm for the 3A4 and 2C9 substrates). Nunc-brand optical bottom, black 96-well plates (#265301) were used in all assays, and a top read of 1.0 second per well was performed for all samples.

*Data analysis*

Duplicate averages (minus background) were determined in *Excel,* and $IC_{50}$ values were calculated in *GraphPad Prism,* v 4.03. For $IC_{50}$ determination, data was fitted using the non-linear regression equation "sigmoidal dose response, variable slope" defined as: Y=Bottom + (Top-Bottom)/(1+10^((LogEC50-X)*HillSlope)) where X is the logarithm of concentration of the indicated compound, and Y is the response.

**Results:**

Ketoconazole, quinidine, tranylcypromine, sulfaphenazole, and furafylline potently inhibited CYP3A4, CYP2D6, CYP2C19, CYP2C9, and CYP1A2 activity, respectively, demonstrating the utility of this *in vitro* CYP screening assay to identify compounds with the potential for significant CYP-mediated drug :drug interactions. None of the investigational substituted acylanilide compounds significantly inhibited CYP3A4, CYP2D6, or CYP1A2, indicating that drug:drug interactions via these CYP isozymes are unlikely to occur. Although all of the investigational substituted acylanilide compounds inhibited CYP2C9 activity to a modest extent, none of the compounds demonstrated potency similar to sulfaphenazole. Unexpectedly, only *S*-(I) failed to inhibit CYP2C19.

**Table 2. CYP Inhibition Profile of Novel SARMs and Positive Controls.**

| Compound | CYP Inhibition, $IC_{50}$ (μM) | | | | |
|---|---|---|---|---|---|
| | **3A4** | **2D6** | **2C19** | **2C9** | **1A2** |
| *S*-(III) 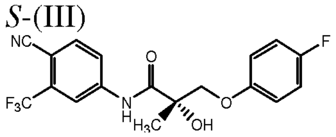 | > 20 | > 20 | 0.1 | 1.3 | > 20 |
| *S*-(II) 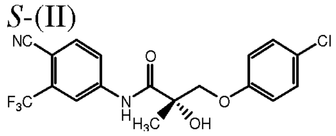 | > 20 | > 20 | 0.1 | 3.8 | > 20 |
| *S*-(I) 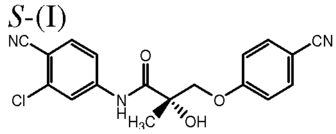 | >20 | > 20 | > 20 | 5.1 | > 20 |
| Ketoconazole | 0.03 | | | | |
| Quinidine | | 0.013 | | | |
| Tranylcypromine | | | 11.2 | | |
| Sulfaphenazole | | | | 0.36 | |
| Furafylline | | | | | 2.02 |

**Conclusions:**

*S*-(II) and *S*-(III) demonstrated moderate inhibition of CYP2C9 and CYP2C19. Unexpectedly, *S*-(I) did not appreciably inhibit CYP2C19 or CYP2C9, indicating *S*-(I)'s unique clinical and therapeutic advantage.

**EXAMPLE 3**

**Androgen Receptor Binding Affinity of SARMs:**

Materials and Methods:

The androgen receptor (AR) binding affinity of SARMs was determined by using an *in vitro* competitive radioligand binding assay with [17α-methyl-$^3$H]-mibolerone ([$^3$H]MIB, PerkinElmer), a high affinity AR ligand. Recombinant androgen receptor ligand binding domain (AR LBD) was combined with [$^3$H]MIB in buffer A (10 mM Tris, pH 7.4, 1.6 mM disodium EDTA, 0.26 M sucrose, 10 mM sodium molybdate, 1 mM PMSF) to determine the equilibrium dissociation constant ($K_d$) of [$^3$H]MIB. Protein was incubated with increasing concentrations of [$^3$H]MIB with and without a high concentration of unlabeled MIB in order to determine total and non-specific binding. Non-specific binding was then subtracted from total binding to determine specific binding and graphed using SigmaPlot® and non-linear regression for ligand binding curve with one site saturation to determine the $K_d$ of MIB (1.84 nM). In addition, the concentration of [$^3$H]MIB required to saturate AR LBD was determined to be 4 nM.

The S isomer of the compound of formula (I) (S-(I)) was tested in a range of concentrations from $10^{-11}$ to $10^{-6}$ M using the conditions described above. Following incubation, plates were harvested with GF/B filters on the Unifilter-96 Harvester (PerkinElmer) and washed three times with ice-cold buffer B (60 mM Tris, pH 7.2). The filter plates were dried at RT, then 36 μl Microscint-O cocktail was added to each well and sealed with TopSeal-A. The receptor bound radioligand was then determined with the TopCount® NXT Microplate Scintillation Counter (PerkinElmer).

The specific binding of [$^3$H]MIB at each concentration of SARM was determined by subtracting the nonspecific binding of [$^3$H]MIB (determined by incubating with $10^{-6}$ M unlabeled MIB), and expressed as a percentage of the specific binding in the absence of each SARM. The concentration of SARM required to decrease the [$^3$H]MIB binding by 60%, $IC_{60}$ value, was determined by computer-fitting the data with SigmaPlot® and non-linear regression with the standard curve four parameter logistic curve. The equilibrium binding constant ($K_i$) of each compound was then determined with

the following equation:

$$K_i = K_d \times IC_{60}/(K_d + L)$$

where $K_d$ is the equilibrium dissociation constant of [$^3$H]MIB (1.84 nM), and $L$ is the concentration of [$^3$H]MIB (4 nM).

**Results:**

The binding affinity for compound of formula (I) [S-(I)] was tested in the radioligand binding assay with AR LBD as the receptor with $K_i$ (nM) = 36.9.

**EXAMPLE 4**

**Cross-Reactivity of *S*-(I) with Other Nuclear Hormone Receptors**

In order to determine whether the substituted acylanilide compound of this invention affected other nuclear hormone receptor signaling, its comparative ability to stimulate (agonist) or inhibit (antagonist) ERα-, ERβ-, GR-, PR-, or MR-mediated transcriptional activation, was compared to other acylanilides.

**Materials and Methods:**

***Transient Transfection***

Rat GR, MR, PR, ER-α and ER-β were individually cloned into a pCR3.1 vector backbone. Sequencing was performed to verify the absence of any mutations. HEK-293 cells were plated at 90,000 cells per well of a 24 well plate in Dulbecco's Minimal Essential Media supplemented with 5% charcoal-stripped FBS. The cells were transfected using Lipofectamine (Invitrogen, Carlsbad, CA) with 0.25 μg GRE-LUC for GR, MR and PR and ERE-LUC for ER-α and ER-β, 0.5 ng CMV-LUC (renilla luciferase) and 12.5-25 ng of the respective expression vector for each receptor. The cells were treated 24 hrs after transfection with investigational substituted acylanilide SARM compounds (*S*-(I), S-(II) and *S*-(III)) in the absence (agonist mode) and presence (antagonist mode) of known agonists (estradiol for ER; dexamethasone for GR; aldosterone for MR; progesterone for PR) as controls. Luciferase assays were performed 48 hrs after transfection. Transcriptional activation values are represented as firefly luciferase normalized to renilla luciferase.

**Results:**

Results of *S*-(III):

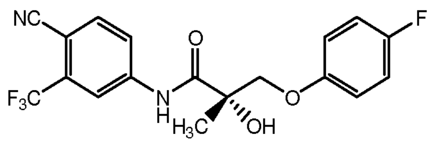

The agonist effects of *S*-(III) on ER-β, ER-α, GR, PR and MR were tested and compared to the activities of the known ligands. As shown in Figure 2, *S*-(III) failed to activate ER-β or ER-α even at the highest tested concentration (1 μM) whereas 1 nM estradiol induced ERα- and ERβ-mediated transactivation by 3- and 5-fold, respectively. Figure 2 also shows the inability of *S*-(III) to activate PR-, GR- or MR-mediated transactivation. *S*-(III) at all the tested concentrations did not induce PR-, GR- or MR-mediated transactivation, whereas the known ligands (dexamethasone, progesterone and aldosterone) induced the activities of GR, PR or MR by 70- 23- and 60-fold, respectively, at a concentration of 1 nM.

Similalry, the ability of *S*-(III) to inhibit the effects of a known agonist for each of the above mentioned receptors was tested.

Figure 3 summarizes the antagonist effects of *S*-(III) on ER-β-, ER-α-, GR-, PR- and MR-mediated transactivation. HEK-293 cells transfected with the indicated receptor and corresponding reporter construct were treated with a titration of *S*-(III) and then with 1 nM estradiol for ER-β and ER-α transactivation, 1 nM aldosterone for MR transactivation, 1 nM dexamethasone for GR transactivation or 1 nM progesterone for PR transactivation. Estradiol increased ER-β-

and ER-α-mediated transactivation by 3- and 5-fold, respectively. Co-incubation of cells with a titration of *S*-(III) failed to alter the estradiol-induced ER-β or ER-α activity. Similarly, dexamethasone-induced GR-mediated transactivation and aldosterone-induced MR-mediated transactivation were not inhibited by *S*-(III) at any tested concentrations.

*S*-(III) significantly inhibited PR activity, however, at the highest concentrations (i.e., 1 and 10 μM), indicating that it could function as a PR antagonist, yet RU486 (a known PR antagonist) completely inhibited PR activity at a 1 nM concentration, indicating about 10,000 fold weaker antagonistic activity for *S*-(III) as compared to RU486.

Results of *S*-(II):

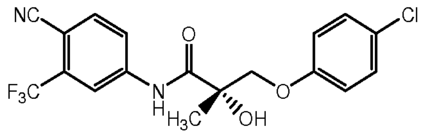

The agonist effects of S-(II) on ER-β, ER-α, GR, PR and MR were tested and compared to the activities of the known ligands. Similar to S-(III), as shown in Figure 4, S-(II) failed to activate ER-β or ER-α even at the highest tested concentration (1 μM) whereas 1 nM estradiol induced ERα- and ERβ-mediated transactivation by 3- and 5-fold, respectively. Figure 4 also shows the inability of S-(II) to activate PR-, GR- or MR-mediated transactivation. *S*-(II) at all the tested concentrations did not induce PR-, GR- or MR-mediated transactivation, whereas the known ligands (dexamethasone, progesterone and aldosterone) induced the activities of GR, PR or MR by 70- 23- and 60-fold, respectively, at a concentration of 1 nM.

The ability of *S*-(II) to inhibit the effects of a known agonist for each of the above mentioned receptors was evaluated as well.

Figure 5 summarizes the antagonist effects of *S*-(II) on ER-β-, ER-α-, GR-, PR- and MR-mediated transactivation. HEK-293 cells transfected with the indicated receptor were treated with the indicated concentration of S-(II), and the known agonist for the appropriate receptor, as above. Estradiol increased ER-β- and ER-α-mediated transactivation by 3- and 5-fold, respectively. Co-incubation of cells with a titration of *S*-(II) failed to alter the estradiol-induced ER-β or ER-α activity. Similarly, dexamethasone-induced GR-mediated transactivation and aldosterone-induced MR-mediated transactivation were not inhibited by *S*-(II) at any tested concentrations. As was the case with S-(III), S-(II) failed to antagonize the ER, GR or MR, under these conditions, at the concentrations tested.

*S*-(II), similar to S-(III) significantly inhibited PR activity at the highest concentrations (i.e., 1 and 10 μM) tested, however it too exhibited about 10,000 fold weaker antagonistic activity in comparisong to RU486. GTx-830 [should be GTx-832?, i.e S-(II)] as compared to RU486.

Results of *S*-(I):

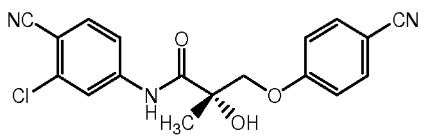

The agonist effects of *S*-(I) on ER-β, ER-α, GR, PR and MR were tested and compared to the activities of the known ligands, as well (Figure 6). *S*-(I) failed to activate ER-β or ER-α even at the highest tested concentration (1 μM) whereas 1 nM estradiol induced ERα- and ERβ-mediated transactivation by 3- and 5-fold, respectively. *S*-(I) failed to activate PR-, GR- or MR-mediated transactivation. *S*-(I) at all the tested concentrations did not induce GR- or MR-mediated transactivation, whereas the known ligands (dexamethasone, and aldosterone) induced the activities of GR or MR by 70- and 60-fold, respectively, at a concentration of 1 nM. However, *S*-(I) increased the transactivation of PR at 1μM and 10 μM by 3 and 8 fold respectively. Progesterone activated PR by 23 fold at a 1 nM concentration, indicating that *S*-(I) is greater than 10,000-fold weaker than the endogenous agonist for PR.

The ability of *S*-(I) to inhibit the effects of a known agonist for each of the above mentioned receptors was tested as well, as in S-(II) and S-(III).

Co-incubation of HEK 293 cells with the indicated concentrations of *S*-(I) failed to alter the estradiol-induced

ER-β or ER-α activity, dexamethasone-induced GR-mediated transactivation or aldosterone-induced MR-mediated transactivation.

Unlike S-(II) and S-(III), while *S*-(I) significantly partially inhibited PR activity at concentrations 0.1 μM and 10 μM, it completely inhibited PR-mediated transactivation at 1 μM. In comparison to RU486, *S*-(I) was about 1,000 fold weaker a PR antagonist, than RU486.

S-(II) and *S*-(III) were found to be specific for the AR, failing to stimulate or inhibit receptor-mediated transactivation of ERα, ERβ, GR, or MR. S-(II) and S-(III), and being extremely weak antagonists for PR, requiring concentrations approximately 10,000 fold higher than RU486 to inhibit PR-mediated transcriptional activation.

*S*-(I) is similarly specific for the AR and does not stimulate or inhibit receptor-mediated transactivation of ERα, ERβ, GR, or MR. Unexpectedly, *S*-(I) exhibited partial agonist activity for PR.

## EXAMPLE 5

### Preclinical Anabolic and Androgenic Pharmacology of *S*-(I) in Intact and Castrate Male Rats.

Anabolic and androgenic efficacy of compound of formula (I) administered by daily oral gavage were tested. The *S*-isomer of compound (I) [*S*-(I)] was synthesized and tested as described herein

### Materials and Methods:

Male Sprague-Dawley rats weighing approximately 200 g were purchased from Harlan Bioproducts for Science (Indianapolis, IN). The animals were maintained on a 12-h light/dark cycle with food (7012C LM-485 Mouse/Rat Sterilizable Diet, Harlan Teklad, Madison, WI) and water available *ad libitum.* The animal protocol was reviewed and approved by the Institutional Animal Care and The anabolic and androgenic activity of compounds of formula (I) in intact animals was tested, as well as a dose response evaluation in acutely orchidectomized (ORX) animals. Regenerative effects of the compound of formula (I) in chronically (9 days) ORX rats was similarly evaluated.

The test article for this study was weighed and dissolved in 10% DMSO (Fisher) diluted with PEG 300 (Acros Organics, NJ) for preparation of the appropriate dosage concentrations. The animals were housed in groups of 2 to 3 animals per cage. Animals were randomly assigned to one of seven groups consisting of 4 to 5 animals per group. Control groups (intact and ORX) were administered vehicle daily. Compound of formula *S*-(I) was administered via oral gavage at doses of 0.01, 0.03, 0.1, 0.3, 0.75, and 1 mg/day to both intact and ORX groups. Where appropriate, animals were castrated on day one of the study. Treatment with compound of formula *S*-(I) began nine days post ORX and was administered daily via oral gavage for fourteen days.

The animals were sacrificed under anesthesia (ketamine/xyalzine, 87:13 mg/kg) and body weights were recorded. In addition, ventral prostate, seminal vesicles, and levator ani muscle were removed, individually weighed, normalized to body weight, and expressed as a percentage of intact control. Student's T-test was used to compare individual dose groups to the intact control group. Significance was defined *a priori* as a P-value < 0.05. Ventral prostate and seminal vesicle weights were evaluated as a measure of androgenic activity, whereas levator ani muscle weight was evaluated as a measure of anabolic activity. Blood was collected from the abdominal aorta, centrifuged, and sera were frozen at -80°C prior to determination of serum hormone levels. Serum luteinizing hormone (LH) and follicle stimulating hormone (FSH) concentrations were determined.

### Results:

A series of dose-response studies in intact and castrated rats in order to evaluate the potency and efficacy of compound of formula *S*-(I) in both androgenic (prostate and seminal vesicles) and anabolic (levator ani muscle) tissue was conducted. In intact animals, compound of formula *S*-(I) treatment resulted in decreases in the weight of both prostate and seminal vesicles while the levator ani muscle weight was significantly increased. Levator ani muscle weight following Compound *S*-(I) treatment were 107% ± 5%, 103% ± 7%, 97% ± 7%, 103% ± 5%, 118% ± 7%, and 118% ± 7% of intact controls following doses of 0.01, 0.03, 0.1, 0.3, 0.75, and 1 mg/day, respectively. The prostate weights were 103% ± 10%, 99% ± 10%, 58% ± 10%, 58% ± 15%, 65% ± 20%, and 77% ± 23% of intact controls following doses of 0.01, 0.03, 0.1, 0.3, 0.75, and 1 mg/day, respectively. These results are significant since current androgen therapies are contraindicated in some patient populations due to the proliferative androgenic effects in prostate and breast tissues. However, many patients in these populations could benefit from the anabolic actions of androgens in muscle and bone. Since compound of formula *S*-(I) exhibited tissue selective anabolic effects, it may be possible to treat patient groups in which androgens were contraindicated in the past.

In castrated, ORX animals, prostate weights following Compound *S*-(I) treatment were 12% ± 2%, 17% ± 6%, 31% ± 3%, 43% ± 15%, 54% ± 17%, 58% ± 10%, and 73% ± 12% of intact controls following doses of 0, 0.01, 0.03,

0.1, 0.3, 0.75, and 1 mg/day, respectively (Figure 8). Similarly, seminal vesicle weights were 10% ± 2%, 10% ± 3%, 13% ± 4%, 21% ± 6%, 43% ± 8%, 51% ± 9%, and 69% ± 14% of intact controls following doses of 0, 0.01, 0.03, 0.1, 0.3, 0.75, and 1 mg/day, respectively (Figure 8). Significant increases were seen in levator ani muscle weights of in all dose groups, when compared to intact controls. The levator ani muscle weights were 40% ± 5%, 52% ± 8%, 67% ± 9%, 98% ± 10%, 103% ± 12%, 105% ± 12% and 110% ± 17%
of intact controls corresponding to 0, 0.01, 0.03, 0.1, 0.3, 0.75, and 1.0 mg/day dose groups, respectively (Figure 8).

Testosterone propionate (TP) and *S*-3-(4-Acetylaminophenoxy)-2-hydroxy-2-methyl-*N*-(4-nitro-3-trifluoromethylphenyl) propionamide (S-4), maximally stimulated the levator ani muscle weight to 104% and 101%, respectively. These data show that compound of formula *S*-(I) exhibited significantly greater efficacy and potency than either TP or S-4. As a whole, these data show that compound of formula *S*-(I) is able to stimulate muscle growth in the presence or absence of testosterone while exerting anti-proliferative effects on the prostate. These data show that the compound of formula *S*-(I) restores lost muscle mass in patients with sarcopenia or cachexia. Additionally, the antiproliferative effects of the compound of formula (I) on the prostate may allow some patient populations, in which androgens are currently contraindicated, access to anabolic agents.

Anabolic ratios were derived comparing muscle/prostate weight in castrated rats. Values obtained were 3.02, 2.13, 2.27, 1.90, 1.83 and 1.51 following doses of 0.01, 0.03, 0.1, 0.3, 0.75 and 1 mg/day, respectively.

Animals receiving 1 mg/day of compound *S*-(I) exhibited a prostate weight of 77% ± 23% and levator ani muscle weight of 118% ± 7% of intact control values, respectively. Compound of formula *S*-(I) maintained prostate weight following orchidectomy at 73 ± 12% of intact controls and levator ani muscle weight at 110± 17% of intact controls. A derived dose of 0.1 mg/day of Compound *S*-(I) would restore levator ani muscle weight to 100%, while such dose would only restore 43 ± 15% prostate weight.

**EXAMPLE 6**

**Metabolic Stability of Compound *S*-(I):**

Metabolic stability assays were performed in order to assess the *in vitro* half-life of the compound of formula *S*-(I) when incubated with human liver microsomes. Intrinsic clearance values were extrapolated. Permeability of the compound across human, intestinal epithelial monolayers (Caco-2 cells) was assessed as a measure of intestinal permeability as well as an indicator of efflux potential. Caco-2 cells are often used as an early screening surrogate for oral bioavailability. Microsomal half-life can be converted to *in vitro* clearance values as a means to predict hepatic intrinsic clearance. Intrinsic clearance is defined as the functional ability of the liver to metabolize a drug or other compound.

**Materials and Methods:**

*Metabolic Stability Measured in Human Liver Microsomes:*

Compounds of formula S-(I) in this study were incubated at a final concentration of 0.6 μM. Microsome reactions were performed under either Phase I or "Phase I and II" conditions, where indicated. Compound stocks (10 mM ACN) were initially diluted to a concentration of 60 μM (in 60% ACN/$H_20$) resulting in a "working stock" solution of 100X. Human liver microsomes were utilized at a final concentration of 0.6 mg/ml. Duplicate wells were used for each time point (0, 6, 10, 30, and 60 minutes). Reactions were carried out at 37°C in a shaking water bath, and the final concentration of solvent was kept constant at 0.6%. The final volume for each reaction was 600 μl, comprised of 368 μl of 100 mM $KPO_4$ buffer, (pH 7.4); 12.6 μl of HLM (from a 20 mg/ml stock); 6 μl of 100X "working stock" drug compound, and 126 μl of NRS "master mix" solution. At each time point, 100 μl of reaction was removed and added to a sample well containing 100 μl of ice-cold, 100% ACN (plus internal standards), to stop the reaction. The NRS "master mix" is a solution of glucose 6-phosphate dehydrogenase, $NADP^+$, $MgCl_2$, and glucose 6-phosphate, prepared per manufacturer's instructions (BD Biosciences, Waltham, MA). Each 6.0 ml stock of NRS "master mix" solution contains 3.8 ml $H_20$, 1.0 ml solution "A" (Cat. #461220), and 0.2 ml solution "B" (Cat. #461200). Human liver microsomes (lot #0610279, Xenotech Corp.) represented a pool of 60 donors.

Samples were centrifuged at 3,000 rpm for 10 minutes at 4°C to remove debris and precipitated protein. Approximately 160 μl of supernatant was subsequently transferred to a new sample block for analysis. The concentration of parent drug remaining in each well (expressed as percent remaining versus Time '0', at the beginning of the reaction) was measured by LC/MS, as detailed below. The intrinsic clearance rates (CLint) were calculated from 0 - 60 minutes based on first order decay kinetics as a function of microsomal protein concentration.

*Permeability across Human, Intestinal Epithelial Monolayers:*

Permeability was measured in the Apical (pH 6.6) to Basolateral (pH 7.4) and Basolateral (pH 7.4) to Apical (pH 6.6) directions across polarized, Caco-2 epithelial monolayers. Compound stocks (10 mM acetonitrile) were tested in the study at a final concentration of 10 μM. The concentration of drug in the receiver well was measured by LC/MS/MS using a standard curve. The apparent permeability (Papp) for each compound was calculated, and values (A-B) were classified as: Poor (Papp: < 1), Low (Papp 1-2), Medium (Papp 2-10) or High (Papp >10).

$$\text{Papp } (\text{x } 10^{-6} \text{ cm/sec}) = \text{Amount transported} / (\text{Area} * \text{Initial concentration} * \text{Time})$$

$$\text{Papp (cm/s)} = [\, V / (\, A{*}Ci \,)] * (\, Cf / T \,)$$

V = volume of the receptor chamber (ml, or $cm^3$)
A = area of the membrane insert ($cm^2$)
Ci = initial concentration of drug (μM)
Cf = final concentration of drug (μM)
T = assay time (seconds)

*Analytical Methods:*

All samples were analyzed on the MDS/Sciex API4000 Q Trap system with electrospray ionization (ESI) in the positive or negative SIM mode, depending on the compounds. The mobile phases were isocratic at 30% A (0.1% formic acid in water) and 70% B (0.1% formic acid in acetonitrile) with a flow rate of 0.4 mL/min. A Phenomenex Luna Phenyl-Hexyl column (60 x 2.0 mm ID, 6μ) was used. The injection volume was 10 μL. The total run time per sample was 1.6 to 3.0 minutes. Tamoxifen and diclofenac were used as internal standards for the positive and negative mode, respectively. The percentage of parent drug compound remaining after each time point was determined relative to the initial measured concentration at the beginning of the reaction ($T_0$ min).

*Data analysis:*

For half-life determination, data was fitted using *GraphPad Prism, v 4.03* with the non-linear regression equation "one phase exponential decay" defined as:
**Y=Span*****exp(-K*****X)** + **Plateau** (decays to Plateau with a first-order rate constant, K).
"-K" is the slope of the curve. The half life (minutes), $T_{1/2}$, = ln 0.6/ -K and is therefore defined as -0.693/-K, or 0.693/K, a/k/a -0.693/slope). Intrinsic Clearance (μl/min/mg protein) is defined as: $CL_{int}$ = 0.693 * (1/ $T_{1/2}$) * (ml incubation/mg protein) * 1000; This equation can also be expressed as (K*1000)/microsome conc.

**Results:**

Table 3. Metabolic Stability Measured in Human Liver Microsomes:

| Compound having formula | Half Life (minutes) **Phase I only** | $CL_{int}$ (μl/min/mg) **Phase I only** | | Half Life (minutes) **Phase I + II** | $CL_{int}$ (μl/min/mg) **Phase I + II** |
|---|---|---|---|---|---|
| **I** | Stable | < 1 | | 348.9 | 2.0 |

The results had shown that *in vitro* half-life as determined from the microsomal assays demonstrated that compound of formula ***S***-(I) under both phase I and phase I/II metabolic conditions. As shown in Table 3, the compound did not exhibit an intrinsic clearance ($CL_{int}$) value greater than 10 μl/min/mg. It is generally accepted that an *in vitro* $CL_{int}$ value of less than 10 μl/min/mg protein represents favorable metabolic stability of the test compound. Compound of formula ***S***-(**I**) exhibited low clearance in human liver microsomes. In conclusion, based on the data reported herein, compound of formula ***S***-(**I**) exhibited favorable metabolic stability profiles *in vivo* studies.

**EXAMPLE 7**

**Pharmacokinetics of Compound (I) in Dogs**

In order to determine the pharmacokinetics of the *S*-isomer of the Compound of formula (I), the compound was administered to beagle dogs perorally, and circulating plasma levels, terminal elimination half-life ($t_{1/2}$), total body clearance (CL), terminal volume distribution (Vz) and absolute bioavailability (F%) (Table 4) were determined. Compound ***S***-(I) was rapidly and completely metabolized.

Table 4: **Pharmacokinetics in Dogs**

| | **Compound *S*-(I)** |
|---|---|
| $t_{1/2}$ (h) | **10.4 ± 0.5** |
| CL (mL/min/kg) | **1.68 ± 0.13** |
| Vz (mL/kg) | **1522 ± 142** |
| F% | **96.2%** |

**Claims**

**1.** A pharmaceutical composition comprising a compound of formula *S*-(I):

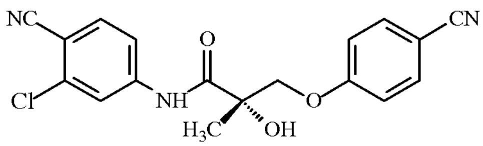

*S*-(I)

or its optical isomer, for use in treating a progestin-responsive tumor in a subject.

**2.** The pharmaceutical composition for use according to claim 1, wherein said progestin-responsive tumor is a breast carcinoma, an ovarian carcinoma, a prostate carcinoma, an endometrial carcinoma, a cervical carcinoma, a leiomyosarcoma, or a meningioma.

**3.** The pharmaceutical composition for use according to claim 2, wherein said progestin-responsive tumor is breast cancer.

**4.** A pharmaceutical composition comprising a compound of formula *S*-(I):

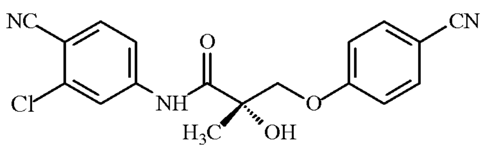

*S*-(I)

or its optical isomer, for use in treating a condition associated with androgen deficiency in aging male (ADAM).

**5.** The pharmaceutical composition for use according to claim 4, wherein said condition associated with androgen deficiency in aging male (ADAM) is fatigue, depression, decreased libido, sexual dysfunction, erectile dysfunction, hypogonadism, osteoporosis, hair loss, obesity, sarcopenia, osteopenia, benign prostate hyperplasia, or alterations in mood and cognition.

6. A pharmaceutical composition comprising a compound of formula *S*-(I):

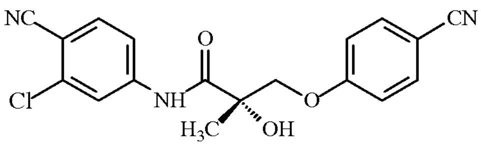

*S*-(I)

or its optical isomer, for use in treating a condition associated with androgen deficiency in a female (ADIF).

7. The pharmaceutical composition for use according to claim 6, wherein said condition associated with androgen deficiency in a female (ADIF) is sexual dysfunction, decreased sexual libido, hypogonadism, sarcopenia, osteopenia, osteoporosis, alterations in cognition and mood, depression, anemia, hair loss, obesity, endometriosis, breast cancer, uterine cancer and ovarian cancer.

**Patentansprüche**

1. Pharmazeutische Zusammensetzung, umfassend eine Verbindung der Formel *S*-(I):

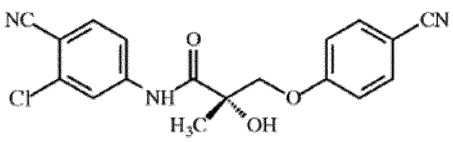

*S*-(I)

oder sein optisches Isomer zur Verwendung beim Behandeln eines auf Progestin ansprechenden Tumors bei einem Subjekt.

2. Pharmazeutische Zusammensetzung zur Verwendung nach Anspruch 1, wobei der auf Progestin ansprechende Tumor ein Brustkarzinom, ein Ovarialkarzinom, ein Prostatakarzinom, ein Endometriumkarzinom, ein Zervixkarzinom, ein Leiomyosarkom oder ein Meningiom ist.

3. Pharmazeutische Zusammensetzung zur Verwendung nach Anspruch 2, wobei der auf Progestin ansprechende Tumor Brustkrebs ist.

4. Pharmazeutische Zusammensetzung, umfassend eine Verbindung der Formel *S*-I) :

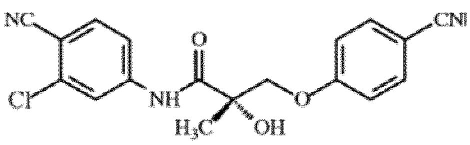

*S*-(I)

oder sein optisches Isomer zur Verwendung beim Behandeln eines Zustands, der mit Androgenmangel beim alternden Mann (Androgen Deficiency in Aging Male - ADAM) verbunden ist.

5. Pharmazeutische Zusammensetzung zur Verwendung nach Anspruch 4, wobei der mit Androgenmangel beim alternden Mann (ADAM) verbundene Zustand Müdigkeit, Depression, verminderte Libido, sexuelle Dysfunktion, erektile Dysfunktion, Hypogonadismus, Osteoporose, Haarausfall, Fettleibigkeit, Sarkopenie, Osteopenie, gutartige Prostatahyperplasie oder Stimmungs- und Wahrnehmungsschwankungen ist.

**6.** Pharmazeutische Zusammensetzung, umfassend eine Verbindung der Formel *S*-(I) :

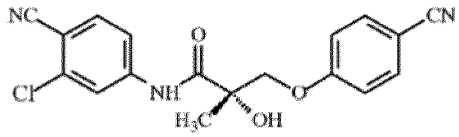

*S*-(I)

oder sein optisches Isomer zur Verwendung beim Behandeln eines Zustands, der mit einem Androgenmangel bei einer Frau (Androgen Deficieny In a Female - ADIF) verbunden ist.

**7.** Pharmazeutische Zusammensetzung zur Verwendung nach Anspruch 6, wobei der mit Androgenmangel bei einer Frau (ADIF) verbundene Zustand sexuelle Dysfunktion, verminderte sexuelle Libido, Hypogonadismus, Sarkopenie, Osteopenie, Osteoporose, Wahrnehmungs- und Stimmungsschwankungen, Depression, Anämie, Haarausfall, Fettleibigkeit, Endometriose, Brustkrebs, Gebärmutterkrebs und Eierstockkrebs ist.

## Revendications

**1.** Composition pharmaceutique comprenant un composé de formule *S*-(I) :

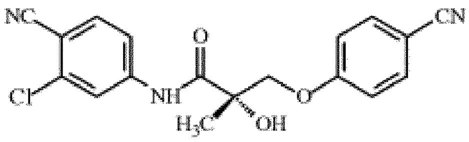

*S*-(I)

ou son isomère optique, destiné à être utilisé dans le traitement d'une tumeur sensible aux progestatifs chez un sujet.

**2.** Composition pharmaceutique à utiliser selon la revendication 1, dans laquelle ladite tumeur sensible aux progestatifs est un carcinome du sein, un carcinome ovarien, un carcinome de la prostate, un carcinome de l'endomètre, un carcinome cervical, un léiomyosarcome ou un méningiome.

**3.** Composition pharmaceutique à utiliser selon la revendication 2, dans laquelle ladite tumeur sensible aux progestatifs est un cancer du sein.

**4.** Composition pharmaceutique comprenant un composé de formule S-(I) :

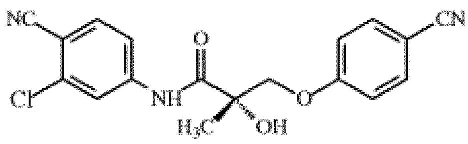

*S*-(I)

ou son isomère optique, destiné à être utilisé dans le traitement d'une affection associée à une carence en androgènes chez l'homme vieillissant (ADAM).

**5.** Composition pharmaceutique à utiliser selon la revendication 4, dans laquelle ladite condition associée à un déficit androgénique lié à l'âge (DALA) chez l'homme vieillissant est une fatigue, une dépression, une baisse de la libido, un dysfonctionnement sexuel, un dysfonctionnement érectile, un hypogonadisme, une ostéoporose, une chute de cheveux, de l'obésité, une sarcopénie, une ostéopénie, une hyperplasie bénigne de la prostate ou une altérations de l'humeur et de la cognition.

**6.** Composition pharmaceutique comprenant un composé de formule S-(I) :

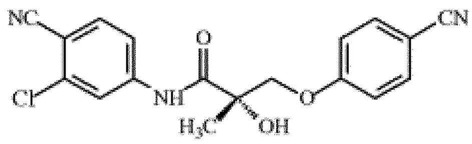

S-(I)

ou son isomère optique, destiné à être utilisé dans le traitement d'une affection associée à un déficit androgénique lié à l'âge (DALA) chez une femme.

**7.** Composition pharmaceutique à utiliser selon la revendication 6, dans laquelle ladite condition associée à un déficit androgénique lié à l'âge (DALA) chez une femme est un dysfonctionnement sexuel, une diminution de la libido sexuelle, un hypogonadisme, une sarcopénie, une ostéopénie, une ostéoporose, des altérations de la cognition et de l'humeur, une dépression, une anémie, une perte de cheveux, de l'obésité, une endométriose, un cancer du sein, un cancer de l'utérus et un cancer de l'ovaire.

## Figure 1A

## I. Synthesis process for (*S*)-I :

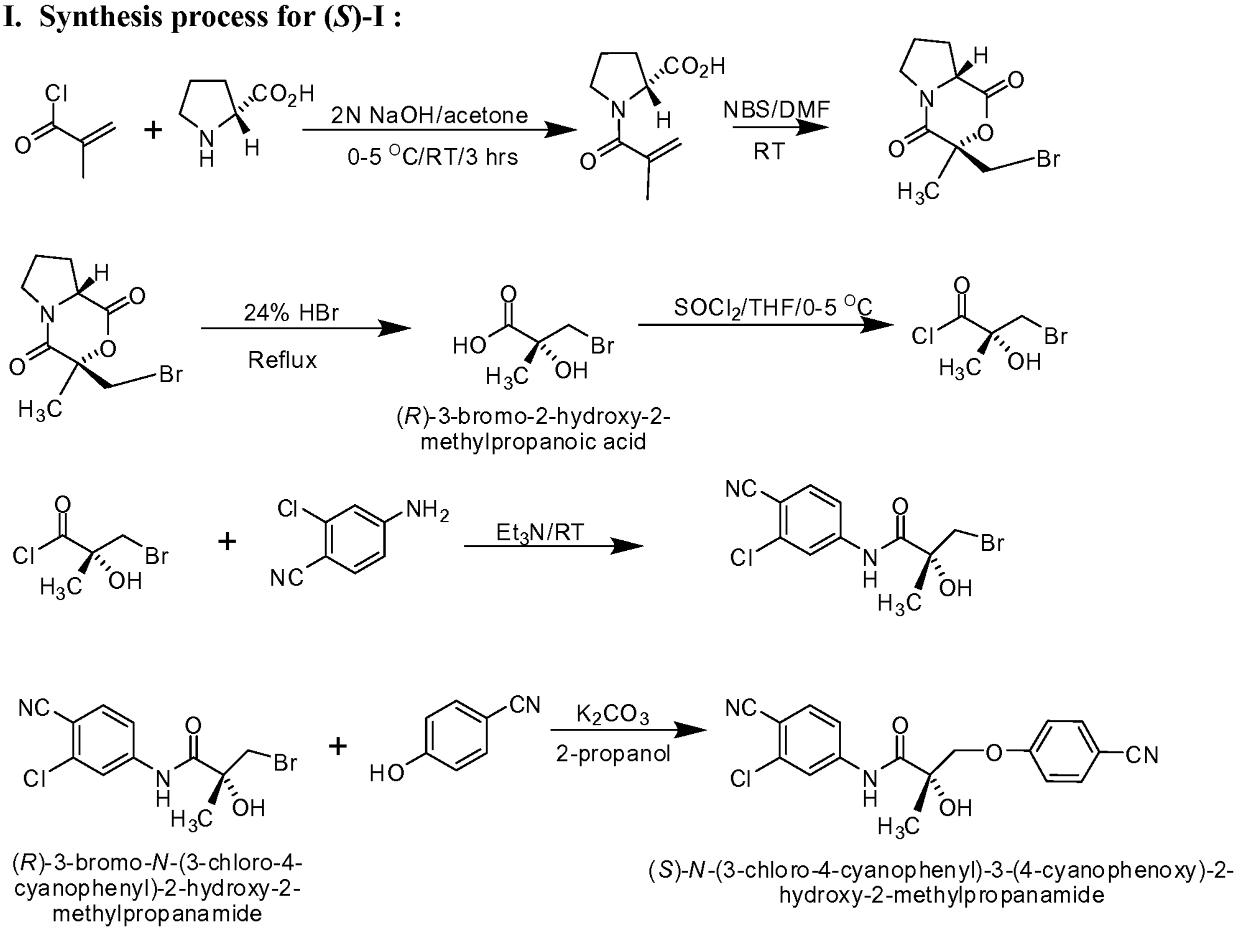


(*R*)-3-bromo-2-hydroxy-2-methylpropanoic acid

(*R*)-3-bromo-*N*-(3-chloro-4-cyanophenyl)-2-hydroxy-2-methylpropanamide

(*S*)-*N*-(3-chloro-4-cyanophenyl)-3-(4-cyanophenoxy)-2-hydroxy-2-methylpropanamide

**Figure 1B**

**II. Synthesis process for (*R*)-I :**

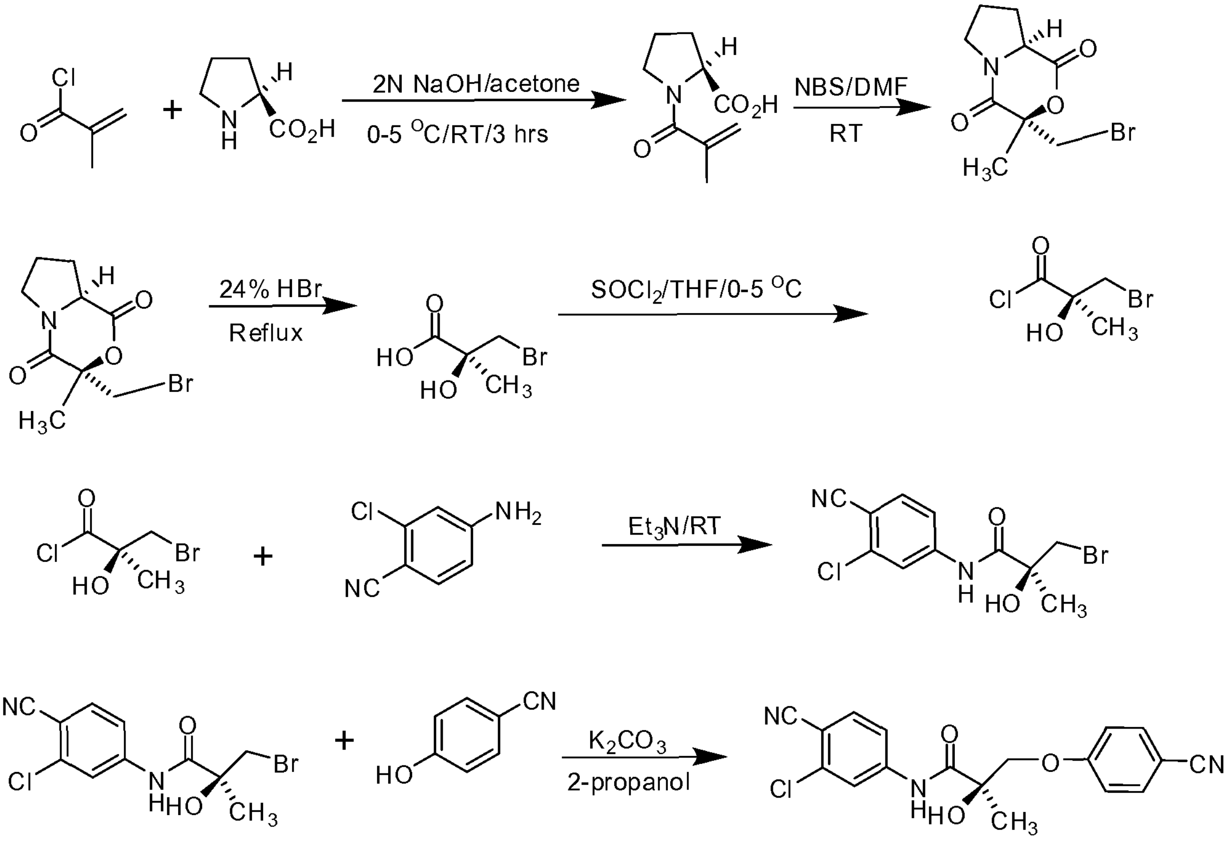

**Figure 1C**

**III. Synthetic process for (*S*)-I (oxirane intermediate):**

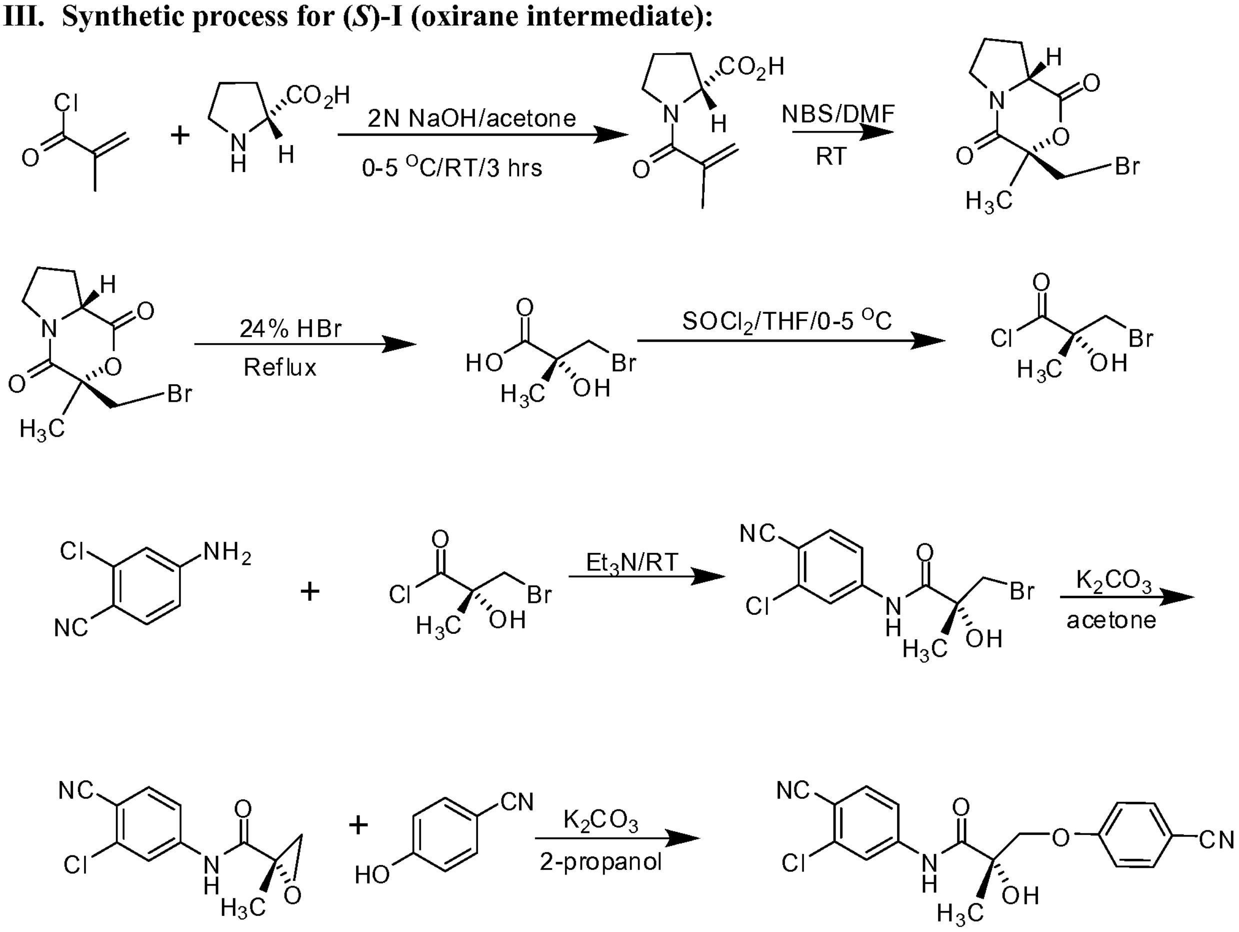

**Figure 1D**

**IV. Synthetic process for (*R*)-I (oxirane intermediate):**

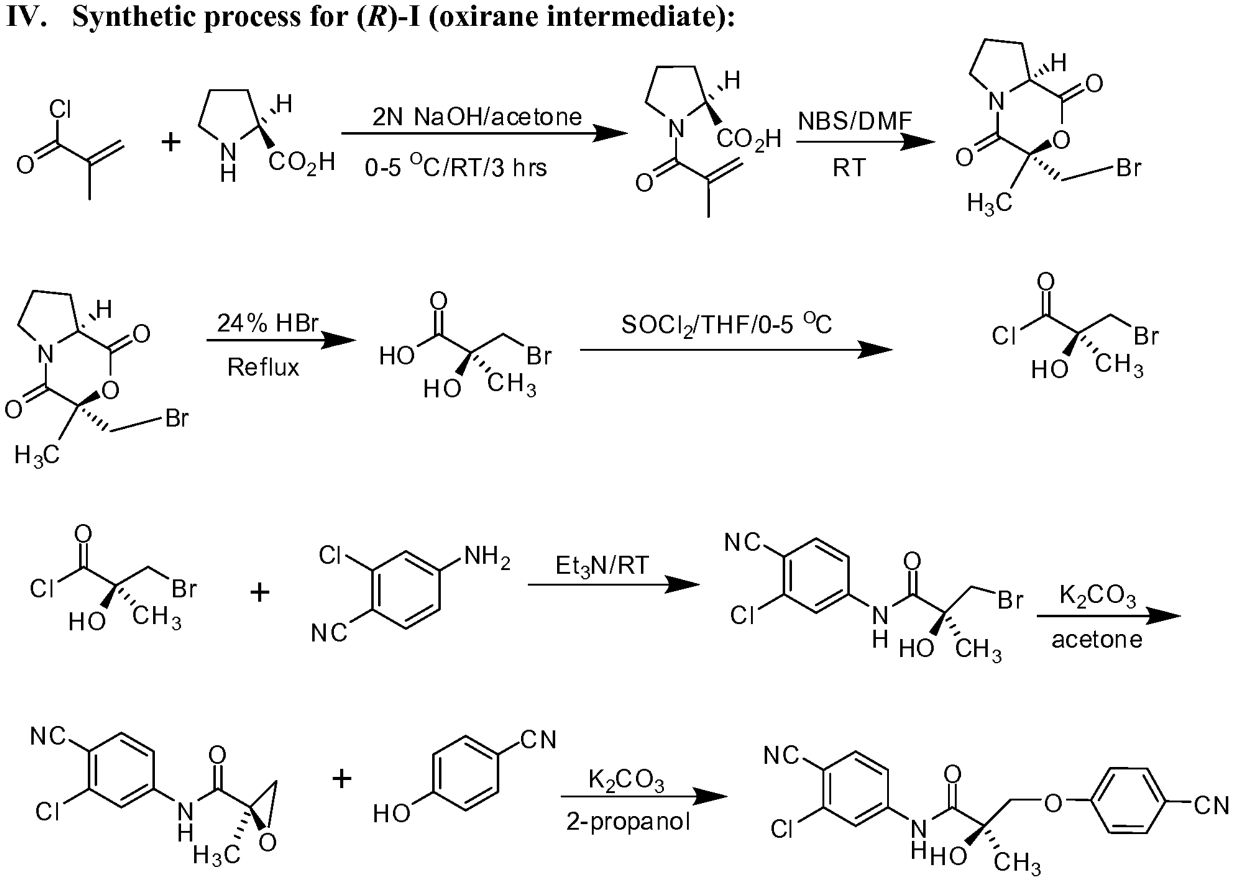

**Figure 1E**

**V. Synthetic process for (*S*)-I involving B-ring addition prior to A-ring addition:**

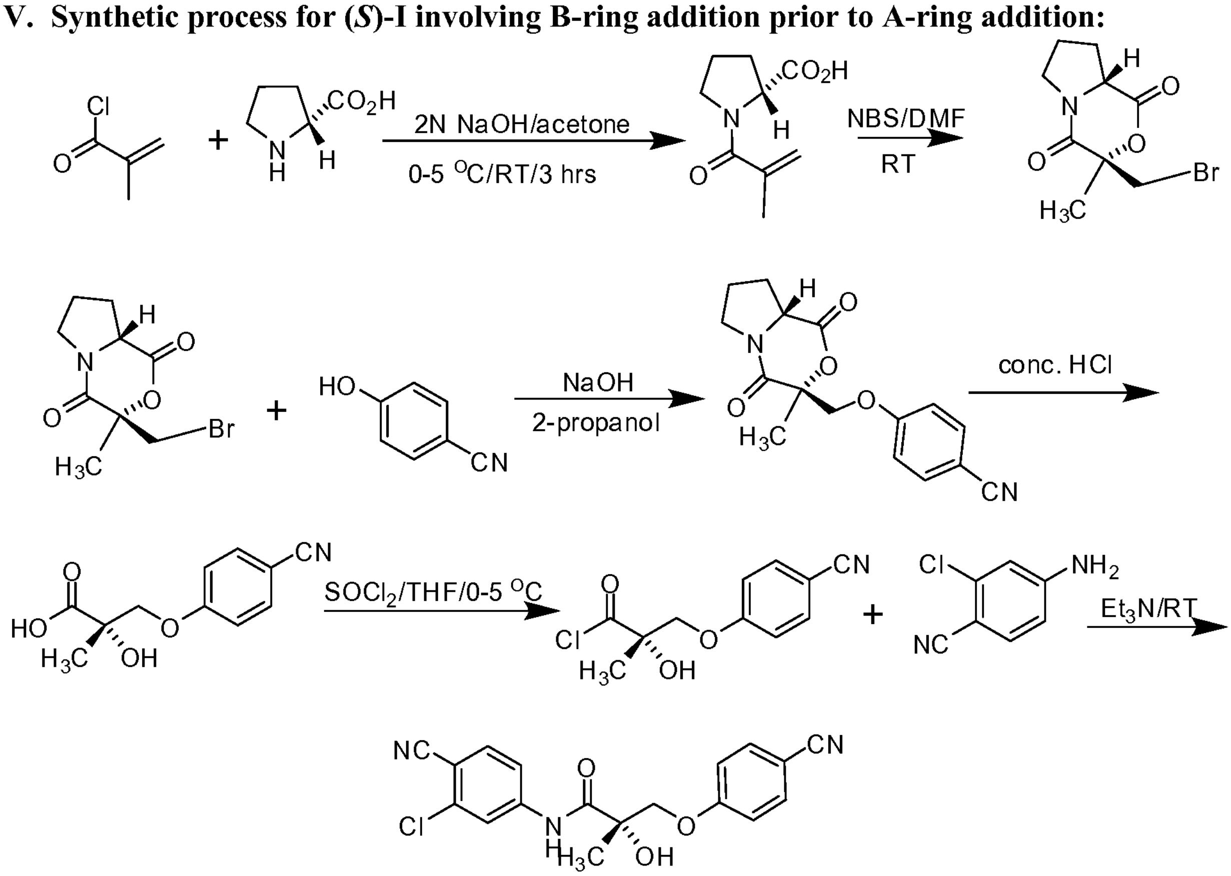

**Figure 1F**

**VI. Synthetic process for (*R*)-I involving B-ring addition prior to A-ring addition:**

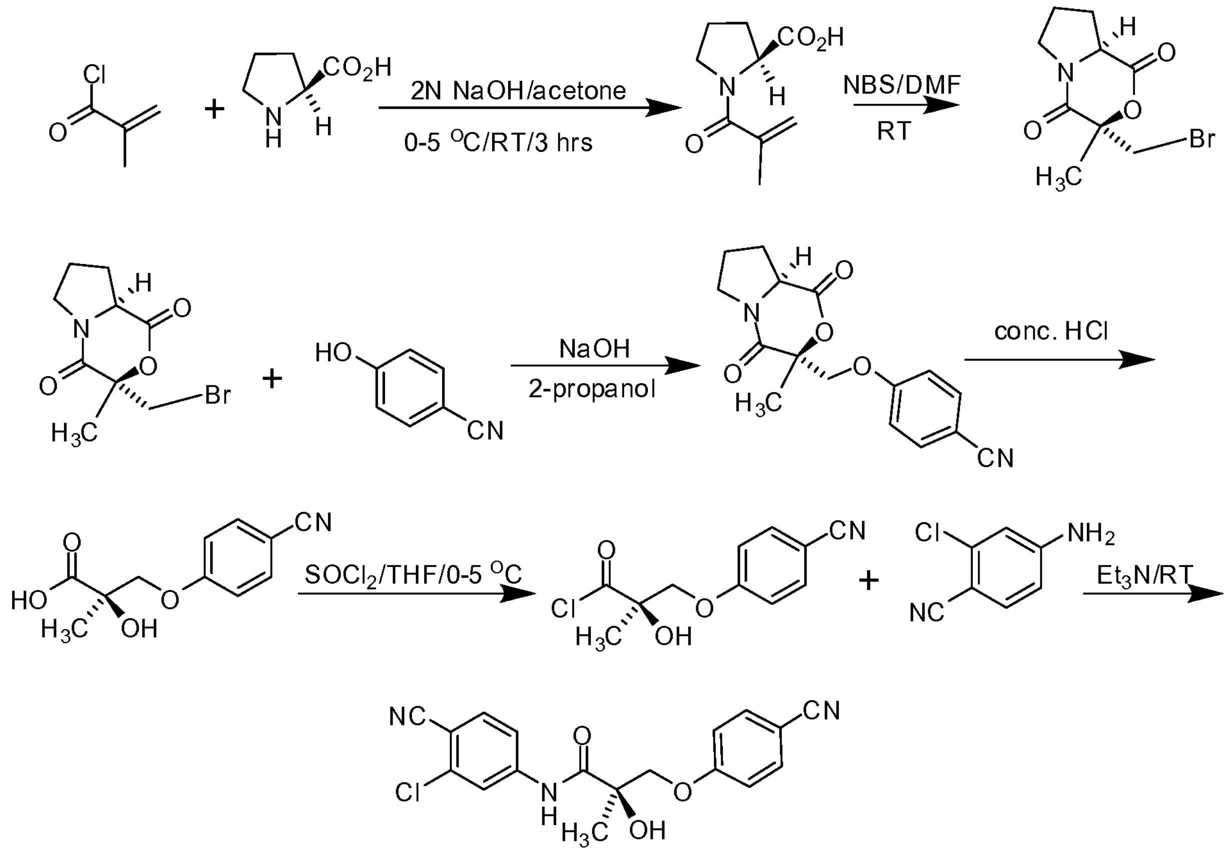

**Figure 1G**

**VII. Synthetic process for (*S*)-I using chiral intermediate and involving B-ring addition prior to A-ring addition:**

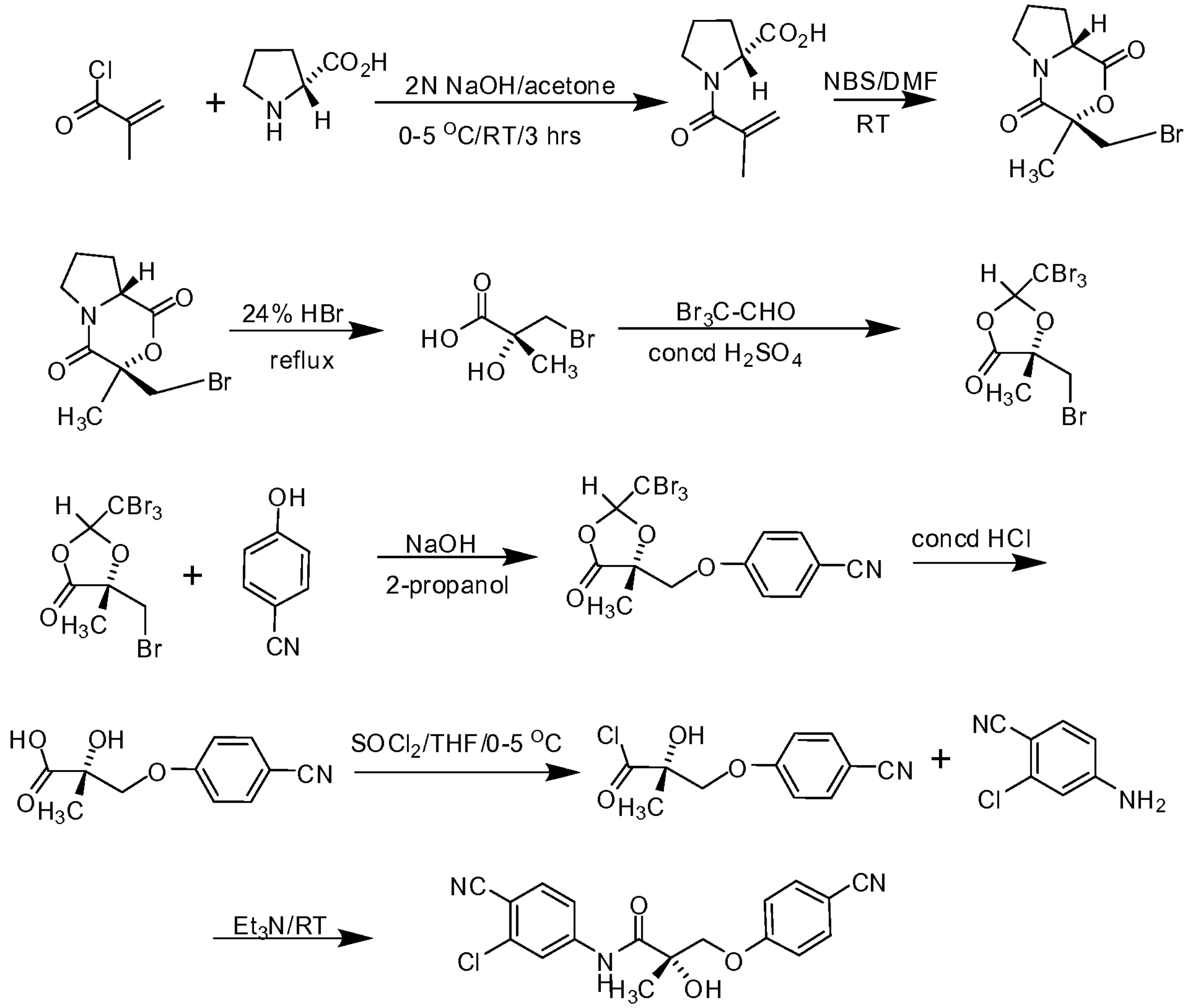

**Figure 1H**

**VIII. Synthetic process for (*R*)-I using chiral intermediate and involving B-ring addition prior to A-ring addition:**

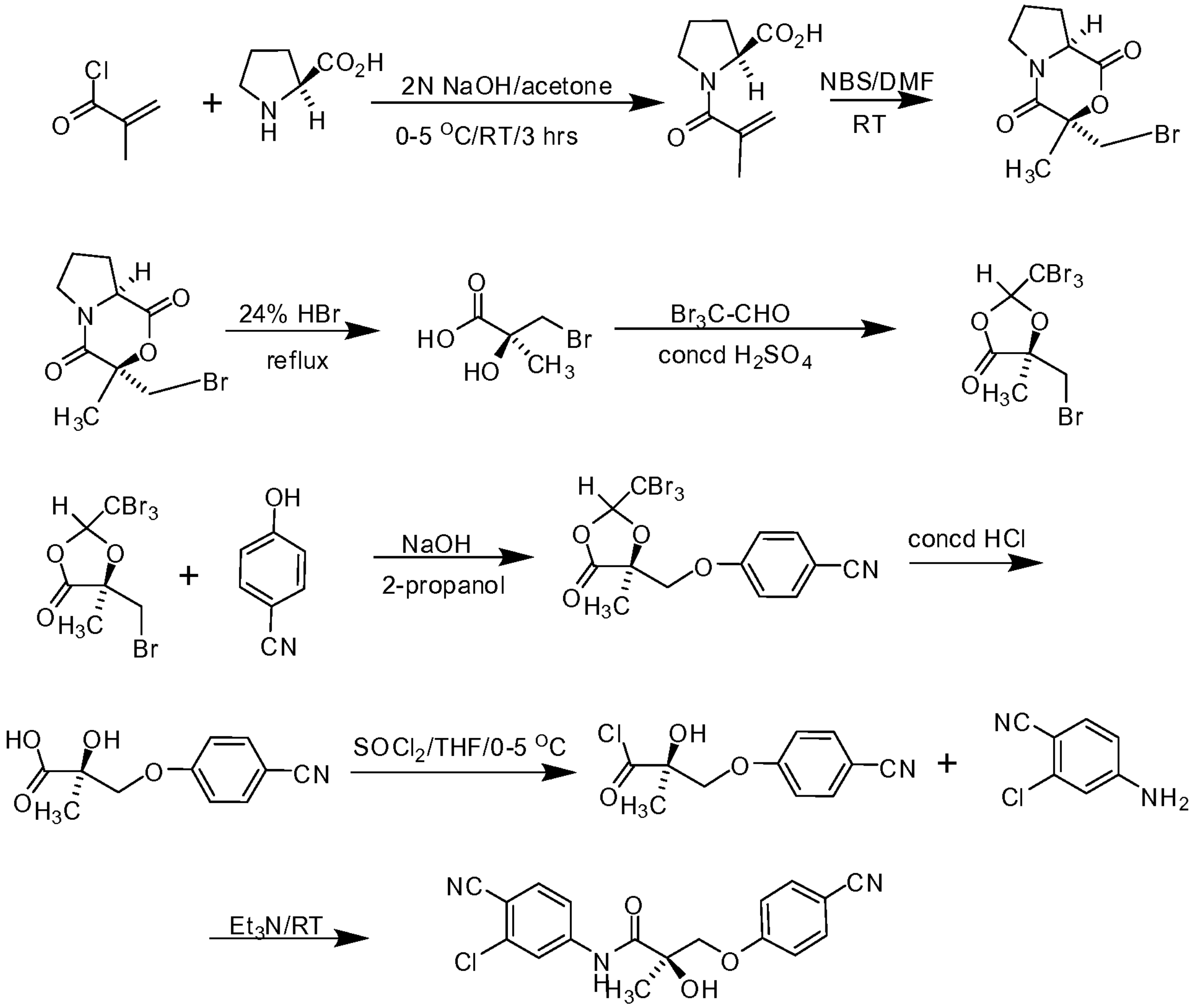

**Figure 1I**

**IX. Racemic synthetic process for compound I involving oxazolidinedione intermediate:**

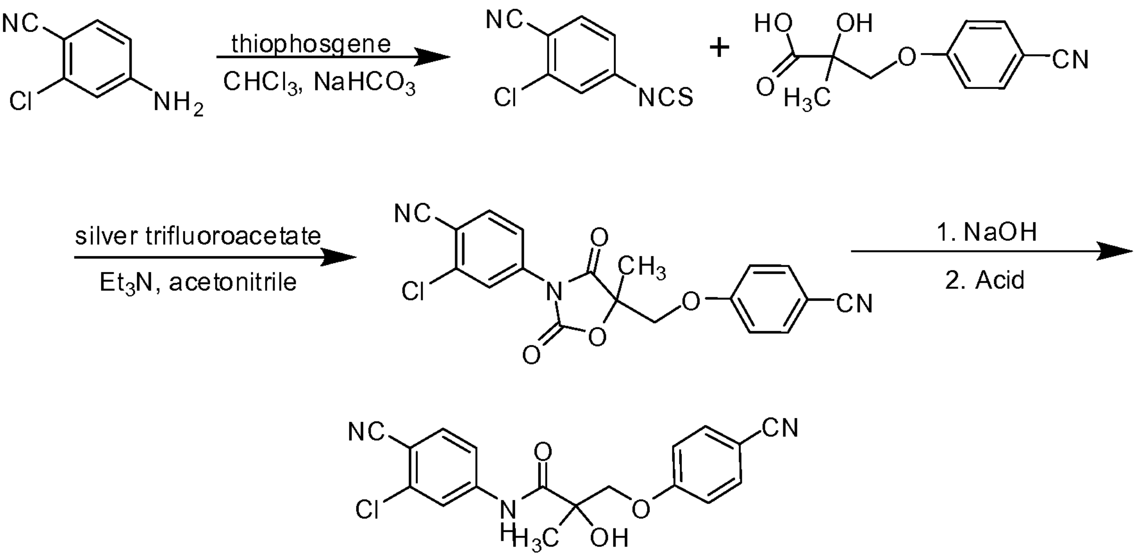

**Figure 1J**

**X. Racemic synthetic process for compound I involving oxirane intermediate:**

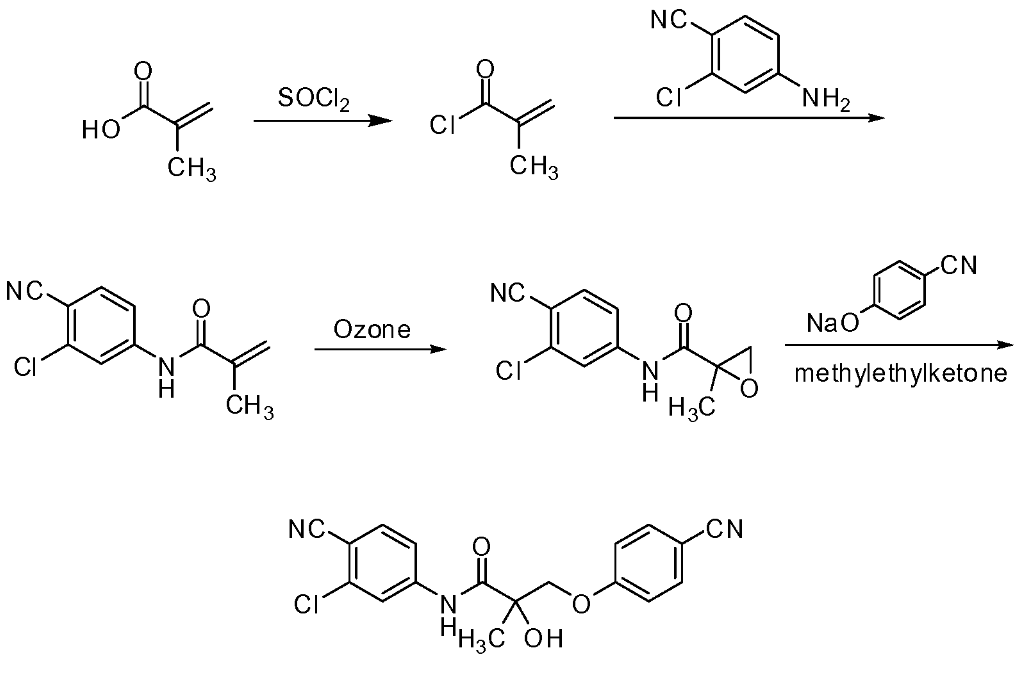

**Figure 1K**

**XI. Large-scale synthetic process for (*S*)-I:**

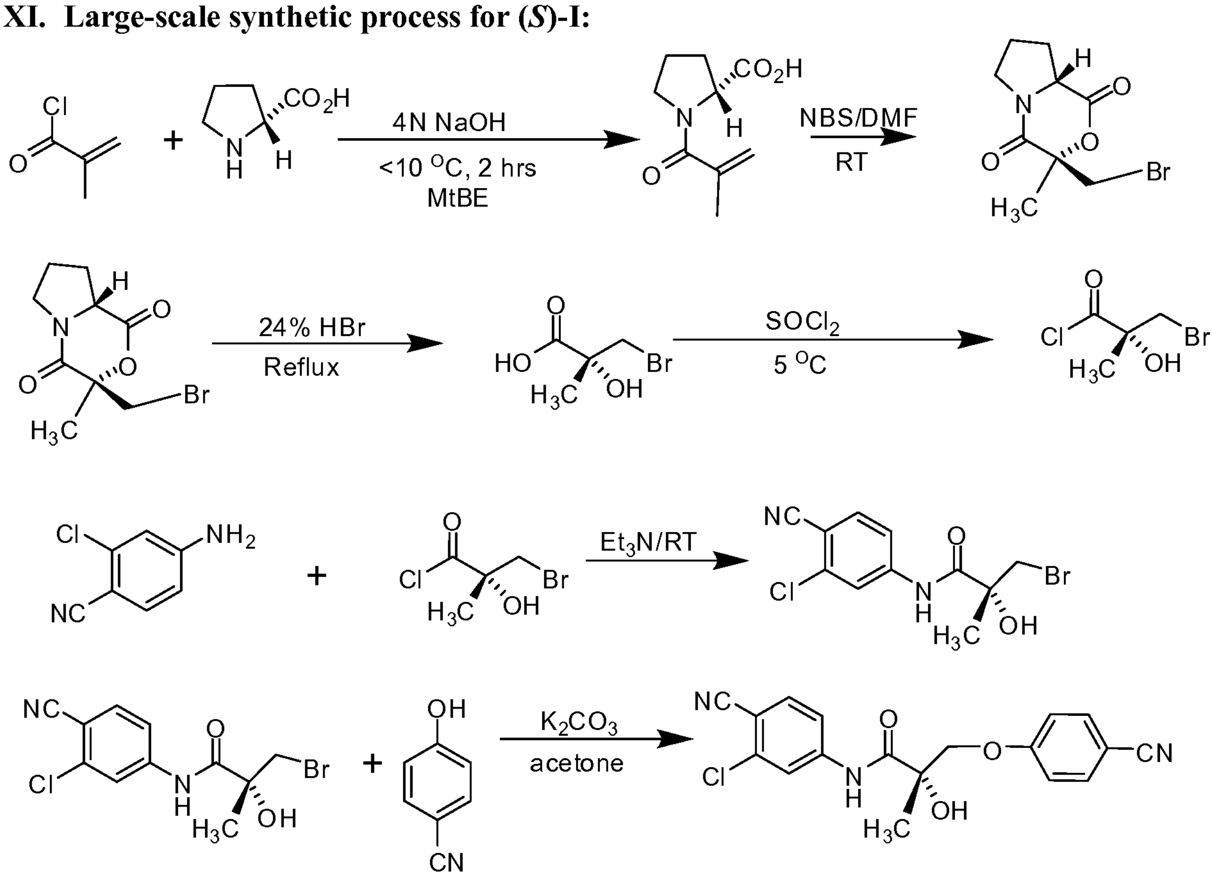

**Figure 1L**

**XII. Large-scale synthetic process for (*S*)-I:**

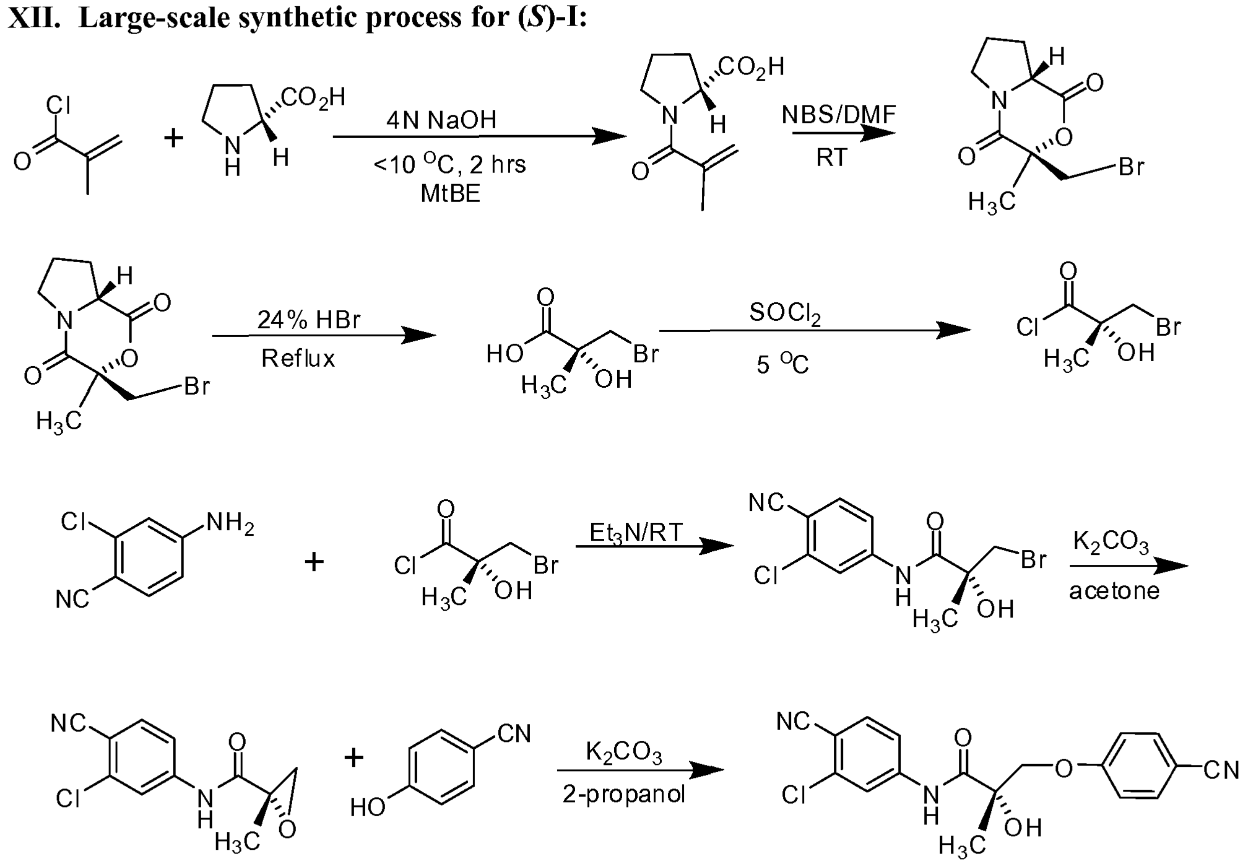

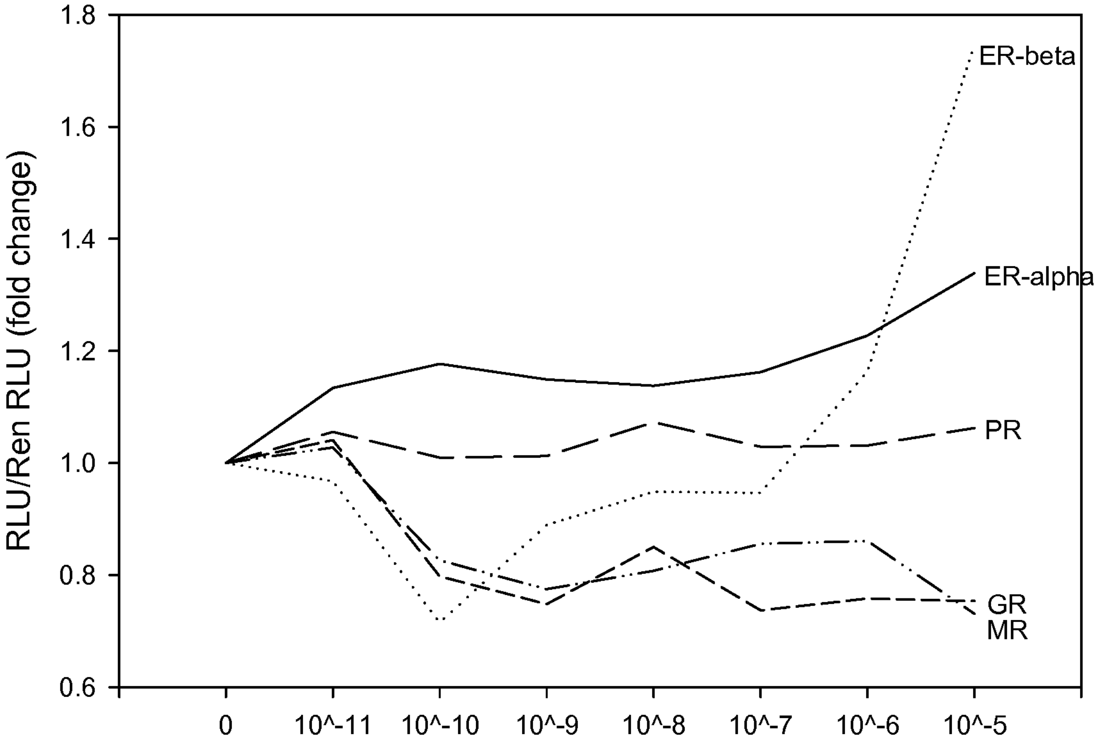
1.8
1.6
1.4
1.2
1.0
0.8
0.6
RLU/Ren RLU (fold change)
0
10^-11
10^-10
10^-9
10^-8
10^-7
10^-6
10^-5
ER-beta
ER-alpha
PR
GR
MR

FIGURE 2

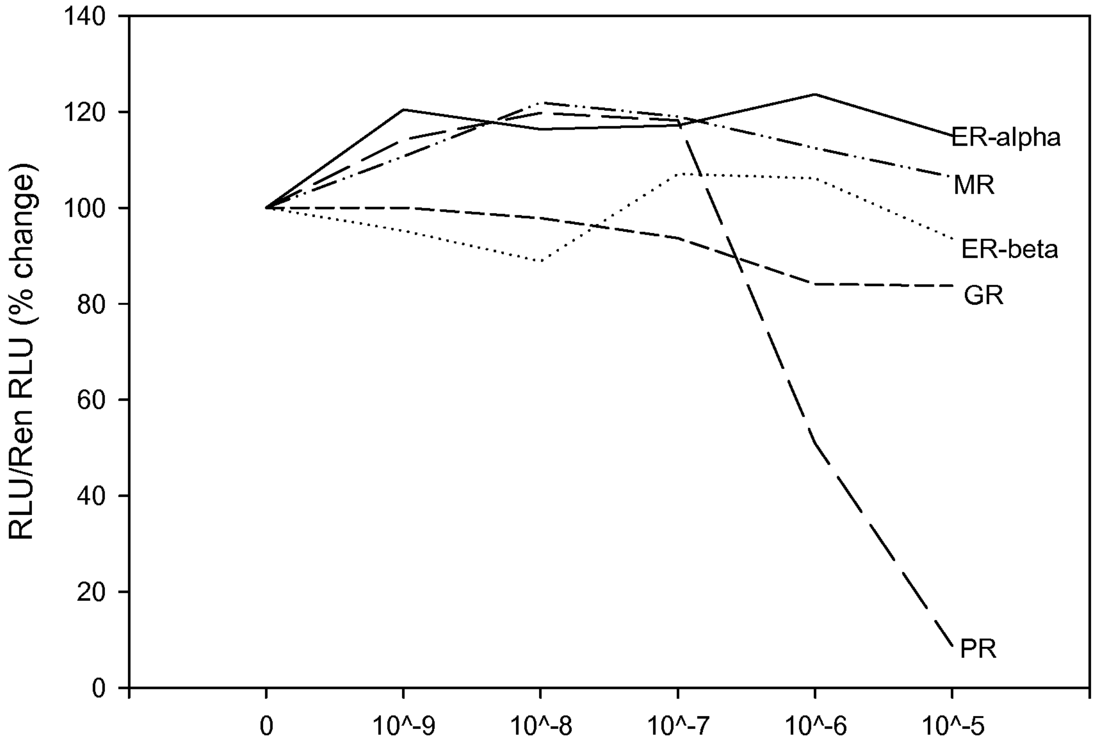
140
120
100
80
60
40
20
0
RLU/Ren RLU (% change)
0
10^-9
10^-8
10^-7
10^-6
10^-5
ER-alpha
MR
ER-beta
GR
PR

FIGURE 3

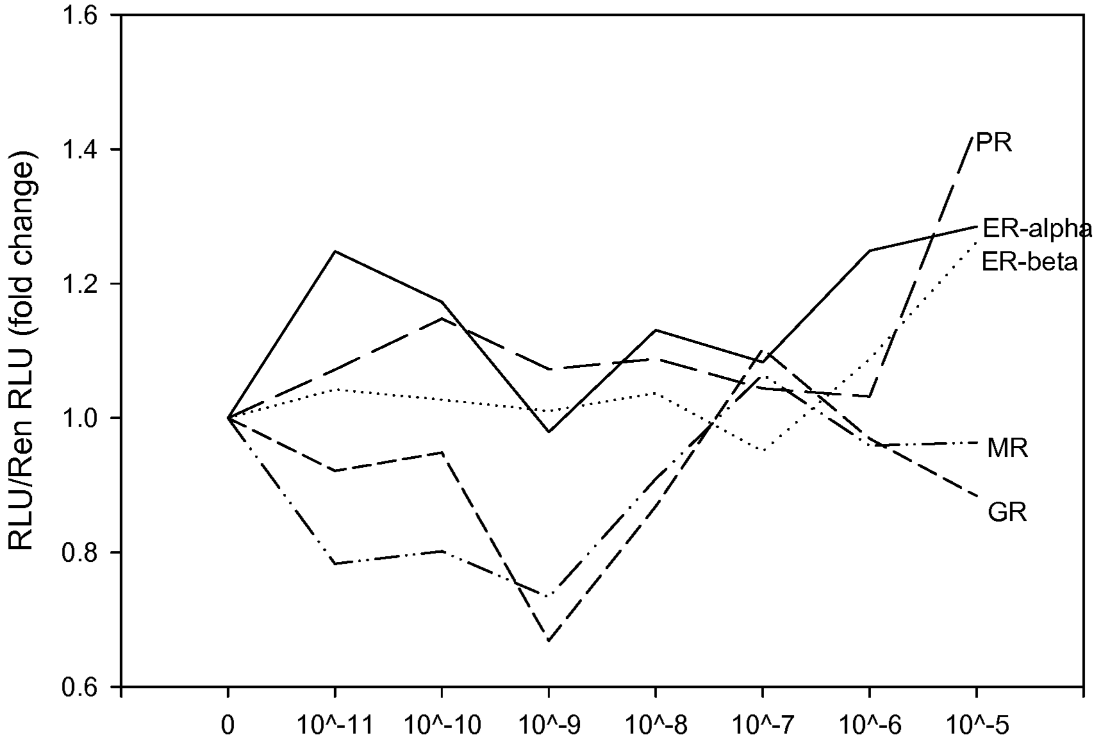
1.6
1.4
1.2
1.0
0.8
0.6
RLU/Ren RLU (fold change)
0
10^-11
10^-10
10^-9
10^-8
10^-7
10^-6
10^-5
PR
ER-alpha
ER-beta
MR
GR

FIGURE 4

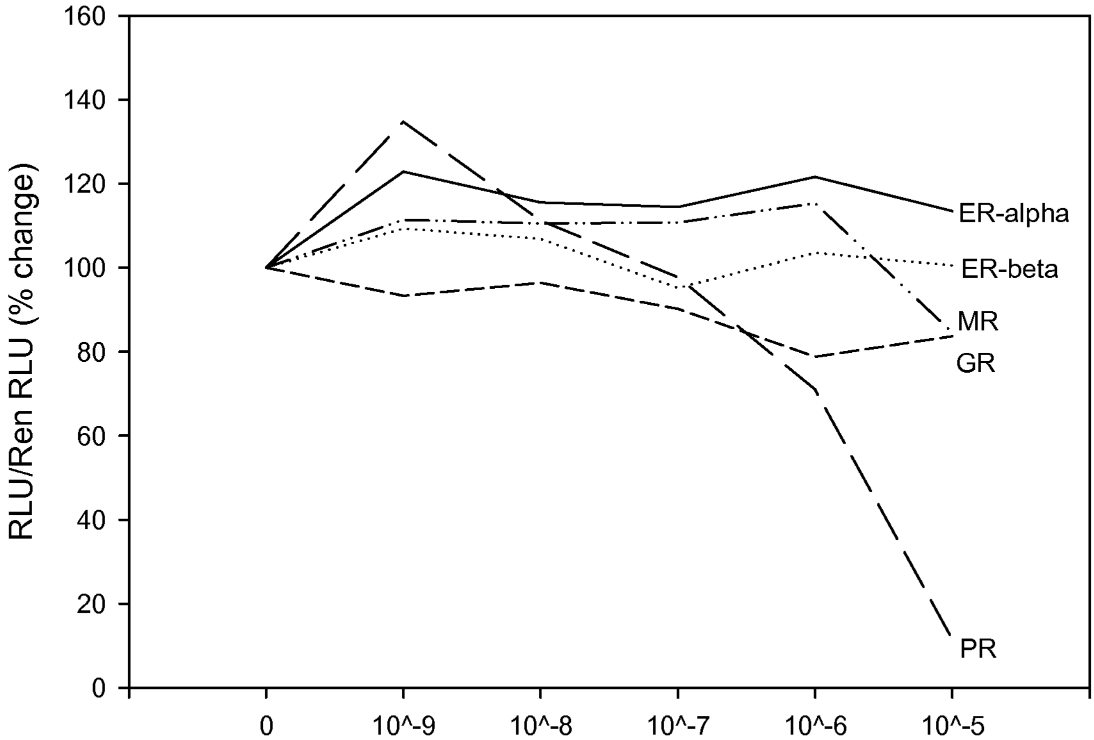
160
140
120
100
80
60
40
20
0
RLU/Ren RLU (% change)
0
10^-9
10^-8
10^-7
10^-6
10^-5
ER-alpha
ER-beta
MR
GR
PR

FIGURE 5

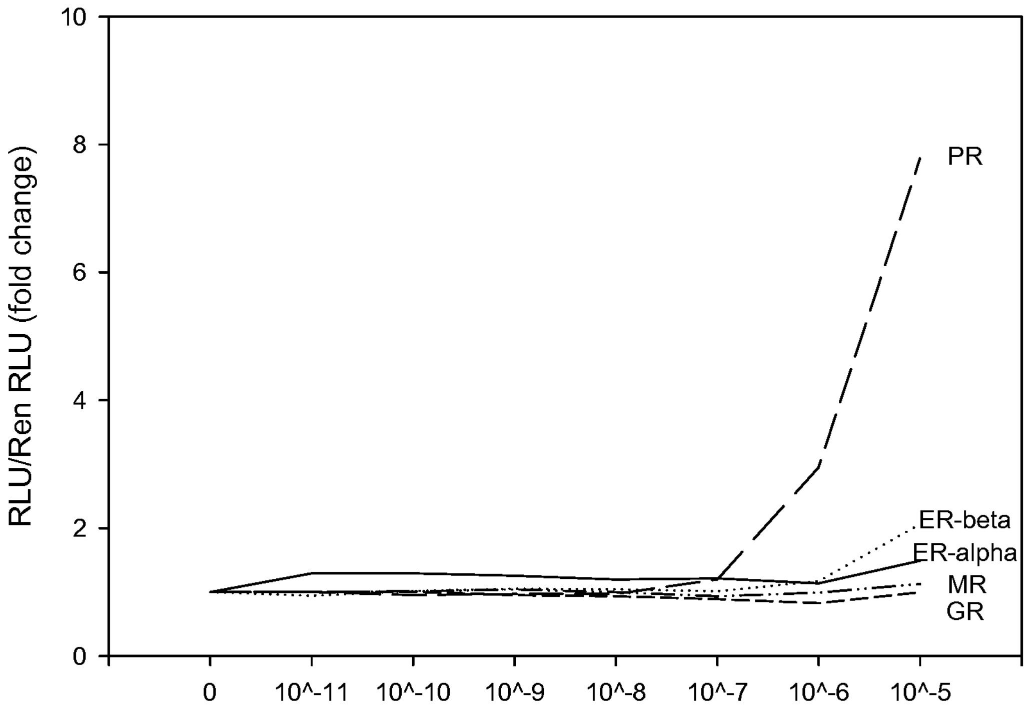
10
8
6
4
2
0
RLU/Ren RLU (fold change)
PR
ER-beta
ER-alpha
MR
GR
0
10^-11
10^-10
10^-9
10^-8
10^-7
10^-6
10^-5

FIGURE 6

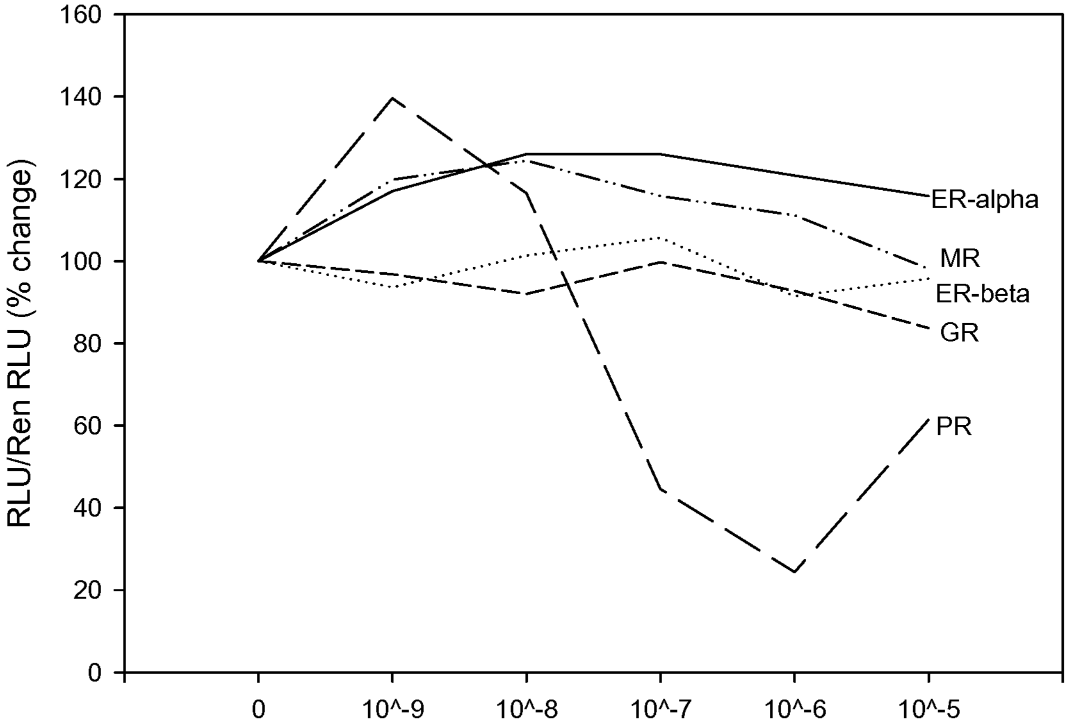
RLU/Ren RLU (% change)
160
140
120
100
80
60
40
20
0
0
10^-9
10^-8
10^-7
10^-6
10^-5
ER-alpha
MR
ER-beta
GR
PR

FIGURE 7

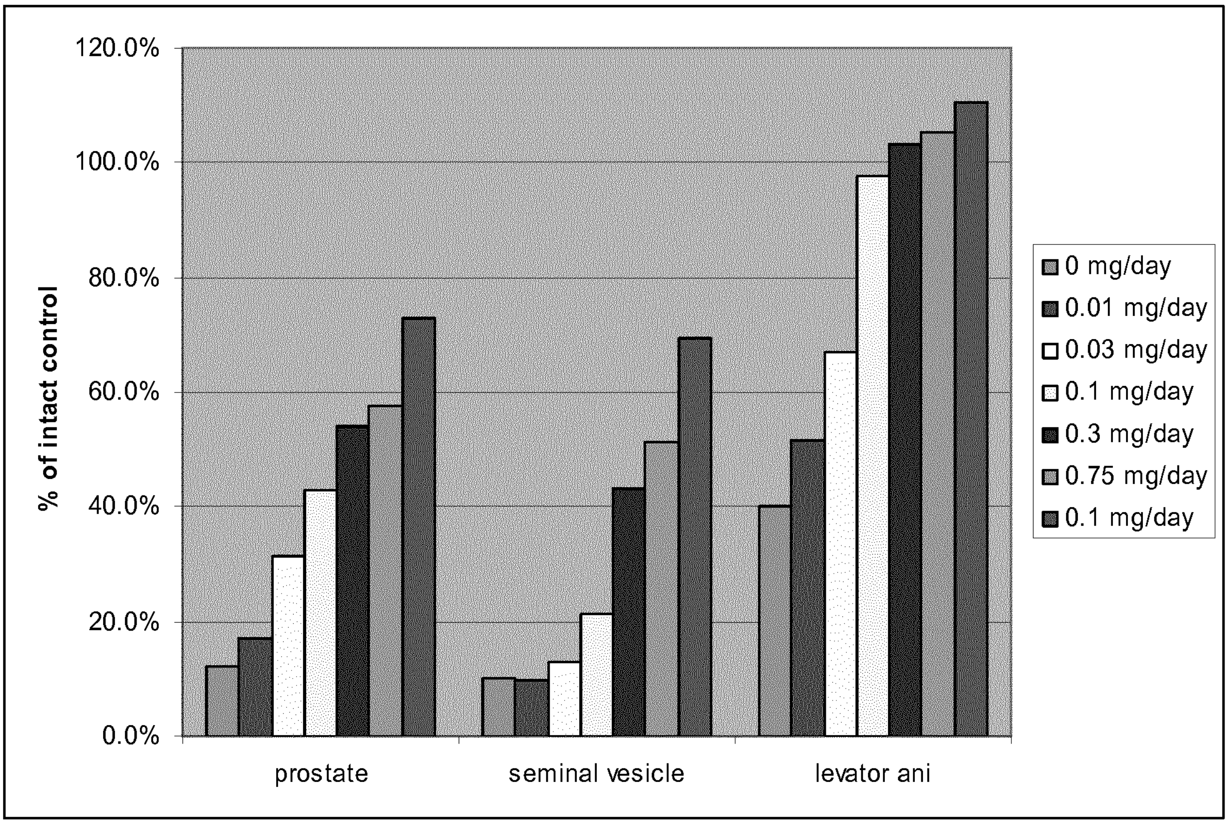
120.0%
100.0%
80.0%
60.0%
40.0%
20.0%
0.0%
% of intact control
prostate
seminal vesicle
levator ani
0 mg/day
0.01 mg/day
0.03 mg/day
0.1 mg/day
0.3 mg/day
0.75 mg/day
0.1 mg/day

FIGURE 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004064747 A2
- WO 2005037201 A2
- US 2006111441 A1


### Non-patent literature cited in the description


- **MATSUMOTO.** *Endocrinol. Met. Clin. N. Am.,* 1994, vol. 23, 857-75
- **ZHOU et al.** *Molec. Endocrinol.,* 1995, vol. 9, 208-18
- **SUNDARAM et al.** 7 Alpha-Methyl-Nortestosterone(MENT): The Optimal Androgen For Male Contraception. *Ann. Med.,* 1993, vol. 25, 199-205
- **KIM et al.** *Journal of Pharmacology and Experimental Therapeutics,* 2005, vol. 315 (1), 230-239
- **KASTNER et al.** *EMBO J,* 1990, vol. 9, 1603-1614
- **WEI et al.** *Mol Endo,* 1996, vol. 10, 1379-1387
- **STEINBERGER et al.** *Effect of Chronic Administration of Testosterone Enanthate on Sperm Production and Plasma Testosterone, Follicle Stimulating Hormone, and Luteinizing Hormone Levels: A Preliminary Evaluation of a Possible Male Contraceptive, Fertility and Sterility,* 1977, vol. 28, 1320-28
- Contraceptive Efficacy of Testosterone-Induced Azoospermia and Oligospermia in Normal Men. Fertility and Sterility. World Health Organization Task Force on Methods And Regulation of Male Fertility, 1996, vol. 65, 821-29
- **WU.** Effects of Testosterone Enanthate in Normal Men: Experience From a Multicenter Contraceptive Efficacy Study. *Fertility and Sterility,* 1996, vol. 65, 626-36
- **LANGER.** *Science,* 1990, vol. 249, 1627-1633
- **TREAT et al.** Liposomes in the Therapy of Infectious Disease and Cancer. Liss, 1989, 363-366
- LIPOSOMES IN THE THERAPY OF INFECTIOUS DISEASE AND CANCER. 317-327
- **SEFTON.** *CRC Crit. Ref. Biomed. Eng.,* 1987, vol. 14, 201
- **BUCHWALD et al.** *Surgery,* 1980, vol. 88, 607
- **SAUDEK et al.** *N. Engl. J. Med.,* 1989, vol. 321, 674
- **GOODSON.** *Medical Applications of Controlled Release,* 1984, vol. 2, 116-138